# EUROPEAN PATENT APPLICATION

(11) **EP 2 210 617 A1**
(43) Date of publication of application: **28.07.2010**
(21) Application number: 10155955.7
(22) Date of filing: 20.10.2004
(51) Int. Cl.: A61K 48/00, C12N 15/12, C07K 14/475, C12N 15/867, A61P 25/16, C12N 5/10

(54) **Mammalian cells secreting Neurturin and their therapeutic use**

(30) Priority: 20.10.2003 DK 200301543; 22.10.2003 US 512918 P
(62) Divisional of application: 04791259.7
(71) Applicant: NsGene A/S, 2750 Bellerup (DK)
(72) Inventor: Tornøe, Jens, 2750, Ballerup (DK); Rosenblad, Carl, 22240, Lund (SE); Wahlberg, Lars, Little Compton, RI 02837 (US)
(74) Representative: Høiberg A/S

(57) **Abstract**

The present invention concerns methods and compositions for gene therapy, in particular in vivo gene therapy for delivery of bioactive Neurturin for the treatment of Parkinson's Disease. In another aspect the invention relates to virus expression constructs comprising a mammalian signal peptide linked to a mature or N-terminally truncated Neurturin without a functional pro-region between the signal peptide and the Neurturin. These viral expression constructs are required for efficient secretion of bioactive Neurturin in in vivo gene therapy. The invention also concerns mammalian cells capable of producing Neurturin in increased amounts as well as the use of these cells for recombinant production of bioactive Neurturin and for therapeutic use.

SEQUENCE LISTING

<110>  NsGene A/S
       Tornøe, Jens
       Rosenblad, Carl
       Wahlberg, Lars

<120>  In vivo gene therapy of Parkinson's Disease

<130>  513-204-WO

<150>  DK PA 2003 01543
<151>  2003-10-20

<150>  US 06/512,918
<151>  2003-10-22

<160>  62

<170>  PatentIn version 3.1

<210>  1
<211>  19
<212>  PRT
<213>  Homo sapiens

<400>  1
Met Asp Cys Thr Trp Arg Ile Leu Phe Leu Val Ala Ala Ala Thr Gly
1               5                   10                  15

Thr His Ala

<210>  2
<211>  19
<212>  PRT
<213>  Macaca mulatta

<400>  2

Met Lys His Leu Trp Phe Phe Leu Leu Leu Val Ala Ala Pro Arg Trp
1               5                   10                  15

Val Leu Ser


<210>  3

<211>  19

<212>  PRT

<213>  Callithrix jacchus


<400>  3

Met Asp Trp Thr Trp Arg Ile Phe Leu Leu Val Ala Thr Ala Thr Gly
1               5                   10                  15

Ala His Ser


<210>  4

<211>  19

<212>  PRT

<213>  Mus musculus


<400>  4

Met Lys Cys Ser Trp Val Ile Phe Phe Leu Met Ala Val Val Thr Gly
1               5                   10                  15

Val Asn Ser


<210>  5

<211>  19

<212>  PRT

<213>  Sus scrofa


<400>  5

Met Glu Phe Arg Leu Asn Trp Val Val Leu Phe Ala Leu Leu Gln Gly
1               5                   10                  15

Val Gln Gly

<210> 6

<211> 19

<212> PRT

<213> Rattus norvegicus

<400> 6

Met Lys Cys Ser Trp Ile Ile Leu Phe Leu Met Ala Leu Thr Thr Gly
1               5                   10                  15

Val Asn Ser

<210> 7

<211> 309

<212> DNA

<213> Homo sapiens

<220>

<221> CDS

<222> (1)..(309)

<223>

<400> 7
gcg cgg ttg ggg gcg cgg cct tgc ggg ctg cgc gag ctg gag gtg cgc 48
Ala Arg Leu Gly Ala Arg Pro Cys Gly Leu Arg Glu Leu Glu Val Arg
1               5                   10                  15

gtg agc gag ctg ggc ctg ggc tac gcg tcc gac gag acg gtg ctg ttc 96
Val Ser Glu Leu Gly Leu Gly Tyr Ala Ser Asp Glu Thr Val Leu Phe
            20                  25                  30

cgc tac tgc gca ggc gcc tgc gag gct gcc gcg cgc gtc tac gac ctc 144
Arg Tyr Cys Ala Gly Ala Cys Glu Ala Ala Ala Arg Val Tyr Asp Leu
        35                  40                  45

ggg ctg cga cga ctg cgc cag cgg cgg cgc ctg cgg cgg gag cgg gtg 192
Gly Leu Arg Arg Leu Arg Gln Arg Arg Arg Leu Arg Arg Glu Arg Val
    50                  55                  60

cgc gcg cag ccc tgc tgc cgc ccg acg gcc tac gag gac gag gtg tcc 240
Arg Ala Gln Pro Cys Cys Arg Pro Thr Ala Tyr Glu Asp Glu Val Ser
65                  70                  75                  80

ttc ctg gac gcg cac agc cgc tac cac acg gtg cac gag ctg tcg gcg 288
Phe Leu Asp Ala His Ser Arg Tyr His Thr Val His Glu Leu Ser Ala
                85                  90                  95

cgc gag tgc gcc tgc gtg tga                                                309
Arg Glu Cys Ala Cys Val
                100

<210>  8

<211>  102

<212>  PRT

<213>  Homo sapiens

<400>  8

Ala Arg Leu Gly Ala Arg Pro Cys Gly Leu Arg Glu Leu Glu Val Arg
1               5                   10                  15

Val Ser Glu Leu Gly Leu Gly Tyr Ala Ser Asp Glu Thr Val Leu Phe
            20                  25                  30

Arg Tyr Cys Ala Gly Ala Cys Glu Ala Ala Ala Arg Val Tyr Asp Leu
        35                  40                  45

Gly Leu Arg Arg Leu Arg Gln Arg Arg Arg Leu Arg Arg Glu Arg Val
    50                  55                  60

Arg Ala Gln Pro Cys Cys Arg Pro Thr Ala Tyr Glu Asp Glu Val Ser
65                  70                  75                  80

Phe Leu Asp Ala His Ser Arg Tyr His Thr Val His Glu Leu Ser Ala
                85                  90                  95

Arg Glu Cys Ala Cys Val
                100

<210>  9

<211>  96

<212>  PRT

<213>  Homo sapiens

<400>  9

Pro Cys Gly Leu Arg Glu Leu Glu Val Arg Val Ser Glu Leu Gly Leu
1               5                   10                  15

Gly Tyr Ala Ser Asp Glu Thr Val Leu Phe Arg Tyr Cys Ala Gly Ala
            20                  25                  30

Cys Glu Ala Ala Ala Arg Val Tyr Asp Leu Gly Leu Arg Arg Leu Arg
        35                  40                  45

Gln Arg Arg Arg Leu Arg Arg Glu Arg Val Arg Ala Gln Pro Cys Cys
    50                  55                  60

Arg Pro Thr Ala Tyr Glu Asp Glu Val Ser Phe Leu Asp Ala His Ser
65                  70                  75                  80

Arg Tyr His Thr Val His Glu Leu Ser Ala Arg Glu Cys Ala Cys Val
                85                  90                  95

<210>  10

<211>  100

<212>  PRT

<213>  Mus musculus

<400>  10

Pro Gly Ala Arg Pro Cys Gly Leu Arg Glu Leu Glu Val Arg Val Ser
1               5                   10                  15

Glu Leu Gly Leu Gly Tyr Thr Ser Asp Glu Thr Val Leu Phe Arg Tyr
            20                  25                  30

Cys Ala Gly Ala Cys Glu Ala Ala Ile Arg Ile Tyr Asp Leu Gly Leu
        35                  40                  45

Arg Arg Leu Arg Gln Arg Arg Arg Val Arg Arg Glu Arg Ala Arg Ala
    50                  55                  60

His Pro Cys Cys Arg Pro Thr Ala Tyr Glu Asp Glu Val Ser Phe Leu
65                  70                  75                  80

Asp Val His Ser Arg Tyr His Thr Leu Gln Glu Leu Ser Ala Arg Glu
                85                  90                  95

Cys Ala Cys Val
            100

<210>  11

<211>  100

<212>  PRT

<213>  Rattus norvegicus

<400>  11

Pro Gly Ser Arg Pro Cys Gly Leu Arg Glu Leu Glu Val Arg Val Ser
1               5                   10                  15

Glu Leu Gly Leu Gly Tyr Thr Ser Asp Glu Thr Val Leu Phe Arg Tyr

20                  25                  30

Cys Ala Gly Ala Cys Glu Ala Ala Ile Arg Ile Tyr Asp Leu Gly Leu
        35                  40                  45

Arg Arg Leu Arg Gln Arg Arg Arg Val Arg Lys Glu Arg Val Arg Ala
    50                  55                  60

His Pro Cys Cys Arg Pro Thr Ala Tyr Glu Asp Glu Val Ser Phe Leu
65                  70                  75                  80

Asp Val His Ser Arg Tyr His Thr Leu Gln Glu Leu Ser Ala Arg Glu
                85                  90                  95

Cys Ala Cys Val
            100

<210>  12

<211>  197

<212>  PRT

<213>  Homo sapiens


<220>

<221>  SIGNAL

<222>  (1)..(19)

<223>


<220>

<221>  PROPEP

<222>  (20)..(95)

<223>


<220>

<221>  VARIANT

<222>  (96)..(96)

<223>  A->S


<220>

<221>  mat_peptide

<222>  (96)..()

<223>

<400>  12

Met Gln Arg Trp Lys Ala Ala Ala Leu Ala Ser Val Leu Cys Ser Ser
-95                 -90                 -85                 -80

Val Leu Ser Ile Trp Met Cys Arg Glu Gly Leu Leu Leu Ser His Arg
                -75                 -70                 -65

Leu Gly Pro Ala Leu Val Pro Leu His Arg Leu Pro Arg Thr Leu Asp
            -60                 -55                 -50

Ala Arg Ile Ala Arg Leu Ala Gln Tyr Arg Ala Leu Leu Gln Gly Ala
        -45                 -40                 -35

Pro Asp Ala Met Glu Leu Arg Glu Leu Thr Pro Trp Ala Gly Arg Pro
    -30                 -25                 -20

Pro Gly Pro Arg Arg Arg Ala Gly Pro Arg Arg Arg Arg Ala Arg Ala
-15                 -10                 -5                  -1  1

Arg Leu Gly Ala Arg Pro Cys Gly Leu Arg Glu Leu Glu Val Arg Val
            5                   10                  15

Ser Glu Leu Gly Leu Gly Tyr Ala Ser Asp Glu Thr Val Leu Phe Arg
        20                  25                  30

Tyr Cys Ala Gly Ala Cys Glu Ala Ala Ala Arg Val Tyr Asp Leu Gly
    35                  40                  45

Leu Arg Arg Leu Arg Gln Arg Arg Arg Leu Arg Arg Glu Arg Val Arg
50                  55                  60                  65

Ala Gln Pro Cys Cys Arg Pro Thr Ala Tyr Glu Asp Glu Val Ser Phe
                70                  75                  80

Leu Asp Ala His Ser Arg Tyr His Thr Val His Glu Leu Ser Ala Arg
            85                  90                  95

Glu Cys Ala Cys Val
        100

<210>  13

<211>  195

<212>  PRT

<213>  Mus musculus

<220>

<221>  SIGNAL
<222>  (1)..(23)
<223>

<220>
<221>  SIGNAL
<222>  (1)..(19)
<223>

<220>
<221>  mat_peptide
<222>  (96)..()
<223>

<400>  13
Met Arg Arg Trp Lys Ala Ala Ala Leu Val Ser Leu Ile Cys Ser Ser
-95                 -90                 -85                 -80

Leu Leu Ser Val Trp Met Cys Gln Glu Gly Leu Leu Leu Gly His Arg
                -75                 -70                 -65

Leu Gly Pro Ala Leu Ala Pro Leu Arg Arg Pro Pro Arg Thr Leu Asp
            -60                 -55                 -50

Ala Arg Ile Ala Arg Leu Ala Gln Tyr Arg Ala Leu Leu Gln Gly Ala
        -45                 -40                 -35

Pro Asp Ala Val Glu Leu Arg Glu Leu Ser Pro Trp Ala Ala Arg Ile
    -30                 -25                 -20

Pro Gly Pro Arg Arg Arg Ala Gly Pro Arg Arg Arg Arg Ala Arg Pro
-15                 -10                 -5                  -1  1

Gly Ala Arg Pro Cys Gly Leu Arg Glu Leu Glu Val Arg Val Ser Glu
            5                   10                  15

Leu Gly Leu Gly Tyr Thr Ser Asp Glu Thr Val Leu Phe Arg Tyr Cys
        20                  25                  30

Ala Gly Ala Cys Glu Ala Ala Ile Arg Ile Tyr Asp Leu Gly Leu Arg
    35                  40                  45

Arg Leu Arg Gln Arg Arg Arg Val Arg Arg Glu Arg Ala Arg Ala His
50                  55                  60                  65

Pro Cys Cys Arg Pro Thr Ala Tyr Glu Asp Glu Val Ser Phe Leu Asp
                70                  75                  80

Val His Ser Arg Tyr His Thr Leu Gln Glu Leu Ser Ala Arg Glu Cys
            85                  90                  95

Ala Cys Val
        100

<210>  14

<211>  195

<212>  PRT

<213>  Rattus norvegicus


<220>

<221>  SIGNAL

<222>  (1)..(23)

<223>


<220>

<221>  SIGNAL

<222>  (1)..(19)

<223>


<220>

<221>  mat_peptide

<222>  (96)..()

<223>


<400>  14

Met Arg Cys Trp Lys Ala Ala Ala Leu Val Ser Leu Ile Cys Ser Ser
-95                 -90                 -85                 -80

Leu Leu Ser Val Trp Met Cys Gln Glu Gly Leu Leu Leu Gly His Arg
                -75                 -70                 -65

Leu Gly Pro Ala Leu Ala Pro Leu Arg Arg Pro Pro Arg Thr Leu Asp
            -60                 -55                 -50

Ala Arg Ile Ala Arg Leu Ala Gln Tyr Arg Ala Leu Leu Gln Gly Ala

-45                 -40                 -35

Pro Asp Ala Val Glu Leu Arg Glu Leu Ser Pro Trp Val Ala Arg Pro
    -30                 -25                 -20

Ser Gly Pro Arg Arg Arg Ala Gly Pro Arg Arg Arg Arg Ala Arg Pro
-15                 -10                 -5                  -1  1

Gly Ser Arg Pro Cys Gly Leu Arg Glu Leu Glu Val Arg Val Ser Glu
            5                   10                  15

Leu Gly Leu Gly Tyr Thr Ser Asp Glu Thr Val Leu Phe Arg Tyr Cys
        20                  25                  30

Ala Gly Ala Cys Glu Ala Ala Ile Arg Ile Tyr Asp Leu Gly Leu Arg
    35                  40                  45

Arg Leu Arg Gln Arg Arg Arg Val Arg Lys Glu Arg Val Arg Ala His
50                  55                  60                  65

Pro Cys Cys Arg Pro Thr Ala Tyr Glu Asp Glu Val Ser Phe Leu Asp
                70                  75                  80

Val His Ser Arg Tyr His Thr Leu Gln Glu Leu Ser Ala Arg Glu Cys
            85                  90                  95

Ala Cys Val
        100

<210>  15

<211>  392

<212>  DNA

<213>  Homo sapiens


<220>

<221>  CDS

<222>  (17)..(379)

<223>


<220>

<221>  sig_peptide

<222>  (17)..(73)

<223>

<220>

<221>  mat_peptide

<222>  (74)..()

<223>


<400>  15
tataggatcc gccacc atg cag cgc tgg aag gcg gcg gcc ttg gcc tca gtg      52
                  Met Gln Arg Trp Lys Ala Ala Ala Leu Ala Ser Val
                                  -15                 -10

ctc tgc agc tcc gtg ctg tcc gcg cgg ttg ggg gcg cgg cct tgc ggg      100
Leu Cys Ser Ser Val Leu Ser Ala Arg Leu Gly Ala Arg Pro Cys Gly
        -5                  -1  1               5

ctg cgc gag ctg gag gtg cgc gtg agc gag ctg ggc ctg ggc tac gcg      148
Leu Arg Glu Leu Glu Val Arg Val Ser Glu Leu Gly Leu Gly Tyr Ala
10                  15                  20                  25

tcc gac gag acg gtg ctg ttc cgc tac tgc gca ggc gcc tgc gag gct      196
Ser Asp Glu Thr Val Leu Phe Arg Tyr Cys Ala Gly Ala Cys Glu Ala
                30                  35                  40

gcc gcg cgc gtc tac gac ctc ggg ctg cga cga ctg cgc cag cgg cgg      244
Ala Ala Arg Val Tyr Asp Leu Gly Leu Arg Arg Leu Arg Gln Arg Arg
            45                  50                  55

cgc ctg cgg cgg gag cgg gtg cgc gcg cag ccc tgc tgc cgc ccg acg      292
Arg Leu Arg Arg Glu Arg Val Arg Ala Gln Pro Cys Cys Arg Pro Thr
        60                  65                  70

gcc tac gag gac gag gtg tcc ttc ctg gac gcg cac agc cgc tac cac      340
Ala Tyr Glu Asp Glu Val Ser Phe Leu Asp Ala His Ser Arg Tyr His
    75                  80                  85

acg gtg cac gag ctg tcg gcg cgc gag tgc gcc tgc gtg tgactcgagt      389
Thr Val His Glu Leu Ser Ala Arg Glu Cys Ala Cys Val
90                  95                  100

ata                                                                  392


<210>  16

<211>  121

<212>  PRT

<213>  Homo sapiens


<400>  16

Met Gln Arg Trp Lys Ala Ala Ala Leu Ala Ser Val Leu Cys Ser Ser
                -15                 -10                 -5

Val Leu Ser Ala Arg Leu Gly Ala Arg Pro Cys Gly Leu Arg Glu Leu
        -1  1               5                   10

Glu Val Arg Val Ser Glu Leu Gly Leu Gly Tyr Ala Ser Asp Glu Thr
    15                  20                  25

Val Leu Phe Arg Tyr Cys Ala Gly Ala Cys Glu Ala Ala Ala Arg Val
30                  35                  40                  45

Tyr Asp Leu Gly Leu Arg Arg Leu Arg Gln Arg Arg Arg Leu Arg Arg
                50                  55                  60

Glu Arg Val Arg Ala Gln Pro Cys Cys Arg Pro Thr Ala Tyr Glu Asp
            65                  70                  75

Glu Val Ser Phe Leu Asp Ala His Ser Arg Tyr His Thr Val His Glu
        80                  85                  90

Leu Ser Ala Arg Glu Cys Ala Cys Val
    95                  100

<210>  17

<211>  484

<212>  DNA

<213>  Artificial sequence


<220>

<223>  IgSP (mouse) - mature NTN (man)

<220>

<221>  CDS

<222>  (12)..(57)

<223>


<220>

<221>  CDS

<222>  (137)..(453)

<223>


<220>

<221>  sig_peptide

<222>  (12)..(147)

<223>


<220>

<221>  mat_peptide

<222>  (148)..()

<223>

<400>  17
gatccgccac c atg aaa tgc agc tgg gtt atc ttc ttc ctg atg gca gtg      50
             Met Lys Cys Ser Trp Val Ile Phe Phe Leu Met Ala Val
                             -15                 -10

gtt aca g gtaaggggct cccaagtccc aaacttgagg gtccataaac tctgtgacag      107
Val Thr
    -5

tggcaatcac tttgcctttc tttctacag gg  gtg aat tcg gcg cgg ttg ggg      159
                                Gly Val Asn Ser Ala Arg Leu Gly
                                            -1  1

gcg cgg cct tgc ggg ctg cgc gag ctg gag gtg cgc gtg agc gag ctg      207
Ala Arg Pro Cys Gly Leu Arg Glu Leu Glu Val Arg Val Ser Glu Leu
5                   10                  15                  20

ggc ctg ggc tac gcg tcc gac gag acg gtg ctg ttc cgc tac tgc gca      255
Gly Leu Gly Tyr Ala Ser Asp Glu Thr Val Leu Phe Arg Tyr Cys Ala
                25                  30                  35

ggc gcc tgc gag gct gcc gcg cgc gtc tac gac ctc ggg ctg cga cga      303
Gly Ala Cys Glu Ala Ala Ala Arg Val Tyr Asp Leu Gly Leu Arg Arg
            40                  45                  50

ctg cgc cag cgg cgg cgc ctg cgg cgg gag cgg gtg cgc gcg cag ccc      351
Leu Arg Gln Arg Arg Arg Leu Arg Arg Glu Arg Val Arg Ala Gln Pro
        55                  60                  65

tgc tgc cgc ccg acg gcc tac gag gac gag gtg tcc ttc ctg gac gcg      399
Cys Cys Arg Pro Thr Ala Tyr Glu Asp Glu Val Ser Phe Leu Asp Ala
    70                  75                  80

cac agc cgc tac cac acg gtg cac gag ctg tcg gcg cgc gag tgc gcc      447
His Ser Arg Tyr His Thr Val His Glu Leu Ser Ala Arg Glu Cys Ala
85                  90                  95                  100

tgc gtg tgacatatca agcttatcga taccgtcgac c                            484
Cys Val

<210>  18

<211>  121

<212>  PRT

<213>  Artificial sequence

<220>

<223>  IgSP (mouse) - mature NTN (man)

<400>  18

Met Lys Cys Ser Trp Val Ile Phe Phe Leu Met Ala Val Val Thr Gly
                -15                 -10                 -5

Val Asn Ser Ala Arg Leu Gly Ala Arg Pro Cys Gly Leu Arg Glu Leu

-1  1               5                   10

Glu Val Arg Val Ser Glu Leu Gly Leu Gly Tyr Ala Ser Asp Glu Thr
    15                  20                  25

Val Leu Phe Arg Tyr Cys Ala Gly Ala Cys Glu Ala Ala Ala Arg Val
30                  35                  40                  45

Tyr Asp Leu Gly Leu Arg Arg Leu Arg Gln Arg Arg Arg Leu Arg Arg
                50                  55                  60

Glu Arg Val Arg Ala Gln Pro Cys Cys Arg Pro Thr Ala Tyr Glu Asp
            65                  70                  75

Glu Val Ser Phe Leu Asp Ala His Ser Arg Tyr His Thr Val His Glu
        80                  85                  90

Leu Ser Ala Arg Glu Cys Ala Cys Val
    95                  100

<210>  19

<211>  120

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  19

Met Lys Cys Ser Trp Val Ile Phe Phe Leu Met Ala Val Val Thr Gly
                -15                 -10                 -5

Val Asn Ser Arg Leu Gly Ala Arg Pro Cys Gly Leu Arg Glu Leu Glu
        -1  1               5                   10

Val Arg Val Ser Glu Leu Gly Leu Gly Tyr Ala Ser Asp Glu Thr Val
    15                  20                  25

Leu Phe Arg Tyr Cys Ala Gly Ala Cys Glu Ala Ala Ala Arg Val Tyr
30                  35                  40                  45

Asp Leu Gly Leu Arg Arg Leu Arg Gln Arg Arg Arg Leu Arg Arg Glu
                50                  55                  60

Arg Val Arg Ala Gln Pro Cys Cys Arg Pro Thr Ala Tyr Glu Asp Glu
            65                  70                  75

Val Ser Phe Leu Asp Ala His Ser Arg Tyr His Thr Val His Glu Leu
        80                  85                  90

Ser Ala Arg Glu Cys Ala Cys Val
    95                  100

<210>  20

<211>  119

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  20

Met Lys Cys Ser Trp Val Ile Phe Phe Leu Met Ala Val Val Thr Gly
                -15                 -10                 -5

Val Asn Ser Leu Gly Ala Arg Pro Cys Gly Leu Arg Glu Leu Glu Val
        -1  1               5                   10

Arg Val Ser Glu Leu Gly Leu Gly Tyr Ala Ser Asp Glu Thr Val Leu
    15                  20                  25

Phe Arg Tyr Cys Ala Gly Ala Cys Glu Ala Ala Ala Arg Val Tyr Asp
30                  35                  40                  45

Leu Gly Leu Arg Arg Leu Arg Gln Arg Arg Arg Leu Arg Arg Glu Arg
                50                  55                  60

Val Arg Ala Gln Pro Cys Cys Arg Pro Thr Ala Tyr Glu Asp Glu Val
            65                  70                  75

Ser Phe Leu Asp Ala His Ser Arg Tyr His Thr Val His Glu Leu Ser
        80                  85                  90

Ala Arg Glu Cys Ala Cys Val
    95                  100

<210>  21

<211>  118

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  21

Met Lys Cys Ser Trp Val Ile Phe Phe Leu Met Ala Val Val Thr Gly
                -15                 -10                 -5

Val Asn Ser Gly Ala Arg Pro Cys Gly Leu Arg Glu Leu Glu Val Arg
        -1  1               5                   10

Val Ser Glu Leu Gly Leu Gly Tyr Ala Ser Asp Glu Thr Val Leu Phe
    15                  20                  25

Arg Tyr Cys Ala Gly Ala Cys Glu Ala Ala Ala Arg Val Tyr Asp Leu
30                  35                  40                  45

Gly Leu Arg Arg Leu Arg Gln Arg Arg Arg Leu Arg Arg Glu Arg Val
                50                  55                  60

Arg Ala Gln Pro Cys Cys Arg Pro Thr Ala Tyr Glu Asp Glu Val Ser
            65                  70                  75

Phe Leu Asp Ala His Ser Arg Tyr His Thr Val His Glu Leu Ser Ala
        80                  85                  90

Arg Glu Cys Ala Cys Val
    95

<210>  22

<211>  117

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  22

Met Lys Cys Ser Trp Val Ile Phe Phe Leu Met Ala Val Val Thr Gly
                -15                 -10                 -5

Val Asn Ser Ala Arg Pro Cys Gly Leu Arg Glu Leu Glu Val Arg Val
        -1  1               5                   10

Ser Glu Leu Gly Leu Gly Tyr Ala Ser Asp Glu Thr Val Leu Phe Arg

15                  20                  25

Tyr Cys Ala Gly Ala Cys Glu Ala Ala Ala Arg Val Tyr Asp Leu Gly
30                  35                  40                  45

Leu Arg Arg Leu Arg Gln Arg Arg Arg Leu Arg Arg Glu Arg Val Arg
                50                  55                  60

Ala Gln Pro Cys Cys Arg Pro Thr Ala Tyr Glu Asp Glu Val Ser Phe
            65                  70                  75

Leu Asp Ala His Ser Arg Tyr His Thr Val His Glu Leu Ser Ala Arg
        80                  85                  90

Glu Cys Ala Cys Val
    95

<210>  23

<211>  116

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  23

Met Lys Cys Ser Trp Val Ile Phe Phe Leu Met Ala Val Val Thr Gly
                -15                 -10                 -5

Val Asn Ser Arg Pro Cys Gly Leu Arg Glu Leu Glu Val Arg Val Ser
        -1  1               5                   10

Glu Leu Gly Leu Gly Tyr Ala Ser Asp Glu Thr Val Leu Phe Arg Tyr
    15                  20                  25

Cys Ala Gly Ala Cys Glu Ala Ala Ala Arg Val Tyr Asp Leu Gly Leu
30                  35                  40                  45

Arg Arg Leu Arg Gln Arg Arg Arg Leu Arg Arg Glu Arg Val Arg Ala
                50                  55                  60

Gln Pro Cys Cys Arg Pro Thr Ala Tyr Glu Asp Glu Val Ser Phe Leu
            65                  70                  75

Asp Ala His Ser Arg Tyr His Thr Val His Glu Leu Ser Ala Arg Glu
        80                  85                  90

Cys Ala Cys Val
    95

<210>  24

<211>  115

<212>  PRT

<213>  artificial sequence


<220>

<223>  mIgSP-hNTN

<220>

<221>  SIGNAL

<222>  (1)..(19)

<223>


<220>

<221>  mat_peptide

<222>  (20)..()

<223>


<400>  24

Met Lys Cys Ser Trp Val Ile Phe Phe Leu Met Ala Val Val Thr Gly
                -15                 -10                 -5

Val Asn Ser Pro Cys Gly Leu Arg Glu Leu Glu Val Arg Val Ser Glu
        -1  1               5                   10

Leu Gly Leu Gly Tyr Ala Ser Asp Glu Thr Val Leu Phe Arg Tyr Cys
    15                  20                  25

Ala Gly Ala Cys Glu Ala Ala Ala Arg Val Tyr Asp Leu Gly Leu Arg
30                  35                  40                  45

Arg Leu Arg Gln Arg Arg Arg Leu Arg Arg Glu Arg Val Arg Ala Gln
                50                  55                  60

Pro Cys Cys Arg Pro Thr Ala Tyr Glu Asp Glu Val Ser Phe Leu Asp
            65                  70                  75

Ala His Ser Arg Tyr His Thr Val His Glu Leu Ser Ala Arg Glu Cys
        80                  85                  90

Ala Cys Val
    95

<210>  25

<211>  120

<212>  PRT

<213>  Homo sapiens


<220>

<221>  SIGNAL

<222>  (1)..(19)

<223>


<220>

<221>  mat_peptide

<222>  (20)..()

<223>


<400>  25

Met Gln Arg Trp Lys Ala Ala Ala Leu Ala Ser Val Leu Cys Ser Ser
                -15                 -10                 -5

Val Leu Ser Arg Leu Gly Ala Arg Pro Cys Gly Leu Arg Glu Leu Glu
        -1  1               5                   10

Val Arg Val Ser Glu Leu Gly Leu Gly Tyr Ala Ser Asp Glu Thr Val
    15                  20                  25

Leu Phe Arg Tyr Cys Ala Gly Ala Cys Glu Ala Ala Ala Arg Val Tyr
30                  35                  40                  45

Asp Leu Gly Leu Arg Arg Leu Arg Gln Arg Arg Arg Leu Arg Arg Glu
                50                  55                  60

Arg Val Arg Ala Gln Pro Cys Cys Arg Pro Thr Ala Tyr Glu Asp Glu
            65                  70                  75

Val Ser Phe Leu Asp Ala His Ser Arg Tyr His Thr Val His Glu Leu
        80                  85                  90

Ser Ala Arg Glu Cys Ala Cys Val
    95                  100

<210>  26

<211>  119

<212>  PRT

<213>  Homo sapiens


<220>

<221>  SIGNAL

<222>  (1)..(19)

<223>


<220>

<221>  mat_peptide

<222>  (20)..()

<223>


<400>  26

Met Gln Arg Trp Lys Ala Ala Ala Leu Ala Ser Val Leu Cys Ser Ser
                -15                 -10                 -5

Val Leu Ser Leu Gly Ala Arg Pro Cys Gly Leu Arg Glu Leu Glu Val
        -1  1               5                   10

Arg Val Ser Glu Leu Gly Leu Gly Tyr Ala Ser Asp Glu Thr Val Leu
    15                  20                  25

Phe Arg Tyr Cys Ala Gly Ala Cys Glu Ala Ala Ala Arg Val Tyr Asp
30                  35                  40                  45

Leu Gly Leu Arg Arg Leu Arg Gln Arg Arg Arg Leu Arg Arg Glu Arg
                50                  55                  60

Val Arg Ala Gln Pro Cys Cys Arg Pro Thr Ala Tyr Glu Asp Glu Val

65                  70                  75

Ser Phe Leu Asp Ala His Ser Arg Tyr His Thr Val His Glu Leu Ser
        80                  85                  90

Ala Arg Glu Cys Ala Cys Val
    95                  100

<210>  27

<211>  118

<212>  PRT

<213>  Homo sapiens

<220>

<221>  SIGNAL

<222>  (1)..(19)

<223>

<220>

<221>  mat_peptide

<222>  (20)..()

<223>

<400>  27

Met Gln Arg Trp Lys Ala Ala Ala Leu Ala Ser Val Leu Cys Ser Ser
                -15                 -10                 -5

Val Leu Ser Gly Ala Arg Pro Cys Gly Leu Arg Glu Leu Glu Val Arg
        -1  1               5                   10

Val Ser Glu Leu Gly Leu Gly Tyr Ala Ser Asp Glu Thr Val Leu Phe
    15                  20                  25

Arg Tyr Cys Ala Gly Ala Cys Glu Ala Ala Ala Arg Val Tyr Asp Leu
30                  35                  40                  45

Gly Leu Arg Arg Leu Arg Gln Arg Arg Arg Leu Arg Arg Glu Arg Val
                50                  55                  60

Arg Ala Gln Pro Cys Cys Arg Pro Thr Ala Tyr Glu Asp Glu Val Ser
            65                  70                  75

Phe Leu Asp Ala His Ser Arg Tyr His Thr Val His Glu Leu Ser Ala
        80                  85                  90

Arg Glu Cys Ala Cys Val
    95

<210>  28

<211>  117

<212>  PRT

<213>  Homo sapiens


<220>

<221>  SIGNAL

<222>  (1)..(19)

<223>


<220>

<221>  mat_peptide

<222>  (20)..()

<223>


<400>  28

Met Gln Arg Trp Lys Ala Ala Ala Leu Ala Ser Val Leu Cys Ser Ser
                -15                 -10                 -5

Val Leu Ser Ala Arg Pro Cys Gly Leu Arg Glu Leu Glu Val Arg Val
        -1  1               5                   10

Ser Glu Leu Gly Leu Gly Tyr Ala Ser Asp Glu Thr Val Leu Phe Arg
    15                  20                  25

Tyr Cys Ala Gly Ala Cys Glu Ala Ala Ala Arg Val Tyr Asp Leu Gly
30                  35                  40                  45

Leu Arg Arg Leu Arg Gln Arg Arg Arg Leu Arg Arg Glu Arg Val Arg
                50                  55                  60

Ala Gln Pro Cys Cys Arg Pro Thr Ala Tyr Glu Asp Glu Val Ser Phe
            65                  70                  75

Leu Asp Ala His Ser Arg Tyr His Thr Val His Glu Leu Ser Ala Arg
        80                  85                  90

Glu Cys Ala Cys Val
    95

<210>  29

<211>  116

<212>  PRT

<213>  Homo sapiens

<220>

<221>  SIGNAL

<222>  (1)..(19)

<223>

<220>

<221>  mat_peptide

<222>  (20)..()

<223>

<400>  29

Met Gln Arg Trp Lys Ala Ala Ala Leu Ala Ser Val Leu Cys Ser Ser
                -15                 -10                 -5

Val Leu Ser Arg Pro Cys Gly Leu Arg Glu Leu Glu Val Arg Val Ser
        -1  1               5                   10

Glu Leu Gly Leu Gly Tyr Ala Ser Asp Glu Thr Val Leu Phe Arg Tyr
    15                  20                  25

Cys Ala Gly Ala Cys Glu Ala Ala Ala Arg Val Tyr Asp Leu Gly Leu
30                  35                  40                  45

Arg Arg Leu Arg Gln Arg Arg Arg Leu Arg Arg Glu Arg Val Arg Ala
                50                  55                  60

Gln Pro Cys Cys Arg Pro Thr Ala Tyr Glu Asp Glu Val Ser Phe Leu
            65                  70                  75

Asp Ala His Ser Arg Tyr His Thr Val His Glu Leu Ser Ala Arg Glu
        80                  85                  90

Cys Ala Cys Val
    95

<210>  30

<211>  115

<212>   PRT

<213>   Homo sapiens

<220>

<221>   SIGNAL

<222>   (1)..(19)

<223>

<220>

<221>   mat_peptide

<222>   (20)..()

<223>

<400>   30

Met Gln Arg Trp Lys Ala Ala Ala Leu Ala Ser Val Leu Cys Ser Ser
                -15                 -10                 -5

Val Leu Ser Pro Cys Gly Leu Arg Glu Leu Glu Val Arg Val Ser Glu
        -1  1               5                   10

Leu Gly Leu Gly Tyr Ala Ser Asp Glu Thr Val Leu Phe Arg Tyr Cys
    15                  20                  25

Ala Gly Ala Cys Glu Ala Ala Ala Arg Val Tyr Asp Leu Gly Leu Arg
30                  35                  40                  45

Arg Leu Arg Gln Arg Arg Arg Leu Arg Arg Glu Arg Val Arg Ala Gln
                50                  55                  60

Pro Cys Cys Arg Pro Thr Ala Tyr Glu Asp Glu Val Ser Phe Leu Asp
            65                  70                  75

Ala His Ser Arg Tyr His Thr Val His Glu Leu Ser Ala Arg Glu Cys
        80                  85                  90

Ala Cys Val
    95

<210>   31

<211>   120

<212>   PRT

<213>   Homo sapiens

<220>

<221>  SIGNAL

<222>  (1)..(18)

<223>


<220>

<221>  mat_peptide

<222>  (19)..()

<223>


<400>  31

Met Ser Met Leu Phe Tyr Thr Leu Ile Thr Ala Phe Leu Ile Gly Ile
            -15                 -10                 -5

Gln Ala Ala Arg Leu Gly Ala Arg Pro Cys Gly Leu Arg Glu Leu Glu
    -1  1               5                   10

Val Arg Val Ser Glu Leu Gly Leu Gly Tyr Ala Ser Asp Glu Thr Val
15                  20                  25                  30

Leu Phe Arg Tyr Cys Ala Gly Ala Cys Glu Ala Ala Ala Arg Val Tyr
                35                  40                  45

Asp Leu Gly Leu Arg Arg Leu Arg Gln Arg Arg Arg Leu Arg Arg Glu
            50                  55                  60

Arg Val Arg Ala Gln Pro Cys Cys Arg Pro Thr Ala Tyr Glu Asp Glu
        65                  70                  75

Val Ser Phe Leu Asp Ala His Ser Arg Tyr His Thr Val His Glu Leu
    80                  85                  90

Ser Ala Arg Glu Cys Ala Cys Val
95                  100

<210>  32

<211>  120

<212>  PRT

<213>  artificial sequence


<220>

<223>  mNGF signal, hNTN mature

<220>

<221>  SIGNAL

<222>  (1)..(18)

<223>


<220>

<221>  mat_peptide

<222>  (19)..()

<223>


<400>  32

Met Ser Met Leu Phe Tyr Thr Leu Ile Thr Ala Phe Leu Ile Gly Val
            -15                 -10                 -5

Gln Ala Ala Arg Leu Gly Ala Arg Pro Cys Gly Leu Arg Glu Leu Glu
    -1  1               5                   10

Val Arg Val Ser Glu Leu Gly Leu Gly Tyr Ala Ser Asp Glu Thr Val
15                  20                  25                  30

Leu Phe Arg Tyr Cys Ala Gly Ala Cys Glu Ala Ala Ala Arg Val Tyr
                35                  40                  45

Asp Leu Gly Leu Arg Arg Leu Arg Gln Arg Arg Arg Leu Arg Arg Glu
            50                  55                  60

Arg Val Arg Ala Gln Pro Cys Cys Arg Pro Thr Ala Tyr Glu Asp Glu
        65                  70                  75

Val Ser Phe Leu Asp Ala His Ser Arg Tyr His Thr Val His Glu Leu
    80                  85                  90

Ser Ala Arg Glu Cys Ala Cys Val
95                  100

<210>  33

<211>  121

<212>  PRT

<213>  Homo sapiens


<220>

<221>  SIGNAL

<222>  (1)..(19)

<223>

<220>
<221>  mat_peptide
<222>  (20)..()
<223>

<400>  33
Met Lys Leu Trp Asp Val Val Ala Val Cys Leu Val Leu Leu His Thr
                -15                 -10                 -5
Ala Ser Ala Ala Arg Leu Gly Ala Arg Pro Cys Gly Leu Arg Glu Leu
        -1  1               5                   10
Glu Val Arg Val Ser Glu Leu Gly Leu Gly Tyr Ala Ser Asp Glu Thr
    15                  20                  25
Val Leu Phe Arg Tyr Cys Ala Gly Ala Cys Glu Ala Ala Ala Arg Val
30                  35                  40                  45
Tyr Asp Leu Gly Leu Arg Arg Leu Arg Gln Arg Arg Arg Leu Arg Arg
                50                  55                  60
Glu Arg Val Arg Ala Gln Pro Cys Cys Arg Pro Thr Ala Tyr Glu Asp
            65                  70                  75
Glu Val Ser Phe Leu Asp Ala His Ser Arg Tyr His Thr Val His Glu
        80                  85                  90
Leu Ser Ala Arg Glu Cys Ala Cys Val
    95                  100

<210>  34
<211>  121
<212>  PRT
<213>  artificial sequence

<220>
<223>  mGDNF signal, hNTN mature
<220>
<221>  SIGNAL
<222>  (1)..(19)

<223>

<220>
<221>  mat_peptide
<222>  (20)..()
<223>

<400>  34
Met Lys Leu Trp Asp Val Val Ala Val Cys Leu Val Leu Leu His Thr
                -15                 -10                 -5

Ala Ser Ala Ala Arg Leu Gly Ala Arg Pro Cys Gly Leu Arg Glu Leu
        -1  1               5                   10

Glu Val Arg Val Ser Glu Leu Gly Leu Gly Tyr Ala Ser Asp Glu Thr
    15                  20                  25

Val Leu Phe Arg Tyr Cys Ala Gly Ala Cys Glu Ala Ala Ala Arg Val
30                  35                  40                  45

Tyr Asp Leu Gly Leu Arg Arg Leu Arg Gln Arg Arg Arg Leu Arg Arg
                50                  55                  60

Glu Arg Val Arg Ala Gln Pro Cys Cys Arg Pro Thr Ala Tyr Glu Asp
            65                  70                  75

Glu Val Ser Phe Leu Asp Ala His Ser Arg Tyr His Thr Val His Glu
        80                  85                  90

Leu Ser Ala Arg Glu Cys Ala Cys Val
    95                  100

<210>  35
<211>  141
<212>  PRT
<213>  Homo sapiens

<220>
<221>  SIGNAL
<222>  (1)..(39)
<223>

<220>

<221>  mat_peptide
<222>  (40)..()
<223>

<400>  35
Met Glu Leu Gly Leu Gly Gly Leu Ser Thr Leu Ser His Cys Pro Trp
                -35                 -30                 -25
Pro Arg Arg Gln Pro Ala Leu Trp Pro Thr Leu Ala Ala Leu Ala Leu
            -20                 -15                 -10
Leu Ser Ser Val Ala Glu Ala Ala Arg Leu Gly Ala Arg Pro Cys Gly
        -5                  -1  1               5
Leu Arg Glu Leu Glu Val Arg Val Ser Glu Leu Gly Leu Gly Tyr Ala
10                  15                  20                  25
Ser Asp Glu Thr Val Leu Phe Arg Tyr Cys Ala Gly Ala Cys Glu Ala
                30                  35                  40
Ala Ala Arg Val Tyr Asp Leu Gly Leu Arg Arg Leu Arg Gln Arg Arg
            45                  50                  55
Arg Leu Arg Arg Glu Arg Val Arg Ala Gln Pro Cys Cys Arg Pro Thr
        60                  65                  70
Ala Tyr Glu Asp Glu Val Ser Phe Leu Asp Ala His Ser Arg Tyr His
    75                  80                  85
Thr Val His Glu Leu Ser Ala Arg Glu Cys Ala Cys Val
90                  95                  100

<210>  36
<211>  123
<212>  PRT
<213>  Homo sapiens

<220>
<221>  SIGNAL
<222>  (1)..(21)
<223>

<220>

<221>  mat_peptide
<222>  (22)..()
<223>

<400>  36

Met Ala Val Gly Lys Phe Leu Leu Gly Ser Leu Leu Leu Leu Ser Leu
    -20                 -15                 -10

Gln Leu Gly Gln Gly Ala Arg Leu Gly Ala Arg Pro Cys Gly Leu Arg
-5                  -1  1               5                   10

Glu Leu Glu Val Arg Val Ser Glu Leu Gly Leu Gly Tyr Ala Ser Asp
            15                  20                  25

Glu Thr Val Leu Phe Arg Tyr Cys Ala Gly Ala Cys Glu Ala Ala Ala
        30                  35                  40

Arg Val Tyr Asp Leu Gly Leu Arg Arg Leu Arg Gln Arg Arg Arg Leu
    45                  50                  55

Arg Arg Glu Arg Val Arg Ala Gln Pro Cys Cys Arg Pro Thr Ala Tyr
60                  65                  70                  75

Glu Asp Glu Val Ser Phe Leu Asp Ala His Ser Arg Tyr His Thr Val
                80                  85                  90

His Glu Leu Ser Ala Arg Glu Cys Ala Cys Val
            95                  100

<210>  37
<211>  19
<212>  PRT
<213>  Homo sapiens

<400>  37

Met Gln Arg Trp Lys Ala Ala Ala Leu Ala Ser Val Leu Cys Ser Ser
1               5                   10                  15

Val Leu Ser

<210>  38
<211>  19
<212>  PRT
<213>  Mus musculus

<400>  38

Met Arg Arg Trp Lys Ala Ala Ala Leu Val Ser Leu Ile Cys Ser Ser
1               5                   10                  15

Leu Leu Ser


<210>  39

<211>  19

<212>  PRT

<213>  Rattus norvegicus


<400>  39

Met Arg Cys Trp Lys Ala Ala Ala Leu Val Ser Leu Ile Cys Ser Ser
1               5                   10                  15

Leu Leu Ser


<210>  40

<211>  18

<212>  PRT

<213>  Homo sapiens


<400>  40

Met Ser Met Leu Phe Tyr Thr Leu Ile Thr Ala Phe Leu Ile Gly Ile
1               5                   10                  15

Gln Ala


<210>  41

<211>  18

<212>  PRT

<213>  Mus musculus


<400>  41

Met Ser Met Leu Phe Tyr Thr Leu Ile Thr Ala Phe Leu Ile Gly Val
1               5                   10                  15

Gln Ala

<210> 42
<211> 19
<212> PRT
<213> Homo sapiens

<400> 42
Met Lys Leu Trp Asp Val Val Ala Val Cys Leu Val Leu Leu His Thr
1               5                   10                  15

Ala Ser Ala

<210> 43
<211> 19
<212> PRT
<213> Mus musculus

<400> 43
Met Lys Leu Trp Asp Val Val Ala Val Cys Leu Val Leu Leu His Thr
1               5                   10                  15

Ala Ser Ala

<210> 44
<211> 19
<212> PRT
<213> Mus musculus

<400> 44
Met Gly Phe Gly Pro Leu Gly Val Asn Val Gln Leu Gly Val Tyr Gly
1               5                   10                  15

Asp Arg Ile

<210> 45
<211> 39
<212> PRT

<213>  Homo sapiens

<400>  45

Met Glu Leu Gly Leu Gly Gly Leu Ser Thr Leu Ser His Cys Pro Trp
1               5                   10                  15

Pro Arg Arg Gln Pro Ala Leu Trp Pro Thr Leu Ala Ala Leu Ala Leu
            20                  25                  30

Leu Ser Ser Val Ala Glu Ala
        35

<210>  46

<211>  21

<212>  PRT

<213>  Homo sapiens

<400>  46

Met Ala Val Gly Lys Phe Leu Leu Gly Ser Leu Leu Leu Leu Ser Leu
1               5                   10                  15

Gln Leu Gly Gln Gly
            20

<210>  47

<211>  220

<212>  PRT

<213>  Homo sapiens

<400>  47

Met Glu Leu Gly Leu Gly Gly Leu Ser Thr Leu Ser His Cys Pro Trp
1               5                   10                  15

Pro Arg Arg Gln Pro Ala Leu Trp Pro Thr Leu Ala Ala Leu Ala Leu
            20                  25                  30

Leu Ser Ser Val Ala Glu Ala Ser Leu Gly Ser Ala Pro Arg Ser Pro
        35                  40                  45

Ala Pro Arg Glu Gly Pro Pro Pro Val Leu Ala Ser Pro Ala Gly His
    50                  55                  60

Leu Pro Gly Gly Arg Thr Ala Arg Trp Cys Ser Gly Arg Ala Arg Arg
65                  70                  75                  80

Pro Pro Pro Gln Pro Ser Arg Pro Ala Pro Pro Pro Pro Ala Pro Pro
                85                  90                  95

Ser Ala Leu Pro Arg Gly Gly Arg Ala Ala Arg Ala Gly Gly Pro Gly
            100                 105                 110

Ser Arg Ala Arg Ala Ala Gly Ala Arg Gly Cys Arg Leu Arg Ser Gln
        115                 120                 125

Leu Val Pro Val Arg Ala Leu Gly Leu Gly His Arg Ser Asp Glu Leu
    130                 135                 140

Val Arg Phe Arg Phe Cys Ser Gly Ser Cys Arg Arg Ala Arg Ser Pro
145                 150                 155                 160

His Asp Leu Ser Leu Ala Ser Leu Leu Gly Ala Gly Ala Leu Arg Pro
                165                 170                 175

Pro Pro Gly Ser Arg Pro Val Ser Gln Pro Cys Cys Arg Pro Thr Arg
            180                 185                 190

Tyr Glu Ala Val Ser Phe Met Asp Val Asn Ser Thr Trp Arg Thr Val
        195                 200                 205

Asp Arg Leu Ser Ala Thr Ala Cys Gly Cys Leu Gly
    210                 215                 220

<210>  48

<211>  156

<212>  PRT

<213>  Homo sapiens


<400>  48

Met Ala Val Gly Lys Phe Leu Leu Gly Ser Leu Leu Leu Leu Ser Leu
1               5                   10                  15

Gln Leu Gly Gln Gly Trp Gly Pro Asp Ala Arg Gly Val Pro Val Ala
            20                  25                  30

Asp Gly Glu Phe Ser Ser Glu Gln Val Ala Lys Ala Gly Gly Thr Trp
        35                  40                  45

Leu Gly Thr His Arg Pro Leu Ala Arg Leu Arg Arg Ala Leu Ser Gly
    50                  55                  60

Pro Cys Gln Leu Trp Ser Leu Thr Leu Ser Val Ala Glu Leu Gly Leu
65                  70                  75                  80

Gly Tyr Ala Ser Glu Glu Lys Val Ile Phe Arg Tyr Cys Ala Gly Ser
                85                  90                  95

Cys Pro Arg Gly Ala Arg Thr Gln His Gly Leu Ala Leu Ala Arg Leu
            100                 105                 110

Gln Gly Gln Gly Arg Ala His Gly Gly Pro Cys Cys Arg Pro Thr Arg
        115                 120                 125

Tyr Thr Asp Val Ala Phe Leu Asp Asp Arg His Arg Trp Gln Arg Leu
    130                 135                 140

Pro Gln Leu Ser Ala Ala Ala Cys Gly Cys Gly Gly
145                 150                 155

<210>  49

<211>  211

<212>  PRT

<213>  Homo sapiens


<400>  49

Met Lys Leu Trp Asp Val Val Ala Val Cys Leu Val Leu Leu His Thr
1               5                   10                  15

Ala Ser Ala Phe Pro Leu Pro Ala Gly Lys Arg Pro Pro Glu Ala Pro
            20                  25                  30

Ala Glu Asp Arg Ser Leu Gly Arg Arg Arg Ala Pro Phe Ala Leu Ser
        35                  40                  45

Ser Asp Ser Asn Met Pro Glu Asp Tyr Pro Asp Gln Phe Asp Asp Val
    50                  55                  60

Met Asp Phe Ile Gln Ala Thr Ile Lys Arg Leu Lys Arg Ser Pro Asp
65                  70                  75                  80

Lys Gln Met Ala Val Leu Pro Arg Arg Glu Arg Asn Arg Gln Ala Ala
                85                  90                  95

Ala Ala Asn Pro Glu Asn Ser Arg Gly Lys Gly Arg Arg Gly Gln Arg
            100                 105                 110

Gly Lys Asn Arg Gly Cys Val Leu Thr Ala Ile His Leu Asn Val Thr
        115                 120                 125

Asp Leu Gly Leu Gly Tyr Glu Thr Lys Glu Glu Leu Ile Phe Arg Tyr
    130                 135                 140

Cys Ser Gly Ser Cys Asp Ala Ala Glu Thr Thr Tyr Asp Lys Ile Leu
145                 150                 155                 160

Lys Asn Leu Ser Arg Asn Arg Arg Leu Val Ser Asp Lys Val Gly Gln
                165                 170                 175

Ala Cys Cys Arg Pro Ile Ala Phe Asp Asp Asp Leu Ser Phe Leu Asp
            180                 185                 190

Asp Asn Leu Val Tyr His Ile Leu Arg Lys His Ser Ala Lys Arg Cys
        195                 200                 205

Gly Cys Ile
    210

<210>  50

<211>  602

<212>  DNA

<213>  Homo sapiens


<220>

<221>  CDS

<222>  (35)..(571)

<223>


<220>

<221>  sig_peptide

<222>  (35)..(91)

<223>  GDNF signal peptide


<220>

<221>  misc_feature

<222>  (92)..(265)

<223>  GDNF pro-region


<220>

<221>  mat_peptide

<222>  (266)..()

<223>  NTN mature peptide

<400>  50
gatccactag tccagtgtgg tggaattcgc cacc atg aag tta tgg gat gtc gtg      55
                                      Met Lys Leu Trp Asp Val Val
                                                  -75

gct gtc tgc ctg gtg ctg ctc cac acc gcg tcc gcc ttc ccg ctg ccc      103
Ala Val Cys Leu Val Leu Leu His Thr Ala Ser Ala Phe Pro Leu Pro
-70                 -65                 -60                 -55

gcc ggt aag agg cct ccc gag gcg ccc gcc gaa gac cgc tcc ctc ggc      151
Ala Gly Lys Arg Pro Pro Glu Ala Pro Ala Glu Asp Arg Ser Leu Gly
                -50                 -45                 -40

cgc cgc cgc gcg ccc ttc gcg ctg agc agt gac tca aat atg cca gag      199
Arg Arg Arg Ala Pro Phe Ala Leu Ser Ser Asp Ser Asn Met Pro Glu
            -35                 -30                 -25

gat tat cct gat cag ttc gat gat gtc atg gat ttt att caa gcc acc      247
Asp Tyr Pro Asp Gln Phe Asp Asp Val Met Asp Phe Ile Gln Ala Thr
        -20                 -15                 -10

att aaa aga ctg aaa agg gcg cgg ttg ggg gcg cgg cct tgc ggg ctg      295
Ile Lys Arg Leu Lys Arg Ala Arg Leu Gly Ala Arg Pro Cys Gly Leu
    -5                  -1  1               5                   10

cgc gag ctg gag gtg cgc gtg agc gag ctg ggc ctg ggc tac gcg tcc      343
Arg Glu Leu Glu Val Arg Val Ser Glu Leu Gly Leu Gly Tyr Ala Ser
                15                  20                  25

gac gag acg gtg ctg ttc cgc tac tgc gca ggc gcc tgc gag gct gcc      391
Asp Glu Thr Val Leu Phe Arg Tyr Cys Ala Gly Ala Cys Glu Ala Ala
            30                  35                  40

gcg cgc gtc tac gac ctc ggg ctg cga cga ctg cgc cag cgg cgg cgc      439
Ala Arg Val Tyr Asp Leu Gly Leu Arg Arg Leu Arg Gln Arg Arg Arg
        45                  50                  55

ctg cgg cgg gag cgg gtg cgc gcg cag ccc tgc tgc cgc ccg acg gcc      487
Leu Arg Arg Glu Arg Val Arg Ala Gln Pro Cys Cys Arg Pro Thr Ala
    60                  65                  70

tac gag gac gag gtg tcc ttc ctg gac gcg cac agc cgc tac cac acg      535
Tyr Glu Asp Glu Val Ser Phe Leu Asp Ala His Ser Arg Tyr His Thr
75                  80                  85                  90

gtg cac gag ctg tcg gcg cgc gag tgc gcc tgc gtg tgacatatca           581
Val His Glu Leu Ser Ala Arg Glu Cys Ala Cys Val
                95                  100

agcttatcga taccgtcgac c                                              602

<210>  51

<211>  179

<212>  PRT

<213>  Homo sapiens


<220>

<221>  misc_feature

<222>  (92)..(265)

<223>  GDNF pro-region

<400>  51

Met Lys Leu Trp Asp Val Val Ala Val Cys Leu Val Leu Leu His Thr
        -75                 -70                 -65

Ala Ser Ala Phe Pro Leu Pro Ala Gly Lys Arg Pro Pro Glu Ala Pro
    -60                 -55                 -50

Ala Glu Asp Arg Ser Leu Gly Arg Arg Arg Ala Pro Phe Ala Leu Ser
-45                 -40                 -35                 -30

Ser Asp Ser Asn Met Pro Glu Asp Tyr Pro Asp Gln Phe Asp Asp Val
                -25                 -20                 -15

Met Asp Phe Ile Gln Ala Thr Ile Lys Arg Leu Lys Arg Ala Arg Leu
            -10                 -5                  -1  1

Gly Ala Arg Pro Cys Gly Leu Arg Glu Leu Glu Val Arg Val Ser Glu
    5                   10                  15

Leu Gly Leu Gly Tyr Ala Ser Asp Glu Thr Val Leu Phe Arg Tyr Cys
20                  25                  30                  35

Ala Gly Ala Cys Glu Ala Ala Ala Arg Val Tyr Asp Leu Gly Leu Arg
                40                  45                  50

Arg Leu Arg Gln Arg Arg Arg Leu Arg Arg Glu Arg Val Arg Ala Gln
            55                  60                  65

Pro Cys Cys Arg Pro Thr Ala Tyr Glu Asp Glu Val Ser Phe Leu Asp
        70                  75                  80

Ala His Ser Arg Tyr His Thr Val His Glu Leu Ser Ala Arg Glu Cys
    85                  90                  95

Ala Cys Val
100

<210>  52

<211>  29

<212>  DNA

<213>  artificial sequence

<220>

<223>  PCR-primer

<400>  52
tataggatcc ggaggacacc agcatgtag                                    29

<210> 53

<211> 20

<212> DNA

<213> artificial sequence

<220>

<223> PCR primer

<400> 53
tcgccgagga tgaatcacca 20

<210> 54

<211> 31

<212> DNA

<213> artificial sequence

<220>

<223> PCR primer

<400> 54
ggtgaattcg gcgcggttgg gggcgcggcc t 31

<210> 55

<211> 30

<212> DNA

<213> artificial sequence

<220>

<223> PCR primer

<400> 55
tatactcgag tcacacgcag gcgcactcgc 30

<210> 56

<211> 37

<212> DNA

<213> artificial sequence

<220>

<223> PCR primer

<400>  56
tataggatcc gccaccatga aatgcagctg ggttatc                                37

<210>  57

<211>  31

<212>  DNA

<213>  artificial sequence


<220>

<223>  PCR primer

<400>  57
ccaaccgcgc cgaattcacc cctgtagaaa g                                      31

<210>  58

<211>  35

<212>  DNA

<213>  artificial sequence


<220>

<223>  PCR primer

<400>  58
tatagaattc gccaccatga agttatggga tgtcg                                  35

<210>  59

<211>  30

<212>  DNA

<213>  artificial sequence


<220>

<223>  PCR primer

<400>  59
ccaaccgcgc ccttttcagt cttttaatgg                                        30

<210>  60

<211>  31

<212>  DNA

<213>  artificial sequence


<220>

<223>  PCR primer

<400>  60
actgaaaagg gcgcggttgg gggcgcggcc t                                    31

<210>  61

<211>  22

<212>  DNA

<213>  artificial sequence


<220>

<223>  PCR primer

<400>  61
tagactcgag gtcgacggta tc                                              22

<210>  62

<211>  91

<212>  DNA

<213>  artificial sequence


<220>

<223>  PCR primer

<400>  62
tataggatcc gccaccatgc agcgctggaa ggcggcggcc ttggcctcag tgctctgcag     60

ctccgtgctg tccgcgcggt tgggggcgcg g                                    91

## Description

The present application claims priority from Danish patent application No. DK PA 2003 01543 filed on 20 October 2003. It claims the benefit of US provisional application No. 60/512,918 filed on 22 October 2003. All references cited in those applications and in the present application are hereby incorporated by reference in their entirety.

### FIELD OF INVENTION

The present invention concerns methods and compositions for gene therapy, in particular in vivo gene therapy for delivery of bioactive Neurturin for the treatment of Parkinson's Disease. In another aspect the invention relates to virus expression constructs comprising a mammalian signal peptide linked to a mature or N-terminally truncated Neurturin without a functional pro-region between the signal peptide and the Neurturin. These viral expression constructs are required for efficient secretion of bioactive Neurturin in in vivo gene therapy. The invention also concerns mammalian cells capable of producing Neurturin in increased amounts as well as the use of these cells for recombinant production of bioactive Neurturin and for therapeutic use.

### BACKGROUND OF THE INVENTION

Parkinson's disease (PD) is a devastating neurodegenerative disorder that afflicts between 1 and 1.5 million Americans. Over 35,000 new cases are diagnosed each year. The incidence of Parkinson's disease is highest in the over-50 age group, although an alarming number of new cases have been reported in younger patients.

The cardinal features of Parkinson's disease are slowness of movement (bradykinesia), a tremor or trembling in the hands, arms, legs, jaw, and face, stiffness of the limbs and trunk, and postural instability. As these symptoms progress, patients may experience difficulty walking, talking, or completing other simple daily living tasks. These behavioural deficits are linked to the degeneration of the nigrostriatal system in the brain, which is responsible for the production of smooth, purposeful movements. Specifically, nerve cells located in the substantia nigra degenerate and there is an accompanying loss of dopamine that is made by these cells. The substantia nigra nerve cells extend axons or processes to the striatum, where the dopamine is secreted and utilized. It has been estimated that an 80% loss of dopamine within the striatum needs to occur before the symptoms of PD emerge.

At present, levodopa (trade name Sinemet) is the mainstay treatment for Parkinson's disease. In the brain, levadopa is converted to dopamine, which corrects the dopamine deficiency in the brains of patients with Parkinson's disease. When levodopa is administered in combination with the peripheral decarboxylase inhibitor carbidopa, PD patients experience dramatic benefits. The problem, however, is that while levodopa therapy diminishes the symptoms of PD, it does not replace lost nerve cells and does not halt the progression of the disease. As PD progresses, patients require increasing doses of levodopa and side effects, most notably disabling involuntary movements and rigidity, may emerge. In fact, movement disorder specialists often delay the use of levodopa and initially use other dopaminergic drugs in order to save the use of levodopa for later on in the disease process when patients need it the most.

Thus, levodopa has its limitations and additional therapeutic strategies for Parkinson's disease need to be established. In this regard, interest in surgical treatments for PD has been rekindled. Recently, a procedure called deep brain stimulation has gained considerable attention. In this procedure, electrodes are placed in brain regions that are overactive in PD so that electrical stimulation of these brain regions corrects the overactivity. In some patients, dramatic benefits can be achieved. Other surgical interventions are aimed at improving the function of the nigrostriatal system. Transplants of dopaminergic cells have been successful in ameliorating motor deficits in animal models of Parkinson's disease. Initial clinical trials of dopaminergic cell transplantation in humans have been successful, while a single double blind clinical trial revealed benefit in younger, but not older patients. However, some of the patients receiving grafts developed disabling involuntary movements. Thus, at present, cellular transplants should still be considered an experimental approach.

Another approach aims to deliver growth factors into the nigrostriatal system in an attempt to prevent the degeneration of substantia nigra neurons and the accompanying loss of the neurotransmitter dopamine.

Many laboratories across the world have demonstrated that Glial Cell-line Derived Neurotrophic Factor (GDNF) can prevent the structural and functional consequences of degeneration of the nigrostriatal system in studies conducted in rats and primates. Delivery of GDNF to the CNS has been achieved in pre-clinical studies using protein injections, delivery via pumps and by in vivo gene therapy. Numerous studies describe transduction of CNS cells using AAV or lentivirus expressing GDNF (Kordower, Ann Neurol, 2003 53 (suppl 3), s120-s134; WO 03/018821, Ozawa et al; US 2002187951, Aebischer et al; Georgievska et al 2002, Exp Nerol 117(2), 461-474; Georgievska et al 2002, NeuroReport 13(1), 75-82; Wang et al, 2002, Gene Therapy, 9(6), 381-389; US 2002031493, Rohne-Poulenc Rorer SA; US 6,180,613 Roeckefeller University; Kozlowski et al 2000, Exp Neurol, 166(1), 1,15; Bensadoun 2000, Exp Neurol, 164(1), 15-24; Connor et al 1999, Gene Therapy, 6(12), 1936-1951; Mandel et al 1997, PNAS, 94(25), 14083-88; Lapchak et al 1997, Brain Research, 777 (1,2), 153-160; Bilang-Bleuel et al 1997, PNAS 94(16), 8818-8823).

Other in vivo gene therapy approaches to the treatment of Parkinson's Disease include transduction with virus expressing aromatic L-amino acid decarboxylase (AADC), suthalamic glutamic acid decarboxylase (GAD) (Marutso, Nippon Naika Gakkai Zasshi, 2003, 92 (8), 1461-1466; Howard, Nature Biotechnology, 2003, 21 (10), 1117-18).

Although GDNF seems to be a promising candidate for treatment of Parkinson's Disease in human beings, GDNF treatment is reported to result in certain side-effects, mainly weight-loss and allodynia (Hoane et al 1999, 160(1):235-43). Therefore there is a need in the art for developing alternative strategies for treatment of Parkinson's Disease in particular strategies that aim at preventing the degeneration of substantia nigra neurons.

Neurturin is a member of the GDNF family of growth receptors and signals primarily through the GFRα2 receptor. The receptors for NTN and GDNF are differentially expressed both spatially and temporally. Therefore it must be expected that the therapeutic effect of NTN and GDNF are different. This is confirmed in a comparison of GDNF and NTN delivered via osmotic minipumps (Hoane et al 1999, 160(1):235-43). In a comparison of GDNF and NTN in an ex vivo gene therapy study with a 6-hydroxydopamine-induced degeneration of adult dopaminergic neurons (the same animal model as used by the present inventors), the authors noted that both GDNF and NTN prevented death of nigral dopaminergic neurons, but only GDNF induced increased intensity of tyrosine hydroxylase staining and massive sprouting (Åkerud et al, J Neurochemistry, 73, 1:70-78).

Delivery of Neurturin as a protein formulation or by transplanting NTN-overexpressing cells to the CNS in PD models has not yielded results comparable to the results obtained with GDNF. In the protein treatment studies, this could be due to problems with stability of Neurturin protein formulations (precipitation and short half-life).

To date there are no reports on in vivo gene therapy of Parkinson's Disease using Neurturin. One gene therapy study using Neurturin expressing AAV focussed on the treatment of retinal disorders (Jomary et al 2000, Molecular Vision 7:36-41). However, the authors concluded that therapeutic treatment of the rd model of retinal degradation did not appear to be effected by simple modulation of the expression of NTN or GFRα-2. Therefore until today in vivo gene therapy using Neurturin has remained speculative.

### SUMMARY OF THE INVENTION

The present inventors have performed a series of pre-clinical animal studies based on virus transduction delivery of GDNF family growth factors to the striatum in a 6-OHDA lesion model. The 6-OHDA lesion model is a well known animal model for Parkinson's Disease. These experiments have - surprisingly - shown that transduction with a virus coding for Neuturin results in a therapeutical effect which is at least as good as the effect obtained through transduction with GDNF. These data represent the first pre-clinical evidence of the effect of in vivo gene therapy delivery of Neurturin in a Parkinson's Disease model.

The specificity of Neurturin as a preferred neurotrophic factor for the treatment of Parkinson's Disease is confirmed by the absence of any effect of another neurotrophic factor, Neublastin (Artemin), in the 6-OHDA lesion model.

Consequently, in a first aspect the invention relates to a method for treatment of Parkinson's Disease, said method comprising administering to the central nervous system of an individual in need thereof a therapeutically effective amount of a viral expression vector, said vector comprising a promoter sequence capable of directing the expression of an operably linked polypeptide, said polypeptide comprising a signal peptide capable of functioning in a mammalian cell, and a human, murine or rat Neurturin (NTN) selected from the group consisting of pro-NTN, mature NTN, N-terminally truncated mature NTN, and a sequence variant of any such NTN.

The present inventors have also determined that in certain cases there may be a problem with secretion of Neurturin when using a construct coding for pre-pro-Neurturin. Both in vitro transfection and transduction and in vivo transduction with human or mouse pre-pro-Neurturin as described in the appended examples resulted in very limited secretion of Neurturin. Under the same conditions using the same vectors and the same cells for transduction or transfection, GDNF was secreted in significant quantities.

One way of obtaining significantly higher secretion levels has been contemplated by the present inventors. This way includes the deletion of that part of the Neurturin gene, which codes for the pro-peptide. The expression construct according to this embodiment includes a signal peptide fused directly to the mature or a truncated form of Neurturin or a sequence variant of mature or truncated Neurturin. The signal peptide may be the native Neurturin signal peptide or a heterologous signal peptide.

Another way of obtaining sufficiently higher secretion levels includes replacement of the native Neuturin signal peptide with a heterologous signal peptide, including but not limited to the Immunoglobulin heavy chain Signal Peptide (IgSP). In a particularly preferred embodiment, the heterologous signal peptide is fused to a deltapro-NTN. By deltapro-Neurturin is intended a Neurturin polypeptide without a functional proregion.

In contrast to the findings with Neurturin, a deletion of the pro-part of GDNF and replacement of the GDNF signal peptide with IgSP signal peptide resulted in a significant reduction in secretion of GDNF compared to secretion from pre-pro-GDNF.

It was also observed that replacement of the pre-pro part of NTN with the pre-pro part of GDNF did not result in any significant increase in secretion of NTN. Thus the difference between NTN and GDNF cannot solely be ascribed to differences in the pre-pro part of the two neurotrophic factors.

Both in the case of pre-pro-NTN and the expression construct with the pre-pro part of GDNF it was observed that a protein corresponding in molecular weight to an unprocessed pro-NTN was secreted from the mammalian cells. It was also observed that this pro-NTN was not able to bind to either GFRα1 or GFRα2. When "pro-less" expression constructs were used, a protein corresponding in molecular weight to mature Neurturin was secreted. This protein was bioactive as evidenced by the in vivo experiments and was able bind to both GFRα1 and GFRα2.

One important advantage of using the high efficiency expression constructs described in the present invention is that the amount of virus composition administered to the patient can be decreased. Thus for the same therapeutic effect, less virus composition need to be produced and through the administration of less volume of composition, less side effects may be expected. Furthermore the injections can be made more precisely.

In another aspect the invention relates to use of a viral expression vector, said vector comprising a promoter sequence capable of directing the expression of an operably linked polypeptide, said polypeptide comprising a signal peptide capable of functioning in a mammalian cell, and a human, murine or rat Neurturin (NTN) selected from the group consisting of pro-NTN, mature NTN, N-terminally truncated mature NTN, and a sequence variant of any such NTN; for the preparation of a medicament for the treatment of Parkinson's Disease.

In a further aspect the invention relates to a viral expression vector comprising a polynucleotide sequence comprising a promoter sequence capable of directing the expression of an operably linked polypeptide, said polypeptide comprising a signal peptide capable of functioning in a mammalian cell, and a Neurturin selected from the group consisting of mature NTN, N-terminally truncated mature NTN, and a sequence variant of mature or N-terminally truncated NTN. Such a viral expression vector is characterised by the absence of a functional Neurturin pro-region.

This viral expression vector is especially useful for in vivo gene therapy because it results in the expression and secretion of therapeutically relevant quantities of Neurturin. The viral expression vector may also be used for generating mammalian cells secreting high amounts of bioactive Neurturin.

In a further aspect the invention relates to a pharmaceutical composition comprising the vector according to the invention and one or more pharmaceutically acceptable adjuvants, excipients, carriers and/or diluents. The pharmaceutical composition can be used for in vivo and ex vivo gene therapy.

In a further aspect the invention relates to an isolated host cell transduced with the vector according to the invention.

Such transduced host cells have turned out to produce unexpected high amounts of Neurturin compared known to NTN-producing cells and compared to cells transduced with viral vectors encoding Neurturin with an intact propeptide. The transduced host cells of the present invention therefore constitute a promising source of cells for the industrial scale production of Neurturin.

In a further aspect the invention relates to a packaging cell line capable of producing an infective vector particle, said vector particle comprising a retrovirally derived genome comprising a 5' retroviral LTR, a tRNA binding site, a packaging signal, a promoter operably linked to a polynucleotide sequence encoding a fusion protein comprising Neurturin and a heterologous signal peptide, an origin of second strand DNA synthesis and a 3' retroviral LTR. The fusion protein does not comprise a functional Neurturin pro-region.

These packaging cell lines can be used for producing the viral vectors according to the invention. They can also be used for in vivo gene therapy when encapsulated and transplanted to the CNS.

In a further aspect the invention relates to a chimeric non-human mammal comprising at least one cell being transduced with the vector according to the invention. Such animals which overexpress Neurturin can be used for gene profiling and in the screening and development of drugs.

Preferably the transduced cell has the genotype of the individual animal, i.e. is not an allogeneic or xenogeneic transplant.

In a further aspect the invention relates to an implantable cell culture device, the device comprising:
a semipermeable membrane permitting the diffusion of Neurturin therethrough; and
at least one isolated host cell according to the invention.

These capsules can be used for the local delivery of Neurturin upon transplantation into the central nervous system. Localised and prolonged delivery of growth factor is a preferred administration method for the treatment of a number of CNS disorders, including but not limited to Parkinson's disease, Alzheimer's disease, Huntington's disease, stroke, and amyotrophic lateral sclerosis (ALS).

In a further aspect the invention relates to a biocompatible capsule comprising: a core comprising living packaging cells that secrete a viral vector for infection of a target cell, wherein the viral vector is a vector according to the invention; and an external jacket surrounding said core, said jacket comprising a permeable biocompatible material, said material having a porosity selected to permit passage of retroviral vectors of approximately 100 nm diameter thereacross, permitting release of said viral vector from said capsule.

The capsules of this invention provide for the delivery of viral particles to a desired site in a patient using a capsular approach. Encapsulation of vector-producing cell lines permits continuous delivery of the viral particle to the target site, as opposed to a single infusion. In addition, repeat therapy is possible, with reduced likelihood of immune attack. The capsules have pores large enough to allow passage of viral particles released from the packaging cells, yet prevent host-cell passage into the capsule.

This capsular approach increases the safety and control of the therapy because the devices can easily be retrieved (terminating the treatment) or explanted and reimplanted (modifying the treatment). Further, the chance of infection is reduced because the capsular device is not open or externalised.

Finally, because encapsulation prevents the packaging cells from migrating within the patient, and prolongs the viability of the packaging cells upon implant, fewer cells are likely to be needed for this therapy. This may be advantageous in further lowering an immune reaction in the patient.

In a further aspect the invention relates to use of the virus vector according to the invention as a medicament.

In a still further aspect the invention relates to use of the virus vector according to the invention for the preparation of a medicament for the treatment of a nervous system disorder.

In another aspect the invention relates to the use of the vector according to the invention for the preparation of a medicament for the treatment of a CNS disorder.

Furthermore, the invention relates to a method of treating a nervous system disease, said method comprising administering to an individual in need thereof:
a therapeutically effective amount of the vector of the invention; or
a therapeutically effective amount of the pharmaceutical composition of the invention; or
a biocompatible device comprising a packaging cell line according to the invention.

According to this aspect of the invention there is provided improved in vivo gene therapy methods for the treatment of nervous system diseases. As evidenced by the appended examples, in vivo transduction with the viral vectors of the present invention results in hitherto unseen secretion and tissue distribution of the encoded therapeutic factors. e.g. Neurturin, and as a consequence improved therapeutic effect.

In a still further aspect the invention relates to a method of treating a nervous system disease, said method comprising transplanting to an individual in need thereof:
i. a therapeutically effective amount of the transduced cells of the invention; or
ii. an implantable device according to the invention.

This aspect provides another way of treating nervous system disorders based on ex vivo gene therapy and implantation of therapeutic cells capable of secreting increased amounts of the Neurturin.

In a further aspect the invention relates to a mammalian cell capable of secreting neurturin or a functional equivalent thereof in amounts in excess of 500 ng/10⁶ cells/24 hours.

The Neurturin-producing cells described in the present invention produce Neuturin in amounts exceeding that seen in prior art mammalian cells by at least one order of magnitude. The Neurturin producing cells of the present invention make it feasible to produce the protein in fermenters using mammalian cells with the advantage that the protein is correctly processed, glycosylated, and folded and can be recovered easily from the culture medium.

### BRIEF DESCRIPTION OF THE DRAWINGS

Figure 1: Alignment of IgSP sequences from various mammals. Human IgSP (SEQ ID No. 1; Genbank # AASC18285); Rhesus Monkey IgSP (SEQ ID No. 2; Genbank # AAC02637); Marmoset IgSP (SEQ ID No. 3; Genbank #AAM89745); Mouse IgSP (SEQ ID No. 4; Genbank # AAA38502); Pig IgSP (SEQ ID No. 5; Genbank # AAA79743); Rat IgSP (SEQ ID No. 6; Genbank # AAA51349);

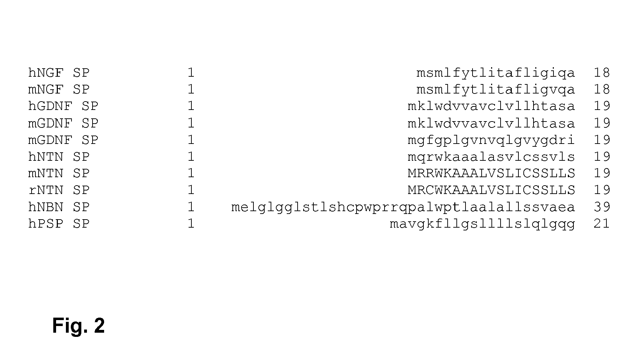

Figure 2: A table of signal sequences from various neurotrophic factors. Human Nerve Growth Factor (hNGF, Genbank # NP_002497; SEQ ID No 40); mouse Nerve Growth Factor (mNGF, Genbank # P01139; SEQ ID No 41); human GDNF (hGDNF, Genbank # NP_000505; SEQ ID No 42), mouse GDNF (mGDNF, SEQ ID No. 43; Genbank accession no U36449); a putative mouse GDNF signal sequence (SEQ ID No 44; N-terminal 19 amino acids from Genbank NM_010275; the start codon seems to be wrongly predicted compated to U36449); human Neublastin (hNBN, Genbank # NP_476501; SEQ ID No 45); human Persephin (hPSP, Genbank # NP_004149; SEQ ID No. 46); human Neurturin (SEQ ID No. 37); mouse Neurturin (SEQ ID No. 38); rat Neurturin (SEQ ID No. 39).
Figure 3: pNS1 nIgSP.NTN plasmid map
Figure 4: Sandwich ELISA of Neurturin produced in vitro from cells transfected with constructs encoding wild type NTN (SEQ ID No 12), NTN with GDNF pre-pro peptide (SEQ ID No. 51), and NTN with IgSP (SEQ ID No 18).
Figure 5: A fuctional RetL2 ELISA assay of Neurturin produced in vitro from cells transfected with constructs encoding wild type NTN (SEQ ID No 12), NTN with GDNF pre-pro peptide (SEQ ID No. 51), and NTN with IgSP (SEQ ID No. 18).
Figure 6: Western blots of preparations of Neurturin from lysates of cells transfected with constructs encoding wild type NTN (SEQ ID No. 12), NTN with GDNF pre-pro peptide (SEQ ID No. 51), and NTN with IgSP (SEQ ID No. 18).
Figure 7: Quantification of the amount of Neurturin produced by ARPE-19 cells stably expressing Neurturin.
Figure 8: pHR'-sC.IgSP-hgNTN.W vector map, the lentivirus vector used for the in vivo gene therapy studies.
Figure 9: Schematic drawing of Intrastriatal injection of lentiviral vector or 6-OHDA.
Figure 10: Coronal sections through the striatum of rats transduced with lentiviral vectors and subsequently processed for immunohistochemistry using antibodies to human NTN or Human GDNF.
   Upper left panel shows an intact, non-operated striatum processed for hNTN. No signal can be detected. Similarly, to the intact side no signal is detected in the rLV-wtNTN transduced striatum (upper right panel). By contrast, a marked specific staining for hNTN is seen in the rLV-IgSP transduced striatum (lower right panel) and the diffuse staining pattern closely resembles that observed in rLV-GDNF transduced animals stained with GDNF-immunohistochemistry (lower left panel).

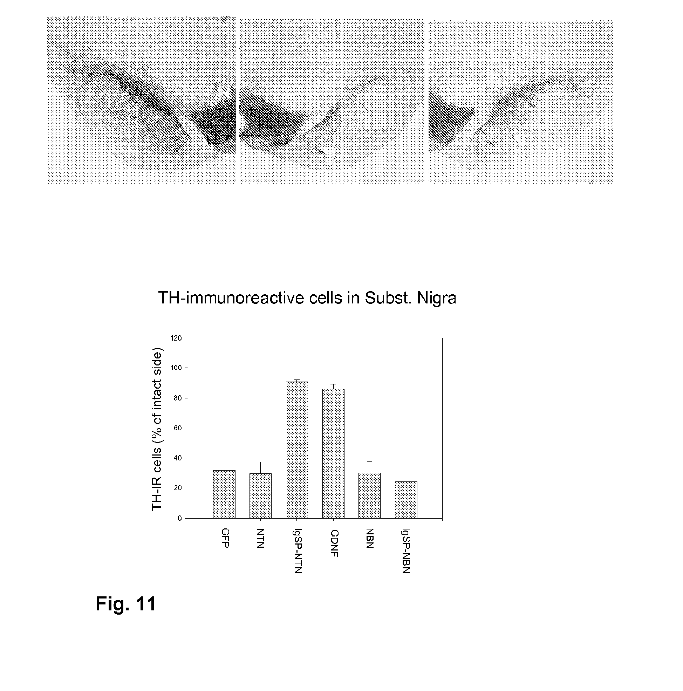

Figure 11: Neuroprotection of nigral dopamine neurons by rLV-IgSPNTN. On the intact side many TH-immunoreactive cells can be seen in the substantia nigra (indicative of dopaminergic cells)(left panel, upper row). On the side subject to a 6-OHDA lesion the number of remaining TH-immunoreactive neuronal profiles are significantly reduced in wtNTN treated animal (middle panel, upper row). By contrast, many more TH-immunoreactive neurons remain in he animals receiving IgSP-NTN treatment (right panel, upper row). Quantification (bar plot) show that IgSP-NTN induce a significant rescue from the toxic insult similar to the effect seen by GDNF treatment. NBN, previously shown not to rescue nigral dopamine neurons in vivo does not protect when fused to an IgSP.

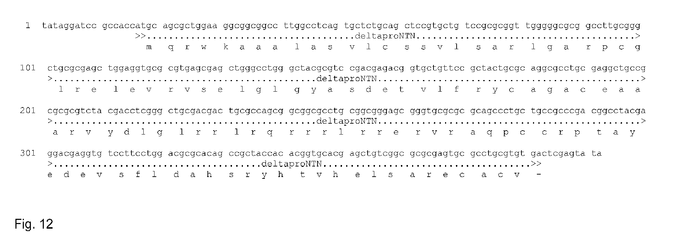

Figure 12: DNA insert (SEQ ID No. 15) and encoded polypeptide (SEQ ID No. 16) from example 1 and 3 showing deltaproNeurturin expression construct.

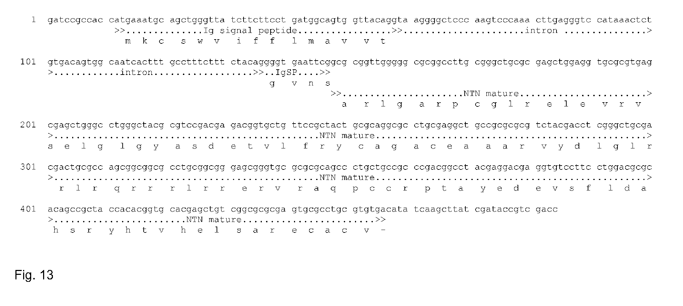

Figure 13: DNA insert (SEQ ID No. 17) and encoded polypeptide (SEQ ID No 18) from example 1 and 3 showing IgSP-Neurturin expression construct.

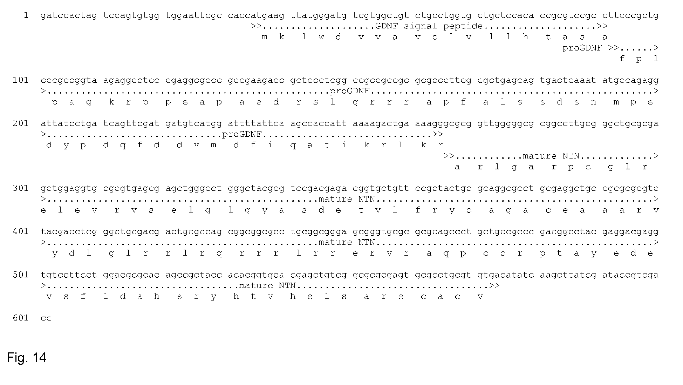

Figure 14: DNA insert (SEQ ID No. 50) and encoded polypeptide (SEQ ID No. 51) from examples 1 and 3 showing preproGDNF-Neurturin expression construct.

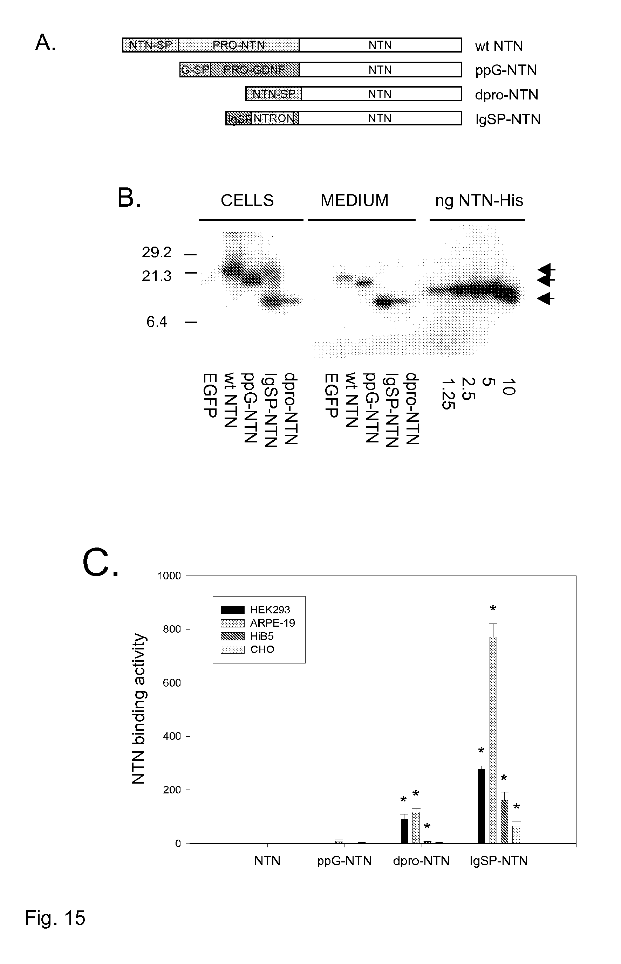

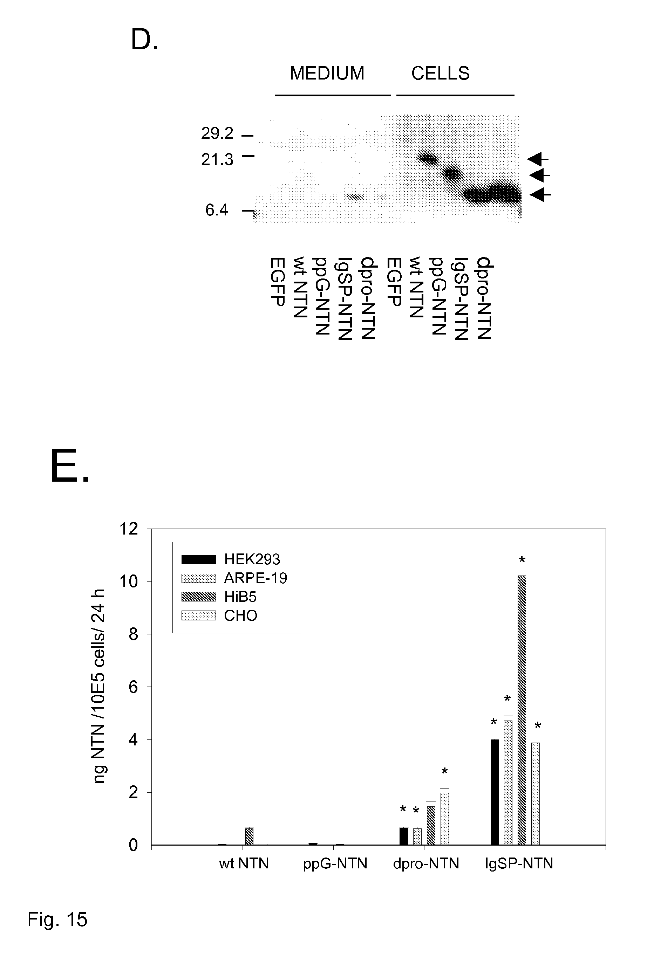

Figure 15: (A) NTN expression constructs including wt pre-pro NTN, NTN with the pre-pro part from GDNF (ppG-NTN, SEQ ID No. 50)), dpro-NTN (SEQ ID No 15) and IgSP-NTN (SEQ ID No 17). The latter DNA sequence contains an intron. See text for further details. (B) NTN Western blot of lysates and conditioned medium from transfected HEK293 cells. Arrows indicate bands with the size of wt pro-NTN, pro(GDNF)-NTN and mature NTN, respectively. Note that the standard is NTN-His which has a slightly higher molecular weight than wt NTN. (C) GFRα2 binding activity of NTN in conditioned medium from four different cell lines transfected with the NTN constructs. (D) NTN Western blot of lysates (not bound to GFRα2), and NTN from conditioned medium bound to GFRα2. (E) NTN sandwich ELISA on conditioned medium from the four cell lines transfected with the NTN constructs. Data are expressed as mean ± SEM (n=3) from a representative experiment and * indicates a significant difference from cells transfected with the wt construct (P<0.05, Kruskal-Wallis one way analysis on ranks followed by Student-Newman-Keuls Method).

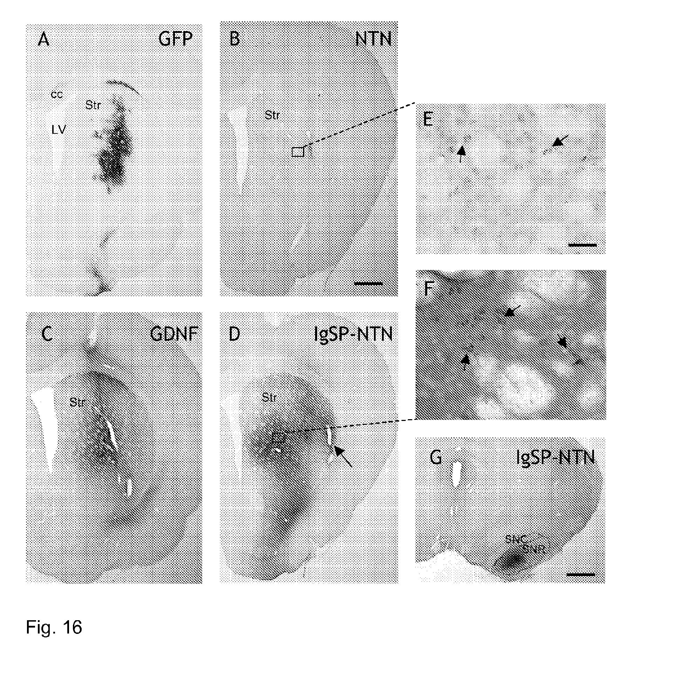

Figure 16: *In vivo* expression of transgene after intrastriatal injections of lentiviral constructs. Coronal sections through the striatum of rats transduced with lentiviral vectors and subsequently processed for immunohistochemistry using antibodies to GFP (A), human NTN (B, D, E, F, G) or GDNF (C). In the GFP control group a distinct intracellular staining pattern was seen after immunohistochemistry using an antibody against GFP (A). Weak intracellular immunoreactivity (arrows), but no extracellular NTN signal is observed in the rLV-NTN transduced striatum (B, E). In contrast, intracellular (arrows) and also a marked specific extracellular staining pattern for NTN is seen in the rLV-IgSP transduced striatum (D, F). (C) GDNF immunostaining in rLV-GDNF transduced animal shows a similar diffuse staining pattern due to secretion of the protein. (G) NTN immunoreactive fibers in the SN pars reticulate, showing that NTN is anterogradely transported from the IgSP-NTN transduced cells in the striatum. Bar 1 mm (A-D, G) or 62.5 µm (E-F).

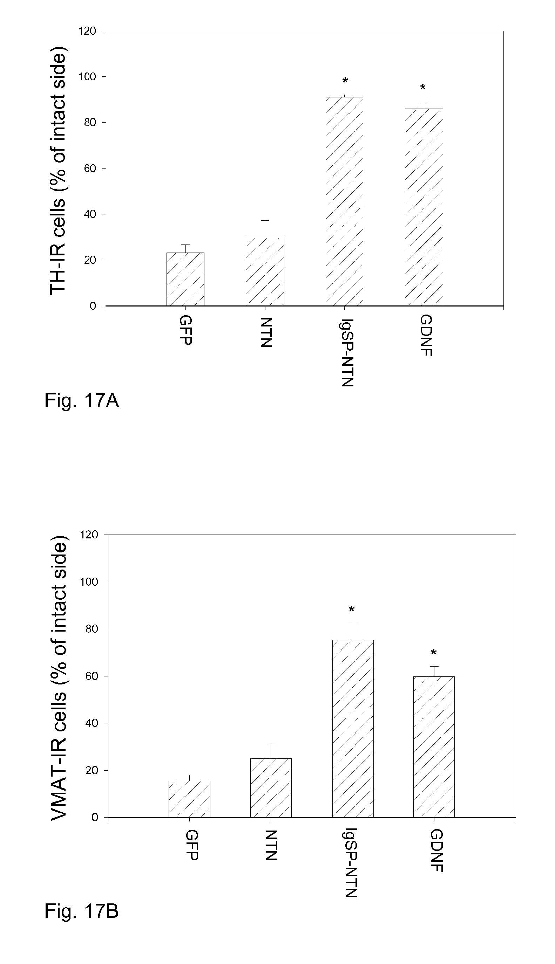

Figure 17: Neuroprotection of nigral dopamine neurons by rLV-IgSP-NTN. (A) Number of nigral neurons expressing TH in the lesion side compared to the intact side in the four different treatment groups. (B) Number of VMAT-immunoreactive nigral neurons in lesion side. Data are expressed as mean ± SEM (n=5-7) and * indicates a significant difference from the GFP group (P<0.05, one way ANOVA , Dunnett's Method)

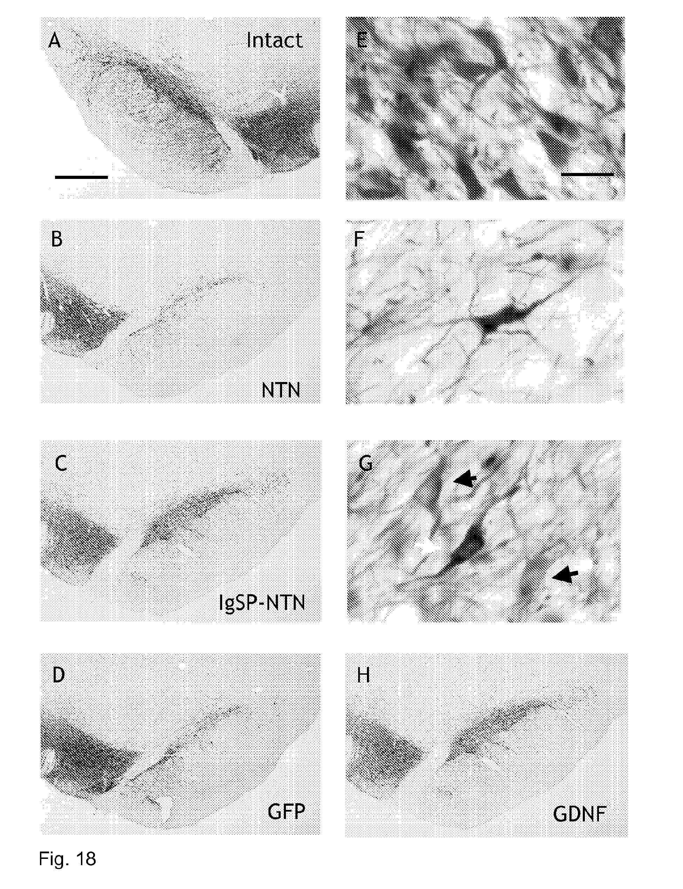

Figure 18: Coronal sections through substantia nigra of GFP, NTN, IgSP-NTN and GDNF-treated animals. On the intact side many TH-immunoreactive cells can be seen (A). On the side subject to a 6-OHDA lesion the number of remaining TH-immunoreactive neuronal profiles is significantly reduced in wt NTN or GFP treated animals (B, D). By contrast, more TH-immunoreactive neurons remain in animals receiving GDNF (H) or IgSP-NTN treatment (C). Note the reduced TH staining intensity in GDNF and IgSP-NTN treated animals. Higher magnification of TH-IR cells in intact side (E), lesion side of IgSP-NTN treated animal (G) and lesion side of wt NTN treated animal (F). Black arrows point at neurons with downregulated TH expression and white arrow point at neuron with normal TH expression. Bar 1 mm (A-E, H) or 25 µm (E-G).

### DEFINITIONS

A functional Neurturin proregion is a peptide located between the signal peptide and the mature peptide, which pro-peptide is cleavable from the mature peptide by furin after cleavage of the signal peptide. "Neurturin pro-region" means a region comprising e.g. at least amino acids corresponding to amino acids - 76 to -3 of SEQ ID No. 12, to amino acids -72 to -3 of SEQ ID No. 13, to amino acids -72 to -3 of SEQ ID No. 14. As these pre-pro-Neurturins contain more than one possible pro-site (RXXR motif), and as actual cleavage by the signal peptidase may vary, the exact length of a functional pro-region may vary accordingly.

Signal peptide - eukaryotic signal peptide. A eukaryotic signal peptide is a peptide present on proteins that are destined to either be secreted or to be membrane components. It is usually N-terminal to the protein. In the present context, all signal peptides identified in SignalP (version 2.0 or preferably version 3.0) as signal peptides are considered a signal peptide.

A mammalian signal peptide is a signal peptide derived from a mammalian protein secreted through the endoplamic reticulum.

Heterologous signal peptide - a signal peptide not naturally being operatively linked to a Neurturin polypeptide.

Mature human Neurturin polypeptide as used herein means the C-terminal 102 amino acids of native human pre-pro-Neurturin, i.e. amino acids 1-102 of SEQ ID No. 12.

Mature mouse Neurturin polypeptide as used herein means the C-terminal 100 amino acids of native mouse pre-pro-Neurturin, i.e. amino acids 1-100 of SEQ ID No. 13.

Mature rat Neurturin polypeptide as used herein means the C-terminal 100 amino acids of native rat pre-pro-Neurturin, i.e. amino acids 1-100 of SEQ ID No. 14.

Neurturin polypeptide: as used herein means a polypeptide comprising amino acids 8-101 of native human Neurturin (SEQ ID No 12), amino acids 6-99 of native mouse Neurturin (SEQ ID No 13), or amino acids 6-99 of native rat Neurturin (SEQ ID No. 14) each with up to 15 amino acid substitutions in the native sequence. In certain contexts it will be understood that "secreted Neurturin polypeptide" means a polypeptide to be secreted as opposed to one that has already been secreted.

Biocativity: the ability to bind when dimerised along with GFRα2 to RET and induce RET dimerisation and autophosphorylation. Bioactivity can be measured with RET L2 Elisa assay as described in the examples. Bioactivity may also be the ability to bind when dimerised along with GFRα1 to RET and induce RET dimerisation and autophosphorylation. Bioactivity can be measured with RET L1 Elisa assay as described in the examples.

Sequence identity: Sequence identity between a reference amino acid sequence and a variant amino acid sequences is performed by aligning the sequences using the default settings of Clustal W (1.82). The number of fully conserved residues is counted and divided by the number of residues in the reference sequence.

### DETAILED DESCRIPTION OF THE INVENTION

### I. Signal sequences

The targeting of secreted and proteins to the secretory pathway is accomplished via the attachment of a short, amino-terminal sequence, known as the signal peptide or signal sequence (von Heijne, G. (1985) J. Mol. Biol. 184, 99-105; Kaiser, C. A. & Botstein, D. (1986), Mol. Cell. Biol. 6, 2382-2391). The signal peptide itself contains several elements necessary for optimal function, the most important of which is a hydrophobic component. Immediately preceding the hydrophobic sequence is often a basic amino acid or acids, whereas at the carboxyl-terminal end of the signal peptide are a pair of small, uncharged amino acids separated by a single intervening amino acid which defines the signal peptidase cleavage site.

A preferred mammalian signal peptide is from 15 to 30 amino acids long (average for eukaryots is 23 amino acids). The common structure of signal peptides from various proteins is commonly described as a positively charged n-region, followed by a hydrophobic h-region and a neutral but polar c-region. The (-3,-1)-rule states that the residues at positions -3 and -1 (relative to the cleavage site) must be small and neutral for cleavage to occur correctly.

The n-region of eukaryotic signal sequences is only slightly Arg rich. The h-region is short and very hydrophobic. The c-region is short and has no observable pattern. As described the -3 and -1 positions consist of small and neutral residues. The amino acid residues C-terminal to the cleavage site is of less importance in eukaryots.

In the C-region the residues at position -1 and -3 are the most important. These are small, uncharged amino acids. At position -1 the residue is preferably A, G, S, I, T or C. More preferably the -1 position is A, G or S. At position -3 the residue is preferably A, V, S, T, G, C, I, or D. More preferably, the -3 position is A, V, S or T.

The hydrophobic region prevalently consist of a hydrophobic residues. These include A, I, L, F, V, and M. Preferably, at positions -6 to -13. Of the 8 amino acids constituting this region, at least 4 residues should be hydrophobic, more preferably at least 5, more preferably at least 6, such as 7 or 8.

Various different signal peptides can be used in the Neurturin constructs according to the present invention. The signal peptide can be any functional signal peptide, such as a heterologous signal peptide such as an Immunoglobulin Signal Peptide (IgSP). The signal peptide may be from any suitable species such as human, mouse, rat, monkey, pig. Preferably it is from human.

As evidenced by the appended examples, the use of the IgSP without the Neurturin pro-peptide in general results in an improved secretion of bioactive Neurturin both in vitro and in vivo. The results were reproducible both with lentivirus-transduced cells and with plasmid transfected cells. The cells secrete the mature protein as a biologically active protein, when the IgSP coding sequence is fused directly to the gene coding for the mature protein, excluding the native pre-pro part of Neuturin.

In another embodiment, the signal peptide is a native Neurturin signal peptide such as a native human Neurturin signal peptide. In this context the latter construct of native Neurturin signal peptide and a Neurturin polypeptide is called deltaproneurturin. Simply removing the sequence coding for the pro-part of Neurturin results in an unexpected increase in the secretion of bioactive Neurturin compared to expression of pre-pro-Neurturin.

The combined observations that secretion of bioactive Neurturin is strongly enhanced by using pro-less expression constructs compared to expression constructs coding for a functional Neurturin or GDNF propeptide and that the presence of a functional pro-region results in secretion of non-bioactive Neurturin has led the present inventors to the conclusion that the absence of a functional propeptide is a pre-requisite for secretion of bioactive Neurturin. The absence of the pro-region also strongly enhances the level of secretion of Neurturin. Selecting a strong signal peptide, such as IgSP, can enhance the secretion even further.

In one embodiment of the invention the encoded signal peptide is selected from the group consisting of NGF signal peptide (SEQ ID No 40 or 41), GDNF signal peptide (SEQ ID No 42 or 43, Persephin signal peptide (SEQ ID No. 46) and Neublastin signal peptide (SEQ ID No 45). Preferably these signal peptides are murine or human, more preferably human.

The signal peptide predictions in Example 6 show that mature Neurturin with NGF and GDNF signal peptides are as strong signal peptides as is IgSP. The Persephin signal peptide linked to mature Neurturin is also predicted to be a strong signal peptide. The deltapro-expression constructs (NTN signal peptide linked to mature or N-terminally truncated NTN) are evaluated to be less strong signal peptides. This is confirmed by the quantitative data shown in the

### examples.

Each of the specified signal peptides can be combined individually with a mature mouse, rat or human NTN (SEQ ID No 10, 11, or 8), or with an N-terminally truncated form of any of these as illustrated in Example 6 and the sequence listing for IgSP NTN (SEQ ID No 18-24) and for deltaproNTN (SEQ ID No 16 and 25-30).

### Cleavage of signal peptide

Before deciding on a specific Neurturin form to incorporate into an expression construct, the likelihood of cleavage of the signal peptide (SP) can be checked using state of the art prediction tools. One such preferred prediction tool is the SignalP software which is available at the SignalP WWW server (http://www.cbs.dtu.dk/services/SignalP-2.0/). or preferably with the newer version 3.0 available from the same server (http://www.cbs.dtu.dk/services/signalP/).

The **SignaIP** WWW server will return three scores between 0 and 1 for each position in your sequence:
**C-score** (raw cleavage site score)

The output score from networks trained to recognize cleavage sites *VS*. other sequence positions. Trained to be:
**high** at position +1 (immediately *after* the cleavage site)
**low** at all other positions.

### S-score (signal peptide score)

The output score from networks trained to recognize signal peptide *VS*. non-signal-peptide positions. Trained to be:
**high** at all positions before the cleavage site
**low** at 30 positions after the cleavage site and in the N-terminals of non-secretory proteins.

### Y-score (combined cleavage site score)

The prediction of cleavage site location is optimized by observing where the C-score is high *and* the S-score changes from a high to a low value. The Y-score formalises this by combining the height of the C-score with the slope of the S-score.

Specifically, the Y-score is a geometric average between the C-score and a smoothed derivative of the S-score (*i.e.,* the difference between the mean S-score over d positions before and *d* positions after the current position, where *d* varies with the chosen network ensemble).

All three scores are averages of five networks trained on different partitions of the data.

For each sequence, **SignaIP** will report the maximal C-, S-, and Y-scores, and the mean S-score between the N-terminal and the predicted cleavage site. These values are used to distinguish between signal peptides and non-signal peptides. If your sequence is predicted to have a signal peptide, the cleavage site is predicted to be immediately before the position with the maximal Y-score.

For a typical **signal peptide,** the C- and Y-scores will be high at position +1, while the S-score will be high before the cleavage site and low thereafter.

For comparison, the prediction can be compared to the predicted cleavage of the wildtype Neurturin signal peptide (cleavage between amino acids no. 19 and 20 of pre-pro-NTN (SEQ ID No 12). For mouse and rat pre-pro-Neurturin the cleavage prediction is less certain. Cleavage is predicted to occur between amino acids 23 and 24 but there is also a likelihood of cleavge at the same position as in human pre-pro NTN.

The best prediction of cleavage site location is provided by the position of the Y-score maximum. The best prediction of sequence type (signal peptide or non-secretory protein) is given by the mean S-score (the average of the S-score in the region between position 1 and the position immediately before the Y-score maximum): if mean S-score is larger than 0.5, the sequence is predicted to be a signal peptide. Accordingly, in a preferred embodiment, the mean S-score is larger than 0.5.

The newer version of SignalP (v. 3.0) also includes a new score D, or Dmax (discrimination score), that describes "signal peptidedness". The D score is found to correlate to level of secretion using said signal peptide with the protein in question. It is preferred to use a Neurturin expression construct which codes for a signal peptide-Neurturin protein exhibiting a Dmax value of at least 0.5, such as at least 0.6, for example at least 0.7, such as at least 0.8.

Preferred signal peptide-Neurturin constructs are those, which have a predicted cleavage between the SP and the Neuturin in either the SignaIP-NN or the SignaIP-HMM program. Particularly preferred are Neurturin constructs which have a predicted signal peptide at this position in both SignaIP-NN and SignaIP-HMM.

When the signal peptide is IgSP, the Neurturin part of the construct may include any human Neurturin from 102 to 96 amino acids long or any mouse or rat Neurturin from 100 to 96 amino acids long. In all of these cases both SignaIP-NN and SignaIP-HMM predict cleavage of the signal peptide following the 19 amino acid signal peptide.

Other preferred signal peptides include GDNF and NGF signal peptides as well as Persephin signal peptides.

In another preferred embodiment the expression construct codes for a signal peptide-Neurturin protein, in which cleavage as predicted by SignaIP version 2.0 or preferably version 3.0 occurs before the first canonical cysteine in mature Neurturin.

References: Henrik Nielsen, Jacob Engelbrecht, Soren Brunak and Gunnar von Heijne: Identification of prokaryotic and eukaryotic signal peptides and prediction of their cleavage sites. Protein Engineering, 10, 1-6 (1997). For the SignaIP-HMM output model: Henrik Nielsen and Anders Krogh: Prediction of signal peptides and signal anchors by a hidden Markov model. In Proceedings of the Sixth International Conference on Intelligent Systems for Molecular Biology (ISMB 6), AAAI Press, Menlo Park, California, pp. 122--130 (1998). Improved prediction of signal peptides: SignalP 3.0. Jannick Dyrløv Bendtsen, Henrik Nielsen, Gunnar von Heijne and Soren Brunak. J. Mol. Biol., 340:783-795, 2004.

### IgSP

According to a particularly preferred embodiment, the signal peptide is the signal peptide from the Immunoglobulin heavy chain. As evidenced by the appended examples the use of this signal peptides in general results in an improved secretion of the encoded Neurturin both in vitro and in vivo. The results were reproducible both with lentivirus-transduced cells (in vivo and in vitro) and with plasmid transfected cells (in vitro). The cells produce the mature protein in the correct size even when the IgSP gene is fused directly to the gene coding for the mature protein (i.e. excluding the pro part).

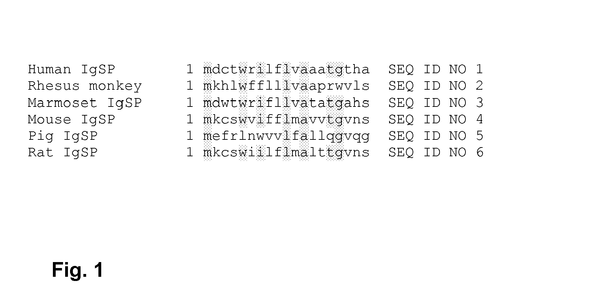

Immunoglobulin signal peptide is a small 19 amino acid peptide known from a large group of mammals. The sequences from human, rhesus monkey, marmoset, rat, mouse and pig are aligned in Figure 1. The percent sequence identity compared to human IgSP varies from 21 (pig) to 68 (marmoset) percent. This relatively large variation indicates that the specific sequence can be altered to a large extent without substantially changing the biological function of the signal peptide. It is also observed that there is cross species reactivity as evidenced by the appended examples. These were carried out with the mouse IgSP, which was functional in rat (in vivo experiments) and in human cells (ARPE 19 cells) as well as Chinese Hamster cells (CHO) and rat cells (HiB5).

Preferably the IgSP is of murine or human origin because the murine IgSP is known to be functional in mouse, rat and human beings. For use in human beings, the IgSP preferably is of human origin in order to reduce the risk of any cross species side effect.

### II. Neurturin

Neurturin is one of the four members of the GDNF (Glial cell-line Derived Neurotrophic Factor). It signals through the GFRα2 and GFRα1 co-receptor. Neurturin has been suggested as a therapeutic candidate for treating a number of degenerative diseases. Where the cellular degeneration involves neuronal degeneration, the diseases include, but are not limited to peripheral neuropathy, amyotrophic lateral sclerosis, Alzheimer's disease, Parkinson's disease, Huntington's disease, ischemic stroke, acute brain injury, acute spinal cord injury, nervous system tumors, multiple sclerosis, peripheral nerve trauma or injury, exposure to neurotoxins, metabolic diseases such as diabetes or renal dysfunctions and damage caused by infectious agents. Where the cellular degeneration involves bone marrow cell degeneration, the diseases include, but are not limited to disorders of insufficient blood cells such as, for example, leukopenias including eosinopenia and/or basopenia, lymphopenia, monocytopenia, neutropenia, anemias, thrombocytopenia as well as an insufficiency of stem cells for any of the above. In particular Neuturin administered with the constructs and methods of the present invention can be used in treating Parkinson's disease.

Neurturin was first described in WO 97/08196 (University of Washington). The pre-pro form of human neurturin is set forth in SEQ ID NO 12, the pre-pro form of mouse neurturin is set forth in SEQ ID NO 13 and the pre-pro form of rat Neurturin is set forth in SEQ ID No 14. The mature form of Neurturin includes amino acids no. 1-102 of SEQ ID No 12 (human mature NTN shown in SEQ ID No 8), 1-100 of SEQ ID No 13 (mouse mature NTN shown in SEQ ID No. 10), and 1-100 of SEQ ID No. 14 (rat mature shown in SEQ ID No 11).

The nucleotide sequence coding for mature human Neurturin is set forth in SEQ ID NO: 7 of the present application. The encoded protein is 102 amino acids long and is set forth in SEQ ID NO: 8. The mature mouse Neurturin is set forth in SEQ ID No. 10. The mature rat Neurturin sequence is set forth in SEQ ID No 11. Example 1 and 3 describe methods for cloning human mature Neurturin. Preferably the Neurturin used in the context of the present invention is human mature Neurturin, but it is likewise contemplated that the corresponding mouse and rat sequences can be used.

Sequence variants of the present invention are suitably defined with reference to the encoded biologically active Neurturin. The percent sequence identify between the growth factors of the GDNF family lies approximately in the range of 50% when determined over the mature part of the growth factors. With such differences between closely related growth factors, it is contemplated that the sequence of Neurturin can be changed without changing the biological activity of the growth factor. In one embodiment of the present invention a sequence variant of Neurturin is a sequence encoding a growth factor, which shares at least 70% sequence identity to the C-terminal 96 amino acids of human or mouse or rat Neurturin (SEQ ID No 9 and 10 and 11). More preferably the sequence variant shares at least 75% sequence identity to said Neurturin, more preferably at least 80%, more preferably at least 85%, more preferably at least 90%, more preferably at least 95%, more preferably at least 97%, more preferably at least 99%. In a particularly preferred embodiment, the sequence identity is determined by comparison to the C-terminal 96 amino acids of human Neurturin (SEQ ID No 9).

It is known in the art that changes in amino acid sequences cannot be made freely without affecting the biological function of the protein. Amino acid residues that most likely cannot be changed without seriously affecting the biological function of Neurturin first and foremost include the seven conserved canonical cystein residues of the mature part (residues nr 8, 35, 39, 69, 70, 99, and 101 of SEQ ID NO. 8).

An alignment of Neurturin against the other GDNF family neurotrophic factors provides the skilled person information about which amino acid residues are most important for conservation of the biological function of a sequence variant of Neurturin. In a preferred embodiment, the sequence variant of Neurturin comprises the fully conserved residues at the positions corresponding to wildtype humanNTN (hNTN). In a more preferred embodiment, the sequence variant comprises the fully conserved and the strongly conserved residues at the positions corresponding to wildtype hNTN. In a still more preferred embodiment, the sequence variant of NTN comprises the fully, the strongly and the weakly conserved residues at the position corresponding to wildtype hNTN.

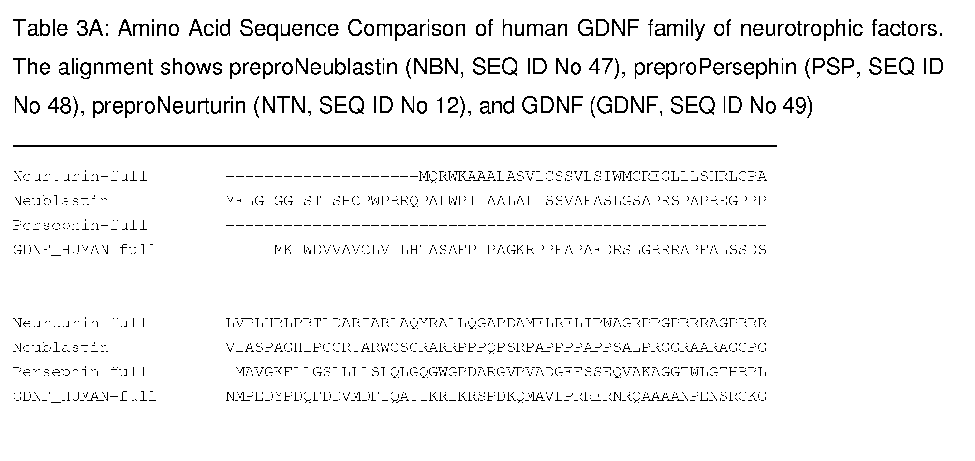

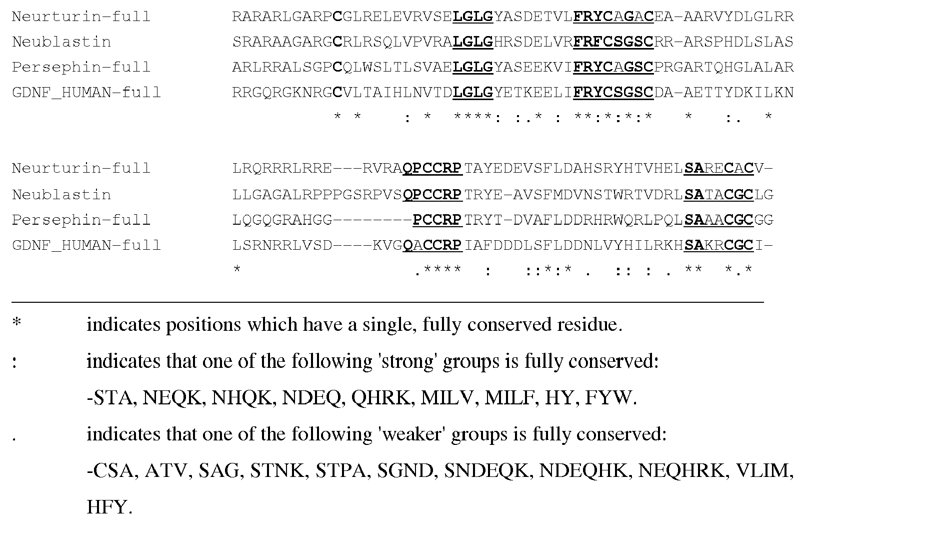

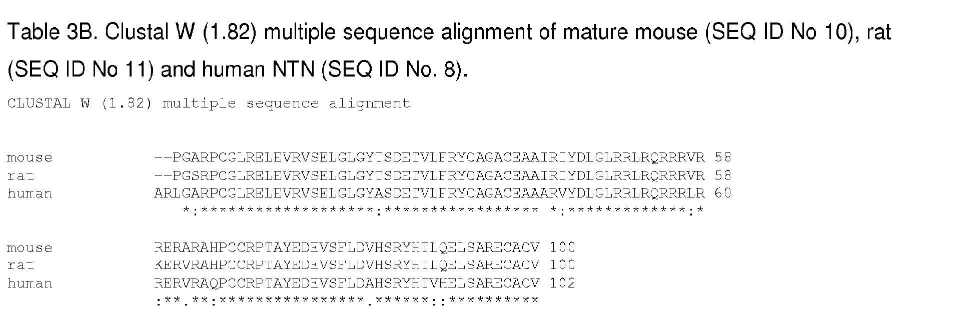

Mutations can be introduced into Neurturin, by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted non-essential amino acid residue in the NTN protein is replaced with another amino acid residue from the same side chain family.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, VLIM, HFY, wherein the letters within each group represent the single letter amino acid code.

GDNF family growth factors are known to be biologically active in N-terminally truncated form (US 6,184,200 describing truncated GDNF; WO 02/072826 describing truncated Neublastin). Neurturin is also belived to be bioactive in N-terminally truncated form. The present inventors have contemplated the use of a DNA sequence coding for N-terminally truncated Neurturin consisting of the C-terminal 101, 100, 99, 98, 97, or 96 amino acids of human mature Neurturin and the corresponding truncated rat and mouse proteins. The shortest human form, which is believed to be as bioactive as the mature protein, consists of the 96 amino acids set forth in SEQ ID NO: 9. Similarly, the mouse and rat proteins may be N-terminally truncated down to the C-terminal 96 amino acids. It is presently believed that there must be one amino acid residue left N-terminal to the first canonical cystein residue.

The C-terminal may also be truncated by removal of the amino acid residue(s) C-terminal to the last canonical cystein residue. In human, mouse, and rat this equals deletion of one C-terminal amino acid. In a preferred embodiment of the invention, this C-terminal amino acid is not deleted.

### III. Target Tissues for Treatment of Neurodegenerative Disorders

Methods of treating or preventing or ameliorating Parkinson's Disease using virus vectors comprising Glial Cell-line Derived Neurotrophic Factor (GDNF) coding sequence are well known. Delivery of GDNF to the CNS has been achieved in pre-clinical studies using protein injections, delivery via pumps and by in vivo gene therapy. Numerous studies describe transduction of CNS cells using AAV or lentivirus expressing GDNF (Kordower, Ann Neurol, 2003 53 (suppl 3), s120-s134; WO 03/018821, Ozawa et al; US 2002187951, Aebischer et al; Georgievska et al 2002, Exp Nerol 117(2), 461-474; Georgievska et al 2002, NeuroReport 13(1), 75-82; Wang et al, 2002, Gene Therapy, 9(6), 381-389; US 2002031493, Rohne-Poulenc Rorer SA; US 6,180,613 Roeckefeller University; Kozlowski et al 2000, Exp Neurol, 166(1), 1,15; Bensadoun 2000, Exp Neurol, 164(1), 15-24; Connor et al 1999, Gene Therapy, 6(12), 1936-1951; Mandel et al 1997, PNAS, 94(25), 14083-88; Lapchak et al 1997, Brain Research, 777 (1,2), 153-160; Bilang-Bleuel et al 1997, PNAS 94(16), 8818-8823). These methods can be used in delivering Neurturin to the central nervous system using the virus vectors of the present invention.

One important parameter for in vivo gene therapy is the selection of a suitable target tissue. A region of the brain is selected for its retained responsiveness to neurotrophic factors in particular to Neurturin. In humans, CNS neurons, which retain responsiveness to neurotrophic factors into adulthood include the cholinergic basal forebrain neurons, the entorhinal cortical neurons, the thalamic neurons, the locus coeruleus neurons, the spinal sensory neurons and the spinal motor neurons. A further characteristic of cells with retained responsiveness to Neurturin is the expression of Ret and one of the two co-receptors GFRα1 and GFRα2.

Abnormalities within the cholinergic compartment of this complex network of neurons have been implicated in a number of neurodegenerative disorders, including AD, Parkinson's disease, and amyotrophic lateral sclerosis (ALS, also known as Lou Gehrig's disease). The cholinergic basal forebrain (particularly, the Ch4 region of the basal forebrain) is a particularly suitable target tissue.

Within the primate forebrain, magnocellular neurons Chl-Ch4 provide cholinergic innervation to the cerebral cortex, thalamus and basolateral nucleus of the amygdala. In subjects with neurodegenerative diseases such as AD, neurons in the Ch4 region (nucleus basalis of Meynert) which have nerve growth factor (NGF) receptors undergo marked atrophy as compared to normal controls (see, e. g., Kobayashi, et al., Mol. Chem. Neuropathol., 15: 193-206 (1991)).

In normal subjects, neurotrophins prevent sympathetic and sensory neuronal death during development and prevents cholinergic neuronal degeneration in adult rats and primates (Tuszynski, et al., Gene Therapy, 3 : 305314 (1996)). The resulting loss of functioning neurons in this region of the basal forebrain is believed to be causatively linked to the cognitive decline experienced by subjects suffering from neurodegenerative conditions such as AD (Tuszynski, et al., supra and, Lehericy, et al., J. Comp. Neurol., 330: 15-31 (1993)).

In human AD, basal forebrain neuronal loss occurs over an intraparenchymal area of approximately 1 cm in diameter. To treat affected neurons over such a large region, treatment with vector composition at upwards of 10 separate in vivo gene vector delivery sites is desirable. However, in treating localized injuries to the basal forebrain, the affected areas of the brain will likely be smaller such that selection of fewer delivery sites (e. g., 5 or fewer) will be sufficient for restoration of a clinically significant number of cholinergic neurons.

Importantly, specific in vivo gene delivery sites are selected so as to cluster in an area of neuronal loss. Such areas may be identified clinically using a number of known techniques, including magnetic resonance imaging (MRI) and biopsy. In humans, non-invasive, in vivo imaging methods such as MRI will be preferred. Once areas of neuronal loss are identified, delivery sites are selected for stereotaxic distribution so each unit dosage of NTN is delivered into the brain at, or within 500 µm from, a targeted cell, and no more than about 10 mm from another delivery site.

### IV. Dosing Requirements and Delivery Protocol

A further important parameter is the dosage of Neurturin to be delivered into the target tissue. In this regard, "unit dosage" refers generally to the concentration of Neurturin/ml of Neurturin composition. For viral vectors, the Neurturin concentration may be defined by the number of viral particles/ml of neurotrophic composition. Optimally, for delivery of Neurturin using a viral expression vector, each unit dosage of Neurturin will comprise 2.5 to 25 µl of a Neurturin composition, wherein the composition includes a viral expression vector in a pharmaceutically acceptable fluid and provides from 10¹⁰ up to 10¹⁵ Neurturin expressing viral particles per ml of Neurturin composition. Such high titers are particularly useful for adeno-associated virus. For lentivirus, the titer is normally lower, such as from 10⁸ to 10¹⁰ transducing units per ml (TU/mL) determined as described in the examples.

Guidance as to the dosing of Neurturin virus in the treatment of Parkinson's Disease can be found in the numerous cited references concerning devlivery of GDNF using in vivo gene therapy.

In a preferred embodiment, the administration site is the striatum of the brain, in particular the caudate and/or the putamen. Injection into the putamen can label target sites located in various distant regions of the brain, for example, the globus pallidus, amygdala, subthalamic nucleus or the substantia nigra. Transduction of cells in the pallidus commonly causes retrograde labelling of cells in the thalamus. In a preferred embodiment the (or one of the) target site(s) is the substantia nigra. Injection may also be into both the striatum and the substantia nigra.

Within a given target site, the vector system may transduce a target cell. The target cell may be a cell found in nervous tissue, such as a neuron, astrocyte, oligodendrocyte, microglia or ependymal cell. In a preferred embodiment, the target cell is a neuron, in particular a TH positive neuron.

The vector system is preferably administered by direct injection. Methods for injection into the brain (in particular the striatum) are well known in the art (Bilang-Bleuel et al (1997) Proc. Acad. Nati. Sci. USA 94:8818-8823; Choi-Lundberg et al (1998) Exp. Neurol.154:261-275; Choi-Lundberg et al (1997) Science 275:838-841; and Mandel et al (1997)) Proc. Acad. Natl. Sci. USA 94:14083-14088). Stereotaxic injections may be given.

As mentioned above, for transduction in tissues such as the brain, it is necessary to use very small volumes, so the viral preparation is concentrated by ultracentrifugation. The resulting preparation should have at least 10⁸ t.u./ml, preferably from 10⁸ to 10¹⁰ t.u./ml, more preferably at least 10⁹ t.u./ml. (The titer is expressed in transducing units per ml (t.u./ml) as described in example 2). It has been found that improved dispersion of transgene expression can be obtained by increasing the number of injection sites and decreasing the rate of injection (Horellou and Mallet (1997) as above). Usually between 1 and 10 injection sites are used, more commonly between 2 and 6. For a dose comprising 1-5×10⁹ t.u./ml, the rate of injection is commonly between 0.1 and 10 µl/min, usually about 1 µl/min.

Due to the high secretion efficiency of the improved vectors provided by the present invention, smaller volumes of virus composition need to be injected to obtain a clinical effect than if vectors coding for pre-pro-NTN are used.

The Neurturin composition is delivered to each delivery cell site in the target tissue by microinjection, infusion, scrape loading, electroporation or other means suitable to directly deliver the composition directly into the delivery site tissue through a surgical incision. The delivery is accomplished slowly, such as over a period of about 5-10 minutes (depending on the total volume of Neurturin composition to be delivered).

Those of skill in the art will appreciate that the direct delivery method employed by the invention obviates a limiting risk factor associated with in vivo gene therapy; to wit, the potential for transduction of non-targeted cells with the vector carrying the Neurturin encoding transgene. In the invention, delivery is direct and the delivery sites are chosen so diffusion of secreted Neurturin takes place over a controlled and pre-determined region of the brain to optimise contact with targeted neurons, while minimizing contact with non-targeted cells.

### V. Viral vectors

Broadly, gene therapy seeks to transfer new genetic material to the cells of a patient with resulting therapeutic benefit to the patient. Such benefits include treatment or prophylaxis of a broad range of diseases, disorders and other conditions.

Ex vivo gene therapy approaches involve modification of isolated cells, which are then infused, grafted or otherwise transplanted into the patient. See, e.g., U.S. Pat. Nos. 4,868,116, 5,399,346 and 5,460,959. In vivo gene therapy seeks to directly target host patient tissue in vivo.

Viruses useful as gene transfer vectors include papovavirus, adenovirus, vaccinia virus, adeno-associated virus, herpesvirus, and retroviruses. Suitable retroviruses include the group consisting of HIV, SIV, FIV, EIAV, MoMLV.

Preferred viruses for treatment of disorders of the central nervous system are lentiviruses and adeno-associated viruses. Both types of viruses can integrate into the genome without cell divisions, and both types have been tested in pre-clinical animal studies for indications in the nervous system, in particular in the central nervous system.

Methods for preparation of AAV are described in the art, e.g. US 5,677,158, US 6,309,634, and US 6,451,306 describe examples of delivery of AAV to the central nervous system.

A special and preferred type of retroviruses include the lentiviruses which can transduce a cell and integrate into its genome without cell division. Thus preferably the vector is a replication-defective lentivirus particle. Such a lentivirus particle can be produced from a lentiviral vector comprising a 5' lentiviral LTR, a tRNA binding site, a packaging signal, a promoter operably linked to a polynucleotide signal encoding said fusion protein, an origin of second strand DNA synthesis and a 3' lentiviral LTR. Methods for preparation and in vivo administration of lentivirus to neural cells are described in US 20020037281 (Methods for transducing neural cells using lentiviral vectors) and US 20020187951 (Lentiviral-mediated growth factor gene therapy for neurodegenerative diseases).

Retroviral vectors are the vectors most commonly used in human clinical trials, since they carry 7-8 kb and since they have the ability to infect cells and have their genetic material stably integrated into the host cell with high efficiency. See, e.g., WO 95/30761; WO 95/24929.

Oncovirinae require at least one round of target cell proliferation for transfer and integration of exogenous nucleic acid sequences into the patient. Retroviral vectors integrate randomly into the patient's genome.

Three classes of retroviral particles have been described; ecotropic, which can infect murine cells efficiently, and amphotropic, which can infect cells of many species. A third class include xenotrophic retrovirus which can infect cells of another species than the species which produced the virus. Their ability to integrate only into the genome of dividing cells has made retroviruses attractive for marking cell lineages in developmental studies and for delivering therapeutic or suicide genes to cancers or tumors. These vectors may be particularly useful in the central nervous system for cancer treatment, where there is a relative lack of cell division in adult patients.

For use in human patients, the retroviral vectors must be replication defective. This prevents further generation of infectious retroviral particles in the target tissue--instead the replication defective vector becomes a "captive" transgene stable incorporated into the target cell genome. Typically in replication defective vectors, the gag, env, and pol genes have been deleted (along with most of the rest of the viral genome). Heterologous DNA is inserted in place of the deleted viral genes. The heterologous genes may be under the control of the endogenous heterologous promoter, another heterologous promoter active in the target cell, or the retroviral 5' LTR (the viral LTR is active in diverse tissues). Typically, retroviral vectors have a transgene capacity of about 7-8 kb.

Replication defective retroviral vectors require provision of the viral proteins necessary for replication and assembly in trans, from, e.g., engineered packaging cell lines. It is important that the packaging cells do not release replication competent virus and/or helper virus. This has been achieved by expressing viral proteins from RNAs lacking the ψ signal, and expressing the gag/pol genes and the env gene from separate transcriptional units. In addition, in some 2. and 3. generation retriviruses, the 5' LTR's have been replaced with non-viral promoters controlling the expression of these genes, and the 3' promoter has been minimised to contain only the proximal promoter. These designs minimize the possibility of recombination leading to production of replication competent vectors, or helper viruses. See, e.g., U.S. Pat. No. 4,861,719, herein incorporated by reference.

### VI. Expression vectors

Construction of vectors for recombinant expression of Neurturin for use in the invention may be accomplished using conventional techniques which do not require detailed explanation to one of ordinary skill in the art. For review, however, those of ordinary skill may wish to consult Maniatis et al., in Molecular Cloning: A Laboratory Manual, Cold Spring Harbor Laboratory, (NY 1982).

The chimeric expression constructs used in the present invention may be created as described in the examples, e.g. by amplifying the desired fragments (a signal sequence and a Neurturin coding sequence) by PCR and fusing these in overlapping PCR. As several of the preferred signal sequences are relatively short, the 5' PCR primer used for amplifying the Neurturin coding sequence may include the sequence coding for the signal sequence as well as a TATA box and other regulatory elements.

Briefly, construction of recombinant expression vectors employs standard ligation techniques. For analysis to confirm correct sequences in vectors constructed, the genes are sequences using, for example, the method of Messing, et al., (Nucleic Acids Res., 9: 309-, 1981), the method of Maxam, et al., (Methods in Enzymology, 65: 499, 1980), or other suitable methods which will be known to those skilled in the art.

Size separation of cleaved fragments is performed using conventional gel electrophoresis as described, for example, by Maniatis, et al., (Molecular Cloning, pp. 133-134,1982).

Expression of a gene is controlled at the transcription, translation or post-translation levels. Transcription initiation is an early and critical event in gene expression. This depends on the promoter and enhancer sequences and is influenced by specific cellular factors that interact with these sequences. The transcriptional unit of many genes consists of the promoter and in some cases enhancer or regulator elements (Banerji et al., Cell 27: 299 (1981); Corden et al., Science 209: 1406 (1980); and Breathnach and Chambon, Ann. Rev. Biochem. 50: 349 (1981)). For retroviruses, control elements involved in the replication of the retroviral genome reside in the long terminal repeat (LTR) (Weiss et al., eds., The molecular biology of tumor viruses: RNA tumor viruses, Cold Spring Harbor Laboratory, (NY 1982)). Moloney murine leukemia virus (MLV) and Rous sarcoma virus (RSV) LTRs contain promoter and enhancer sequences (Jolly et al., Nucleic Acids Res. 11: 1855 (1983); Capecchi et al., In : Enhancer and eukaryotic gene expression, Gulzman and Shenk, eds., pp. 101-102, Cold Spring Harbor Laboratories (NY 1991). Other potent promoters include those derived from cytomegalovirus (CMV) and other wild-type viral promoters.

Promoter and enhancer regions of a number of non-viral promoters have also been described (Schmidt et al., Nature 314: 285 (1985); Rossi and deCrombrugghe, Proc. Natl. Acad. Sci. USA 84: 5590-5594 (1987)). Methods for maintaining and increasing expression of transgenes in quiescent cells include the use of promoters including collagen type I (1 and 2) (Prockop and Kivirikko, N. Eng. J. Med. 311: 376 (1984) ; Smith and Niles, Biochem. 19: 1820 (1980) ; de Wet et al., J. Biol. Chem., 258: 14385 (1983)), SV40 and LTR promoters.

According to one embodiment of the invention, the promoter is a constitutive promoter selected from the group consisting of: ubiquitin promoter, CMV promoter, JeT promoter (US 6,555,674), SV40 promoter, and Elongation Factor 1 alpha promoter (EF1-alpha).

Examples of inducible/repressible promoters include: Tet-On, Tet-Off, Rapamycin-inducible promoter, Mx1.

In addition to using viral and non-viral promoters to drive transgene expression, an enhancer sequence may be used to increase the level of transgene expression. Enhancers can increase the transcriptional activity not only of their native gene but also of some foreign genes (Armelor, Proc. Natl. Acad. Sci. USA 70 : 2702 (1973)). For example, in the present invention collagen enhancer sequences may be used with the collagen promoter 2 (I) to increase transgene expression. In addition, the enhancer element found in SV40 viruses may be used to increase transgene expression. This enhancer sequence consists of a 72 base pair repeat as described by Gruss et al., Proc. Natl. Acad. Sci. USA 78: 943 (1981); Benoist and Chambon, Nature 290: 304 (1981), and Fromm and Berg, J. Mol. Appl. Genetics, 1 : 457 (1982), all of which are incorporated by reference herein. This repeat sequence can increase the transcription of many different viral and cellular genes when it is present in series with various promoters (Moreau et al., Nucleic Acids Res. 9 : 6047 (1981).

Further expression enhancing sequences include but are not limited to Woodchuck hepatitis virus post-transcriptional regulation element, WPRE, SP163, rat Insulinll-intron or other introns, CMV enhancer, and Chicken [beta]-globin insulator or other insulators.

Transgene expression may also be increased for long term stable expression using cytokines to modulate promoter activity. Several cytokines have been reported to modulate the expression of transgene from collagen 2 (I) and LTR promoters (Chua et al., connective Tissue Res., 25: 161-170 (1990); Elias et al., Annals N. Y. Acad. Sci., 580 : 233-244 (1990)); Seliger et al., J. Immunol. 141: 2138-2144 (1988) and Seliger et al., J. Virology 62: 619-621 (1988)). For example, transforming growth factor (TGF), interleukin (IL)-I, and interferon (INF) down regulate the expression of transgenes driven by various promoters such as LTR. Tumor necrosis factor (TNF) and TGF 1 up regulate, and may be used to control, expression of transgenes driven by a promoter. Other cytokines that may prove useful include basic fibroblast growth factor (bFGF) and epidermal growth factor (EGF).

Collagen promoter with the collagen enhancer sequence (Coll (E)) may also be used to increase transgene expression by suppressing further any immune response to the vector which may be generated in a treated brain notwithstanding its immune-protected status. In addition, anti-inflammatory agents including steroids, for example dexamethasone, may be administered to the treated host immediately after vector composition delivery and continued, preferably, until any cytokine-mediated inflammatory response subsides. An immunosuppression agent such as cyclosporin may also be administered to reduce the production of interferons, which downregulates LTR promoter and Coll (E) promoter-enhancer, and reduces transgene expression.

The vector may comprise further sequences such as a sequence coding for the Cre-recombinase protein, and LoxP sequences. A further way of ensuring temporary expression of the neublastin is through the use of the Cre-LoxP system which results in the excision of part of the inserted DNA sequence either upon administration of Cre-recombinase to the cells (Daewoong et al, Nature Biotechnology 19:929-933) or by incorporating a gene coding for the recombinase into the virus construct (Plück, Int J Exp Path, 77:269-278). Incorporating a gene for the recombinase in the virus construct together with the LoxP sites and a structural gene (a neublastin in the present case) often results in expression of the structural gene for a period of approximately five days.

### VII. Pharmaceutical preparations

To form a Neurturin composition for use in the invention, Neurturin encoding expression viral vectors may be placed into a pharmaceutically acceptable suspension, solution or emulsion. Suitable mediums include saline and liposomal preparations.

More specifically, pharmaceutically acceptable carriers may include sterile aqueous of nonaqueous solutions, suspensions, and emulsions. Examples of nonaqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils.

Intravenous vehicles include fluid and nutrient replenishers, electrolyte replenishers (such as those based on Ringer's dextrose), and the like.

Preservatives and other additives may also be present such as, for example, antimicrobials, antioxidants, chelating agents, and inert gases and the like. Further, a composition of Neurturin transgenes may be lyophilized using means well known in the art, for subsequent reconstitution and use according to the invention.

A colloidal dispersion system may also be used for targeted gene delivery.

Colloidal dispersion systems include macromolecule complexes, nanocapsules, microspheres, beads, and lipid-based systems including oil-in-water emulsions, micelles, mixed micelles, and liposoms. Liposomes are artificial membrane vesicles which are useful as delivery vehicles in vitro and in vivo. It has been shown that large unilamellar vesicles (LUV), which range in size from 0.2-4.0 µm can encapsulate a substantial percentage of an aqueous buffer containing large macro molecules. RNA, DNA and intact virions can be encapsulated within the aqueous interior and be delivered to cells in a biologically active form (Fraley, et al., Trends Biochem. Sci., 6: 77,1981). In addition to mammalian cells, liposomes have been used for delivery of operatively encoding transgenes in plant, yeast and bacterial cells. In order for a liposome to be an efficient gene transfer vehicle, the following characteristics should be present: (1) encapsulation of the genes encoding the Neurturin at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino, et al., Biotechniques, 6: 682,1988).

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs which contain sinusoidal capillaries.

Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted gene delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand.

A further example of a delivery system includes transplantation into the therapeutic area of a composition of packaging cells capable of producing vector particles as described in the present invention. Methods for encapsulation and transplantation of such cells are known in the art, in particular from WO 97/44065 (Cytotherapeutics). By selecting a packaging cell line capable of producing lentiviral particles, transduction of non-dividing cells in the therapeutic area is obtained. By using retroviral particles capable of transducing only dividing cells, transduction is restricted to de-novo differentiated cells in the therapeutic area.

### VIII. Encapsulation of cells

Encapsulated cell therapy is based on the concept of isolating cells from the recipient host's immune system by surrounding the cells with a semipermeable biocompatible material before implantation within the host. The invention includes a device in which Neurturin-secreting cells are encapsulated in an immunoisolatory capsule. An "immunoisolatory capsule" means that the capsule, upon implantation into a recipient host, minimizes the deleterious effects of the host's immune system on the cells in the core of the device. Cells are immunoisolated from the host by enclosing them within implantable polymeric capsules formed by a microporous membrane. This approach prevents the cell-to cell contact between host and implanted tissues, eliminating antigen recognition through direct presentation. The membranes used can also be tailored to control the diffusion of molecules, such as antibody and complement, based on their molecular weight (Lysaght et al., 56 J. Cell Biochem. 196 (1996), Colton, 14 Trends Biotechnol. 158 (1996)). Using encapsulation techniques, Cells can be transplanted into a host without immune rejection, either with or without use of immunosuppressive drugs. Useful biocompatible polymer capsules usually contain a core that contains cells, either suspended in a liquid medium or immobilized within an immobilizing matrix, and a surrounding or peripheral region of permselective matrix or membrane ("jacket") that does not contain isolated cells, that is biocompatible, and that is sufficient to protect cells in the core from detrimental immunological attack. Encapsulation hinders elements of the immune system from entering the capsule, thereby protecting the encapsulated cells from immune destruction. The semipermeable nature of the capsule membrane also permits the biologically active molecule of interest to easily diffuse from the capsule into the surrounding host tissue.

The capsule can be made from a biocompatible material. A "biocompatible material" is a material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics. Preferably the capsule of this invention will be similar to those described by PCT International patent applications WO 92/19195 or WO 95/05452, incorporated by reference; or U.S. Pat. Nos. 5,639,275; 5,653,975; 4,892,538; 5,156,844; 5,283,187; or U.S. Pat. No. 5,550,050, incorporated by reference. Such capsules allow for the passage of metabolites, nutrients and therapeutic substances while minimizing the detrimental effects of the host immune system. Components of the biocompatible material may include a surrounding semipermeable membrane and the internal cell-supporting scaffolding. Preferably, the transformed cells are seeded onto the scaffolding, which is encapsulated by the permselective membrane. The filamentous cell-supporting scaffold may be made from any biocompatible material selected from the group consisting of acrylic, polyester, polyethylene, polypropylene polyacetonitrile, polyethylene teraphthalate, nylon, polyamides, polyurethanes, polybutester, silk, cotton, chitin, carbon, or biocompatible metals. Also, bonded fiber structures can be used for cell implantation (U.S. Pat. No. 5,512,600, incorporated by reference). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere (PCT International patent application Ser. No. 98/05304, incorporated by reference). Woven mesh tubes have been used as vascular grafts (PCT International patent application WO 99/52573, incorporated by reference). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

Various polymers and polymer blends can be used to manufacture the surrounding semipermeable membrane, including polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Preferably, the surrounding semipermeable membrane is a biocompatible semipermeable hollow fiber membrane. Such membranes, and methods of making them are disclosed by U.S. Pat. Nos. 5,284,761 and 5,158,881, incorporated by reference. The surrounding semipermeable membrane is formed from a polyether sulfone hollow fiber, such as those described by U.S. Pat. No. 4,976,859 or U.S. Pat. No. 4,968,733, incorporated by reference. An alternate surrounding semipermeable membrane material is poly(acrylonitrile/covinyl chloride).

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired.

When macrocapsules are used, preferably between 10³ and 10⁸ cells are encapsulated, most preferably 10⁵ to 10⁷ cells are encapsulated in each device. Dosage may be controlled by implanting a fewer or greater number of capsules, preferably between 1 and 10 capsules per patient.

The scaffolding may be coated with extracellular matrix (ECM) molecules. Suitable examples of extracellular matrix molecules include, for example, collagen, laminin, and fibronectin. The surface of the scaffolding may also be modified by treating with plasma irradiation to impart charge to enhance adhesion of cells.

Any suitable method of sealing the capsules may be used, including the use of polymer adhesives or crimping, knotting and heat sealing. In addition, any suitable "dry" sealing method can also be used, as described, e.g., in U.S. Pat. No. 5,653,687, incorporated by reference.

The encapsulated cell devices are implanted according to known techniques. Many implantation sites are contemplated for the devices and methods of this invention. These implantation sites include, but are not limited to, the central nervous system, including the brain, spinal cord (see, U.S. Pat. Nos. 5,106,627, 5,156,844, and 5,554,148, incorporated by reference), and the aqueous and vitreous humors of the eye (see, PCT International patent application WO 97/34586, incorporated by reference).

The ARPE-19 cell line is a superior platform cell line for encapsulated cell based delivery technology and is also useful for unencapsulated cell based delivery technology. The ARPE-19 cell line is hardy (i.e., the cell line is viable under stringent conditions, such as implantation in the central nervous system or the intra-ocular environment). ARPE-19 cells can be genetically modified to secrete a substance of therapeutic interest. ARPE-19 cells have a relatively long life span. ARPE-19 cells are of human origin. Furthermore, encapsulated ARPE-19 cells have good in vivo device viability. ARPE-19 cells can deliver an efficacious quantity of growth factor. ARPE-19 cells elicit a negligible host immune reaction. Moreover, ARPE-19 cells are non-tumorigenic.

Methods and apparatus for implantation of capsules into the CNS are described in US 5,487,739.

In one aspect the invention relates to a biocompatible capsule comprising: a core comprising living packaging cells that secrete a viral vector for infection of a target cell, wherein the viral vector is a vector according to the invention; and an external jacket surrounding said core, said jacket comprising a permeable biocompatible material, said material having a porosity selected to permit passage of retroviral vectors of approximately 100 nm diameter thereacross, permitting release of said viral vector from said capsule.

Preferably, the core additionally comprises a matrix, the packaging cells being immobilized by the matrix. According to one embodiment, the jacket comprises a hydrogel or thermoplastic material.

Methods and devices for encapsulation of packaging cells are disclosed in US 6,027,721 hereby incorporated by reference in its entirety.

### IX. Medical use and methods of treatment

In one aspect the invention relates to the use of the vector according to the invention for the preparation of a medicament for the treatment of a nervous system disorder. The nervous system disorder can be a disorder of the peripheral nervous system or the central nervous system.

By treatment is not only intendec curative treatment but also preventive (not absolute prevention) or prophylactic treatment. Treatment may also be ameliorative or symptomatic.

Preferably the CNS disorder is a neurodegenerative or neurological disease. The neurodegenerative or neurological disease may be a disease involving lesioned and traumatic neurons, such as traumatic lesions of peripheral nerves, the medulla, the spinal cord, cerebral ischaemic neuronal damage, neuropathy, peripheral neuropathy, neuropathic pain, Alzheimer's disease, Huntington's disease, Parkinson's disease, amyotrophic lateral sclerosis, memory impairment connected to dementia. The neurodegenerative component of multiple sclerosis is also treatable according to the present invention.

According to one preferred embodiment of the invention the neurodegenerative disease is Parkinson's disease (see the examples).

In another preferred embodiment, the disease is Amyltrophic Lateral Sclerosis or spinal cord injury.

The vectors of the present invention can also be used for the treatment of eye diseases, such as retinitis pigmentosa, macular degeneration, glaucoma, diabetic retinopathy.

Nervous system diseases may be treated by administering to an individual in need thereof a therapeutically effective amount of the virus vector of the invention; or a therapeutically effective amount of the pharmaceutical composition of the invention.

For Parkinson's disease delivery of capsules and virus vector is described above under "Dosing requirements and delivery protocol". For ALS and spinal cord injury, capsules with Neurturin secreting cells or virus vector may be delivered to the intrathecal space, intraventricularly or intralumbarly. For spinal cord injury, delivery may also be to the area with lessioned and/or traumatic neurons. Delivery of capsules or virus vector may be to the cervical/lumbar enlargement in proximity to the lower motor neurons. In particular for ALS, modified rabies virus coding for an expression construct of the present invention may be injected into afflicted muscle tissue, whereby retrograde transport to the affected motor neurons is accomplished.

Although the present invention focuses on in vivo gene therapy, it is also contemplated that, nervous system diseases can be treated by transplanting to an individual in need thereof:
i. a therapeutically effective amount of the transduced cells according to the invention;
ii. an implantable device comprising transduced cells; or
iii. a biocompatible device comprising a packaging cell line.

Said transplantation may comprise an autologous transplant, an allogeneic transplant or a xenogeneic transplant.

Most, if not all, ophthalmic diseases and disorders are associated with one or more of three types of indications: (1) angiogenesis, (2) inflammation, and (3) degeneration. To treat these disorders, the virus vectors, therapeutic cells and encapsulated cells of the present invention permit delivery of Neurturin to the eye.

Delivery of virus vector according to the present invention may be done using subretinal injections, intravitreal injection, or transcleral injection.

Diabetic retinopathy, for example, is characterized by angiogenesis and retinal degeneration. This invention contemplates treating diabetic retinopathy by implanting devices delivering NTN either intraocularly, preferably in the vitreous, or periocularly, preferably in the sub-Tenon's region. We most prefer delivery of capsules, naked cells, or virus vector into the vitreous for this indication. Retinopathy includes, but is not limited to, diabetic retinopathy, proliferative vitreoretinopathy, and toxic retinopathy.

Uveitis involves inflammation and secondary degeneration. This invention contemplates treating uveitis by intraocular, preferably vitreal or anterior chamber, implantation of capsules or naked cells secreting NTN or by administering virus vector according to the invention to the vitreous.

Retinitis pigmentosa, by comparison, is characterized by primary retinal degeneration. This invention contemplates treating retinitis pigmentosa by intraocular, preferably vitreal, placement of devices or naked cells secreting NTN or by administering virus vector according to the invention to the vitreous.

Age-related macular degeneration involves both angiogenesis and retinal degeneration. This invention contemplates treating this disorder by using the capsules or naked cells of the invention to deliver NTN intraocularly, preferably to the vitreous, or by using the virus vector according to the invention to deliver NTN intraocularly, preferably to the vitreous. Age-related macular degeneration includes, but is not limited to, dry age-related macular degeneration, exudative age-related macular degeneration, and myopic degeneration.

Glaucoma is characterized by increased ocular pressure and loss of retinal ganglion cells. Treatments for glaucoma contemplated in this invention include delivery of NTN that protect retinal cells from glaucoma associated damage, delivered intraocularly, preferably intravitreally either by capsules, virus vector of naked cells.

Intraocularly, preferably in the vitreous, we contemplate delivery of Neuturin in a dosage range of 50 pg to 500 ng, preferably 100 pg to 100 ng, and most preferably 1 ng to 50 ng per eye per patient per day. For periocular delivery, preferably in the sub-Tenon's space or region, slightly higher dosage ranges are contemplated of up to 1 µg per patient per day.

The present invention may be useful for the treatment of ocular neovascularization, a condition associated with many ocular diseases and disorders and accounting for a majority of severe visual loss. For example, we contemplate treatment of retinal ischemia-associated ocular neovascularization, a major cause of blindness in diabetes and many other diseases; corneal neovascularization, which predisposes patients to corneal graft failure; and neovascularization associated with diabetic retinopathy, central retinal vein occlusion, and possibly age-related macular degeneration.

In one embodiment of the present invention, living cells secreting bioactive Neurturin are encapsulated and surgically inserted (under retrobulbar anesthesia) into the vitreous of the eye. For vitreal placement, the device may be implanted through the sclera, with a portion of the device or tether protruding through the sclera. Most preferably, the entire body of the device is implanted in the vitreous, with no portion of the device protruding into or through the sclera. Preferably the device is tethered to the sclera (or other suitable ocular structure). The tether may comprise a suture eyelet, or any other suitable anchoring means (see e.g. US 6,436,427). The device can remain in the vitreous as long as necessary to achieve the desired prophylaxis or therapy. Such therapies for example include promotion of neuron or photoreceptor survival or repair, or inhibition and/or reversal of retinal or choroidal neovascularization, as well as inhibition of uveal, retinal, and optic nerve inflammation. This embodiment is preferable for delivering NTN to the retina.

With vitreal placement, NTN may be delivered to the retina or the RPE.

In another embodiment, cell-loaded devices are implanted periocularly, within or beneath the space known as Tenon's capsule. This embodiment is less invasive than implantation into the vitreous and thus is generally preferred. This route of administration also permits delivery of NTN to the RPE or the retina. This embodiment is especially preferred for treating choroidal neovascularization and inflammation of the optic nerve and uveal tract. In general, delivery from this implantation site will permit circulation of NTN to the choroidal vasculature, the retinal vasculature, and the optic nerve.

According to this embodiment we prefer periocular delivery (implanting beneath Tenon's capsule) of NTN to the choroidal vasculature to treat macular degeneration (choroidal neovascularization).

Delivery of NTN directly to the choroidal vasculature (periocularly) or to the vitreous (intraocularly) using the devices and methods of this invention may permit the treatment of poorly defined or occult choroidal neovascularization. It may also provide a way of reducing or preventing recurrent choroidal neovascularization via adjunctive or maintenance therapy.

Dosage can be varied by any suitable method known in the art. This includes changing (1) the number of cells per device, (2) the number of devices per eye, or (3) the level of NTN production per cell. We prefer use of 10³ to 10⁸ cells per device, more preferably 5*10⁴ to 5*10⁶ cells per device.

### X. Host cells

In one aspect the invention relates to isolated host cells transduced with the vector according to the invention. These cells preferably are mammalian host cells because these are capable of secreting and processing the encoded Neurturin correctly.

Preferred species include the group consisting of rodent (mouse, rat), rabbit, dog, cat, pig, monkey, human being.

Examples of primary cultures and cell lines that are good candidates for transduction with the vectors of the present invention include the group consisting of CHO, HEK293, COS, PC12, HiB5, RN33b, neuronal cells, foetal cells, ARPE-19, MDX12, C2C12, HeLa, HepG2, striatal cells, neurons, astrocytes, interneurons.

The invention also relates to cells suitable for biodelivery of NTN via naked or encapsulated cells, which are genetically modified to overexpress NTN, and which can be transplanted to the patient to deliver bioactive NTN polypeptide locally. Such cells may broadly be referred to as therapeutic cells.

In a preferred embodiment of the invention, a therapeutic cell line has not been immortalised with the insertion of a heterologous immortalisation gene. As the invention relates to cells which are particularly suited for cell transplantation, whether as naked cells or - preferably as encapsulated cells, such immortalised cell lines are less preferred as there is an inherent risk that they start proliferating in an uncontrolled manner inside the human body and potentially form tumours.

Preferably, the therapeutic cell line is a contact inhibited cell line. By a contact inhibited cell line is intended a cell line which when cultured in Petridishes grow to confluency and then substantially stop dividing. This does not exclude the possibility that a limited number of cells escape the mono-layer. Contact inhibited cells may also be grown in 3D, e.g. inside a capsule. Also inside the capsules, the cells grow to confluency and then significantly slow down proliferation rate or completely stop dividing. A particularly preferred type of cells include epithelial cells which are by their nature contact-inhibited and which form stable monolayers in culture.

Even more preferred are retinal pigment epithelial cells (RPE cells). The source of RPE cells is by primary cell isolation from the mammalian retina. Protocols for harvesting RPE cells are well-defined (Li and Turner, 1988, Exp. Eye Res. 47:911-917; Lopez et al., 1989, Invest. Ophthalmol. Vis. Sci. 30:586-588) and considered a routine methodology. In most of the published reports of RPE cell cotransplantation, cells are derived from the rat (Li and Turner, 1988; Lopez et al., 1989). According to the present invention RPE cells are derived from humans. In addition to isolated primary RPE cells, cultured human RPE cell lines may be used in the practice of the invention.

In another embodiment the therapeutic cell line is selected from the group consisting of:
human fibroblast cell lines, human astrocyte cell lines, human mesencephalic cell lines, and
human endothelial cell lines, preferably immortalised with TERT, SV40T or vmyc.

The method for generating an immortalised human astrocyte cell lines has previously been described (Price TN, Burke JF, Mayne LV. A novel human astrocyte cell line (A735) with astrocyte-specific neurotransmitter function. In Vitro Cell Dev Biol Anim. 1999 May;35(5):279-88.). This protocol may be used to generate astrocyte cell lines.

The following three modifications of that protocol are preferably made to generate additional human astrocyte cell lines.

Human foetal brain tissue dissected from 5-12 weeks old foetuses may be used instead of 12-16 weeks old tissue.
The immortalisation gene *v-myc,* or TERT (telomerase) may be used instead of the *SV40 T antigen.*
Retroviral gene transfer may be used instead of transfection with plasmids by the calcium phosphate precipitation technique.

### XI Support matrix for Neurturin producing cells

The present invention further comprises culturing Neurturin producing cells in vitro on a support matrix prior to implantation into the mammalian nervous system or the eye. The preadhesion of cells to microcarriers prior to implantation is designed to enhance the long-term viability of the transplanted cells and provide long-term functional benefit.

To increase the long term viability of the transplanted cells, i.e., transplanted NTN-secreting cells, the cells to be transplanted can be attached in vitro to a support matrix prior to transplantation. Materials of which the support matrix can be comprised include those materials to which cells adhere following in vitro incubation, and on which cells can grow, and which can be implanted into the mammalian body without producing a toxic reaction, or an inflammatory reaction which would destroy the implanted cells or otherwise interfere with their biological or therapeutic activity. Such materials may be synthetic or natural chemical substances, or substances having a biological origin.

The matrix materials include, but are not limited to, glass and other silicon oxides, polystyrene, polypropylene, polyethylene, polyvinylidene fluoride, polyurethane, polyalginate, polysulphone, polyvinyl alcohol, acrylonitrile polymers, polyacrylamide, polycarbonate, polypentent, nylon, amylases, natural and modified gelatin and natural and codified collagen, natural and modified polysaccharides, including dextrans and celluloses (e.g., nitrocellulose), agar, and magnetite. Either resorbable or non-resorbable materials may be used. Also intended are extracellular matrix materials, which are well-known in the art. Extracellular matrix materials may be obtained commercially or prepared by growing cells which secrete such a matrix, removing the secreting cells, and allowing the cells which are to be transplanted to interact with and adhere to the matrix. The matrix material on which the cells to be implanted grow, or with which the cells are mixed, may be an indigenous product of RPE cells. Thus, for example, the matrix material may be extracellular matrix or basement membrane material, which is produced and secreted by RPE cells to be implanted.

To improve cell adhesion, survival and function, the solid matrix may optionally be coated on its external surface with factors known in the art to promote cell adhesion, growth or survival. Such factors include cell adhesion molecules, extracellular matrix, such as, for example, fibronectin, laminin, collagen, elastin, glycosaminoglycans, or proteoglycans or growth factors.

Alternatively, if the solid matrix to which the implanted cells are attached is constructed of porous material, the growth- or survival promoting factor or factors may be incorporated into the matrix material, from which they would be slowly released after implantation in vivo.

When attached to the support according to the present invention, the cells used for transplantation are generally on the "outer surface" of the support. The support may be solid or porous. However, even in a porous support, the cells are in direct contact with the external milieu without an intervening membrane or other barrier. Thus, according to the present invention, the cells are considered to be on the "outer surface" of the support even though the surface to which they adhere may be in the form of internal folds or convolutions of the porous support material which are not at the exterior of the particle or bead itself.

The configuration of the support is preferably spherical, as in a bead, but may be cylindrical, elliptical, a flat sheet or strip, a needle or pin shape, and the like. A preferred form of support matrix is a glass bead. Another preferred bead is a polystyrene bead.

Bead sizes may range from about 10 µm to 1 mm in diameter, preferably from about 90 µm to about 150 µm. For a description of various microcarrier beads, see, for example, isher Biotech Source 87-88, Fisher Scientific Co., 1987, pp. 72-75; Sigma Cell Culture Catalog, Sigma Chemical Co., St, Louis, 1991, pp. 162-163; Ventrex Product Catalog, Ventrex Laboratories, 1989; these references are hereby incorporated by reference. The upper limit of the bead's size may be dictated by the bead's stimulation of undesired host reactions, which may interfere with the function of the transplanted cells or cause damage to the surrounding tissue. The upper limit of the bead's size may also be dictated by the method of administration. Such limitations are readily determinable by one of skill in the art.

### XII. In vitro production of Neurturin

In another aspect the invention relates to a mammalian cell capable of secreting neurturin or a functional equivalent thereof in amounts in excess of 500 ng/10⁶ cells/24 hours. Preferably the cells are capable of secreting at least of 1000 ng/10⁶ cells/24 hours, more preferably at least 5000, more preferably at least 10,000, more preferably at least 15,000, more preferably at least 20,000, more preferably at least 25,000, more preferably at least 30,000, more preferably at least 35,000. As shown by Example 1, the best plasmid transfected ARPE19 cells produce in excess of 20,000 ng/10⁶ cells/24 hours. Expression can be increased even further by the inclusion of enhancer elements such as WPRE (US 6,136,597). Compared to the prior art BHK cells (Hoane et al 2000, Experimental Neurology 162:189-193), these amounts are very high.

Such high producing cells may be selected from the group consisting of ARPE19 cells, CHO cells, BHK cells, R1.1 cells, COS cells, killer cells, helper T-cells, cytotoxic T-lymphocytes and macrophages. HEK293 cells and HiB5 cells are also suitable producer cells.

Neurturin or a truncated or mutated form thereof or a bioactive sequence variant can thus be produced in significant quantities by culturing these cells and recovering the neurturin from the culture medium. Mammalian produced Neurturin does not need to be refolded in order to be bioactive. A further advantage is that Neurturin is secreted as a mature peptide and does not include the pro-peptide. It has been shown by the present inventors that expressing Neuturin as a pre-pro-Neurturin results in secretion of pro-Neurturin, which does not bind GFRα1 or GFRα2 and therefore is not bioactive.

These Neurturin producing cells can likewise be used for therapeutic purposes and be implanted either as naked (supported or unsupported) or as encapsulated cells for localised delivery of bioactive Neurturin.

### Examples:

### Example 1: In vitro transfection with Neurturin constructs

### Materials & Methods

### Cloning of genomic NTN sequence

Human genomic NTN was cloned from genomic DNA purified from the HEK293 cell line (ATCC, USA) using the PureGene kit (Gentra, Biotech Line, Denmark). PCR with the primers NTNgenom.1s +BamHI (5'-TATAGGATCCGGAGGACACCAGCATGTAG-3', SEQ ID No. 52) and NTNgenom.1as (5'-TCGCCGAGGATGAATCACCA-3'; SEQ ID No. 53) was carried out using HEK293 gDNA as template. pfx polymerase (Invitrogen, Denmark) was used in its corresponding buffer supplemented with 5% DMSO (Sigma-Aldrich, Denmark). The obtained PCR fragment was cloned in pNS1n (NeuroSearch), a custom-designed derivative of pcDNA3neo (InVitrogen), using the BamHI and XhoI restriction sites, resulting in the vector pNS1 n.hNTNgenom. This resulted in cloning of the sequence coding for mature NTN (SEQ ID No. 7).

### Vector construction

Cloning of the IgSP-NTN expression vector pNS1n.IgSP.NTN: The mature fragment of NTN was amplified by PCR from the pNS1n.hNTNgenom vector using the primers NTNs-IgSP.Flap (5'-GGTGAATTCGGCGCGGTTGGGGGCGCGGCCT-3', SEQ ID No 54) and NTN-594as+XhoI (5'-TATACTCGAGTCACACGCAGGCGCACTCGC-3'; SEQ ID No 55). In a second PCR reaction, the IgSP sequence was amplified from the pNUT-IgSP-CNTF vector (US 6,361,771) using the primers IgSPKozak1s+BamHI (5'-TATAGGATCCGCCACCATGAAATGCAGCTGGGTTATC-3'; SEQ ID No. 56) and IgSPas-NTN.Flap (5'-CCAACCGCGCCGAATTCACCCCTGTAGAAAG-3'; SEQ ID No. 57). In the third PCR reaction, the two fragments were fused by overlap. Equal amounts of the two products were used as template with the primers IgSPKozak1s+BamHI and NTN-594as+XhoI.

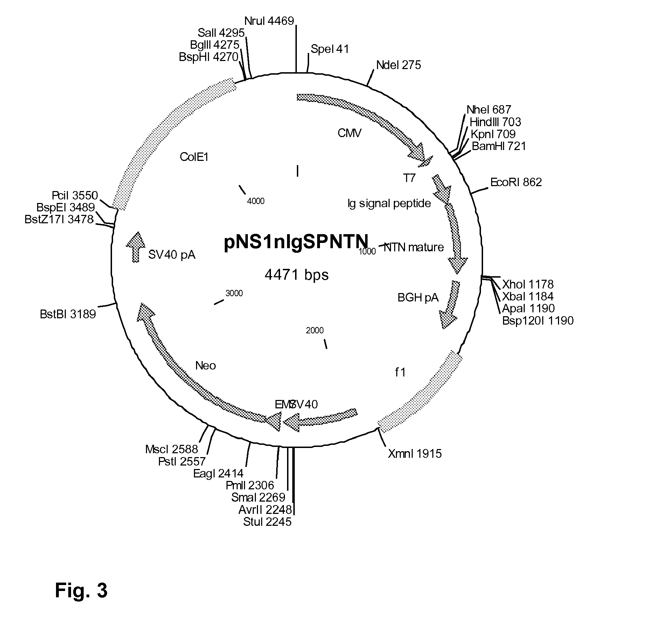

To generate a plasmid-based expression vector the resulting fragment was cloned in pNS1n digested with BamHI/XhoI. In this vector, the IgSP-NTN sequence is placed under transcriptional control of the CMV promoter (see Figure 3). Furthermore, the vector contains the Neo gene that confers G418 resistance when expressed in mammalian cells. The nucleotide sequence of the fragment coding for IgSP-NTN is set forth in Figure 13.

### Cell culture

ARPE-19, a spontaneously arising human retinal pigment epithelial cell line (Dunn et al. 1996), was grown at 37°C in 5% CO2. Growth medium consisted of DMEM/Nutrient Mix F-12 with Glutamax (Invitrogen, Denmark) supplemented with 10% fetal bovine serum (Sigma-Aldrich, Denmark). Cells were passaged approximately twice a week in a 1:5 ratio.

### Transient transfection studies

ARPE-19 cells were transfected with a series of NTN expression vectors. Briefly, cells were seeded in 6-well plates (Corning Costar, Biotech Line, Denmark) at a density of 10⁵ cells/well. The next day, cells were transfected with NTN expression plasmids in duplicate wells using Fugene6 (Roche, Germany) according to the manufacturer's specifications. 72 h post-transfection, sample aliquots were taken from cell supernatants for RetL2 and NTN ELISAs. Cells were harvested for western blot analysis.

### Stable transfections

ARPE-19 cells were seeded in T150 "peel-off" flasks (TPP, Switzerland) at a density of 2.4*10⁶ cells/flask. Cells were transfected with 10 µg DNA/flask using Fugene6. 72 h post-transfection, 800 µg/ml G418 (Sigma-Aldrich, Denmark) was added to the growth media for selection of stable clones. After formation of distinct clones, single clones were expanded for further analysis.

### NTN ELISA

In this conventional immunoassay, NTN is bound and detected from samples using NTN-specific antibodies. In short, Maxisorp plates (Nunc, Denmark) were coated by incubation with 1 µg/ml monoclonal anti-human NTN antibody (#MAB387, R&D Systems, TriChem, Denmark) in coating solution (0.0025 M Na₂CO₃ / 0.0025 M NaHCO₃, pH=8.2) for 16 h at 4 °C. After wash in PBST (0.05% Tween-20 (Sigma-Aldrich, Denmark) in PBS (Invitrogen, Denmark)), wells were blocked in blocking buffer (1% bovine serum albumin (Sigma-Aldrich, Denmark) and 5% sucrose in PBS) for 1 h at room temperature. After wash in PBST, wells were incubated with dilutions in ARPE-19 growth medium of media samples from NTN-prodicing cells and recombinant NTN (#387-NE, R&D Systems, TriChem, Denmark) as standard for 3 h at room temperature. 1µg/ml polyclonal anti-human NTN antibody (#AF387, R&D Systems, TriChem, Denmark) in blocking buffer was added to the wells and incubated for 16 h at 4 °C. After wash in PBST, wells were incubated for 2 h at room temperature in 0.02% anti-goat-HRP (DAKO, Denmark) in blocking buffer supplemented with 1% normal mouse serum (DAKO, Denmark). Following wash in PBST, TMB substrate solution (Promega, Ramcon, Denmark) was added and incubation carried out for 15 min at room temperature. The color formation was stopped by addition of 1 N HCl to the wells, and A₄₅₀ was measured using an ELX-800 plate reader (Cambrex, Denmark).

### RetL2 ELISA

The RetL2 ELISA detects binding of a Ret-AP conjugate to a complex of NTN bound to the NTN-specific GFRα2 receptor. Briefly, an Opti-plate plate (Packard Instruments, Perkin Elmer, Denmark) was coated with 100µl 1µg/ml Goat anti human Fc (Jackson Immunoresearch Laboratories, TriChem, Denmark) in 50mM NaHCO₃ (pH=9.6) for 16 h at 4°C. After wash in PBST, wells were blocked in 0.2% I-Block (Tropix, Roche, Denmark) in PBST for 1 hr at room temperature, followed by a brief wash in PBST. Samples from NTN-producing cells and standard dilutions of recombinant NTN in ARPE-19 growth medium were subsequently incubated in the wells with 1µg/ml GFRα2/Fc fusion protein (R&D Systems, TriChem, Denmark) in RET-AP conditioned media (Biogen, USA) for 1.5 h at room temperature. Wells were then washed first in PBST and then in AP-buffer (200 mM Tris (pH=9.8), 10 mM MgCl₂) followed by 30 min incubation with 10% Sapphire Enhancer (Tropix, Roche, Denmark) and 2% CSPD (Tropix, Roche, Denmark) in AP-buffer. Luminescence was quantified.

### Western blotting

Cells were washed in PBS and lysed in 96 °C sample buffer (2% SDS, 0.4 M Tris (ph=8.0), 10 mM dithiothreitol and 0.25 Na₃VO₄). Proteins were separated by denaturing SDS-PAGE using the MultiPhor II system according to the manufacturer's recommendations (Amersham Pharmacia, Denmark) and blotted to PVDF membranes (BioRad, Denmark). For immunostaining of membranes, standard Western blotting techniques were employed (Maniatis, XX). The polyclonal NTN #AF477 antibody (R&D Systems, TriChem, Denmark) was used as detecting antibody. Membranes were developed using the ECL system (Amersham Pharmacia, Denmark) and subjected to film exposure.

### Results

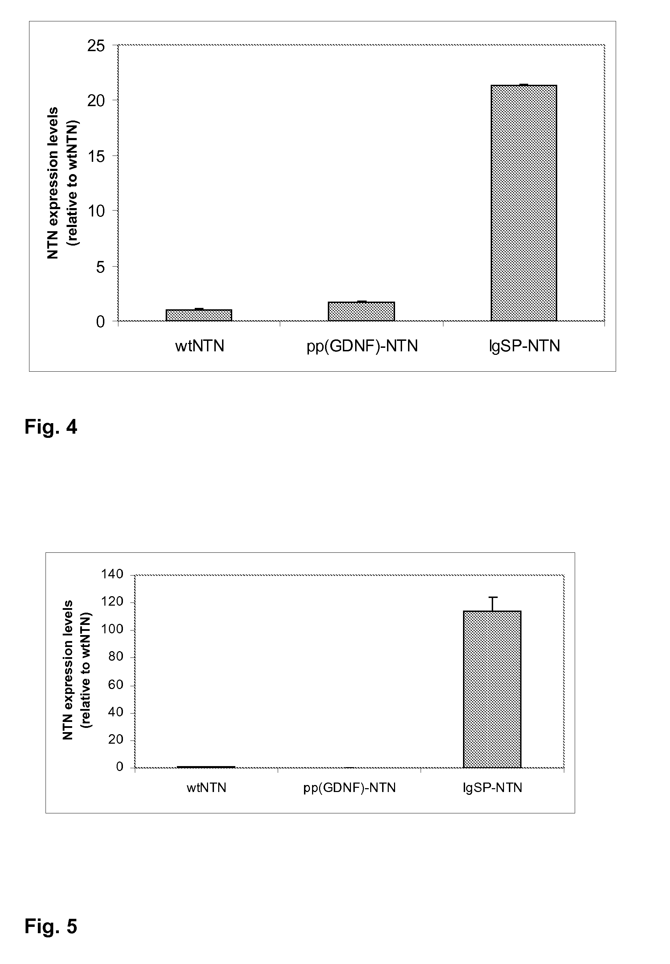

The IgSP element mediates a significant increase in NTN release from cultured cells
From transiently transfected cells, the IgSP expression vector resulted in a strongly increased NTN secretion to the cell culture supernatant compared to the wtNTN and pp(GDNF)-NTN plasmids. Use of the GDNF signal peptide and propeptide also improved the secretion compared to the native NTN signal peptide although not to the same extent as the IgSP element. The positive effect of IgSP on NTN secretion could be detected by standard ELISA techniques using antibodies raised against NTN (fig. 4) as well as by functional RetL2 ELISA assays (fig. 5), where binding of NTN to its receptor, GFRα2, is detected by formation of a ternary complex with the GFR co-receptor Ret. Notably, replacing the pre-pro peptide of wild-type NTN with that of GDNF, a factor known to be abundantly expressed from recombinant cells, did not result in an evident increase in NTN protein expression, as measured by both ELISAs.

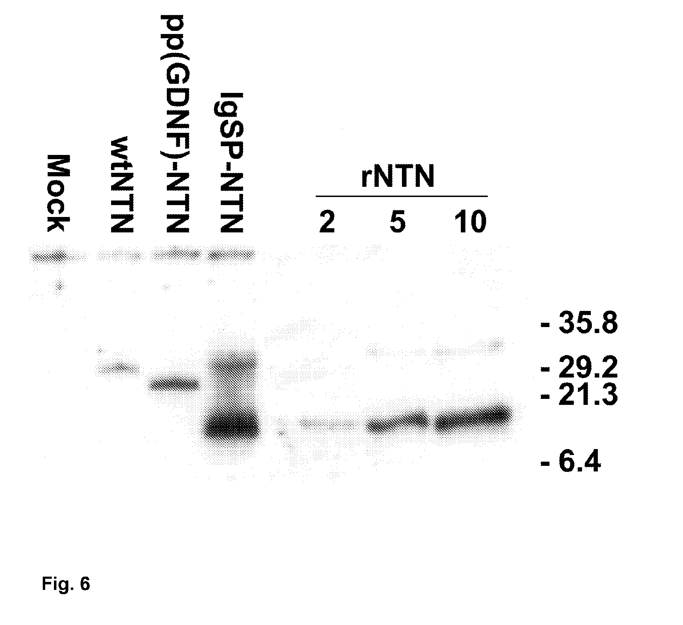

Absence of a pro-peptide element appears to affect intracellular NTN protein processing
Proteins from lysates of transfected cells were separated by denaturing gel electrophoresis with recombinant neurturin as control. Only in the lane loaded with lysate from IgSP-NTN transfected cells, a band similar in size to recombinant neurturin could be observed (fig. 6, the band located between the 6.4 and 21.3 kDa markers). For both the wtNTN and pp(GDNF)-NTN transfected cells, the prominent protein detected by the NTN antibody had a significantly higher molecular weight than recombinant NTN and IgSP from cells. This, combined with the observation that IgSP-NTN is significantly better expressed in vitro, indicates that IgSP-NTN, but not wtNTN and pp(GDNF)-NTN, are correctly processed for secretion by intracellular mechanisms.

### High NTN-expression from isolated clones

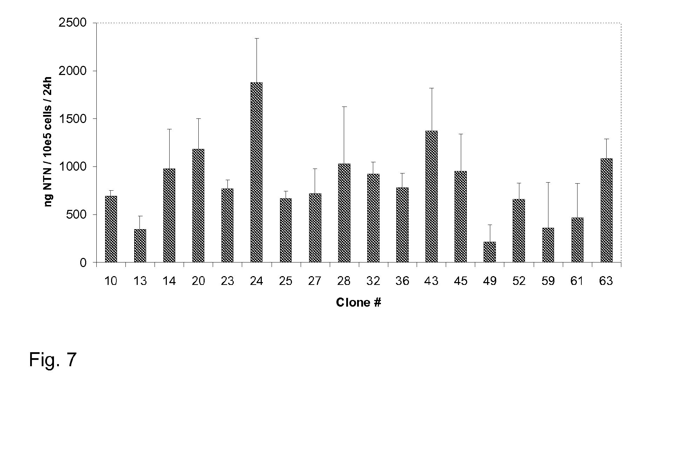

ARPE-19 cells stably expressing NTN were isolated by transfection with pNS1n.IgSP.NTN followed by selection of clones with G418. A range of NTN expression levels was observed from the isolated clones (Figure 7). The highest producer, ARPE-19/pNS1n.IgSP.NTN #24 produced up to 2000 ng NTN / 10⁵ cells / 24 h.

### Example 2: In vivo transduction of rats with Neurturin.

### Materials & Methods

Generation of a lentiviral IgSP-NTN construct and virus stocks

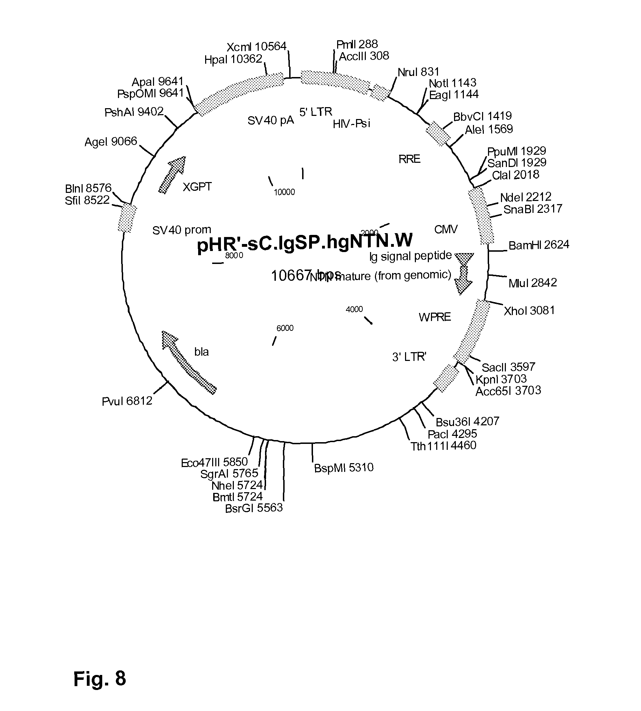

To generate a lentiviral construct, the IgSP-NTN fragment (example 1) was cloned into pHR'-CMV-GFP-W-SIN by cutting out GFP with BamHI and XhoI and inserting IgSP-NTN as a BamHI/XhoI fragment in stead (see Figure 8). pHR'-CMV-GFP-W-SIN is a derivative of a self-inactivating lentiviral transfer construct, pHR'-SIN₋₁₈ including a WPRE element (Dull et al., J.Virol., 72(11):8463-71 (1998); Zufferey et al., J.Virol., 72(12):9873-80(1998): Zufferey et al. J.virol., 73 (4):2886-92 (1999)).

Replication-defective LV-sC.IgSP.NTN.W virus particles are generated by co-transfection of pHsC.IgSP.NTN.W with pMD.G (VSV-G pseudo-typing vector) and pBR8.91 (packaging vector) (Zufferey et al., Nat. Biotech., 15:871-75(1997)) into 293T cells providing the required viral proteins in *trans.* Briefly, 293T cells cultured in DMEM with 4.5 g/l glucose and glutamax (Life Technologies, 32430-027) supplemented with 10 % FCS (Life Technologies, 10099-141) are seeded in T75 flasks (2x106 cells /flask) the day before transfection. For each T75 flask cells are transfected with 5 µg pMD.G, 15 µg pBR8.91 and 20 µg of transfer vector using Lipofectamine+ following the manufacturer's instructions. Virus containing cell supernatant is collected 2-3 days after the transfection, filter-sterilized through a 0.45 µm cellulose acetate or polysulphonic filter and concentrated by ultracentrifugation at 50,000xg for 90 min. at 4°C. After a second round of ultracentrifugation, the concentrated virus pellet is resuspended in DMEM, aliquoted and stored at -80°C. To determine virus titer, reverse transcriptase (RT) activity is assayed (Cepko and Pear, Current Protocols in Molecular Biology, 9.13.5-6, supplement 36) and transducing units (TU)/ml calculated from the determined RT activity using an EGFP lentivirus with known transducing activity as reference.

Similar virusbatches were made with human and murine pre-pro-NTN, pre-pro-GDNF, and GFP.

### Surgical Procedures

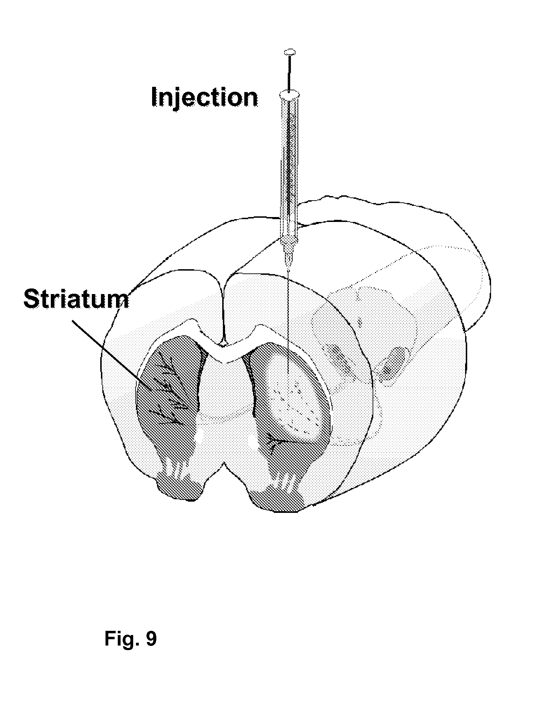

A total of 21 young adult female Sprague-Dawley rats (B&K Universal, Stockholm, Sweden) were used and housed under 12 h light:dark cycle with free access to rat chow and water. Virus injections and 6-OHDA lesion were performed according to Rosenblad et al (2000). The injection procedure is illustrated in Figure 9. Briefly, under isoflourane anesthesia (1.5-2%) animals were injected with a rLV vector carrying the cDNA for GFP, hNTN, mNTN, IgSP-hNTN or GDNF (n = 6/group). Four deposits (0.5 µl/deposit of 1×10⁸ t.u./mL) were made into the striatum along two needle tracts at the following coordinates: AP=1.0 mm, ML=2.6 mm, DV₁ =5.0 mm DV₂ =4.5 mm, and AP =0.0 mm, ML =3.7 mm, DV₁ =5.0 mm, DV₂ =4.5 mm. The tooth bar was set at -3.3 mm. 14 days after rLV injections the animals were reanestesized and with a 10-µl Hamilton syringe a single deposit of 20 µg 6-OHDA (Sigma; calculated as free base and dissolved in 3 µl ice-cold saline supplemented with 0.02% ascorbic acid) was injected into the right striatum at the following coordinates: AP= 1.0 mm; ML=3.0 mm relative to bregma; DV=5.0 mm relative to the dura and incisor bar set to 0.0 mm.

### Histology

At 21 days after the 6-OHDA injection the animals were deeply anesthetized with sodiumpentobarbital and transcardially perfused with saline for one min followed by 200 ml ice-cold 4% PFA in 0.1 M phosphate buffer (pH 7.4). The brains were dissected and postfixed in the same fixative for 3-4 h and then transferred into 25% sucrose/0.1 M phosphate buffer for 48 h. Five series of 40-µm sections were cut on a freezing microtome. Immunohistochemistry for hNTN and hGDNF was performed by incubating sections with goat-anti-hNTN or goat-anti-hGDNF primary antibodies (R&D Systems; 1:2000 in phosphate buffered saline containing 2% normal horse serum and 0.25% triton X-100) followed by incubation with biotinylated horse-anti-mouse (Jackson Immunoresearch, USA) for two hours, and avidin-biotin complex (ABC) kit according to manufacturers instructions (Vector Laboratories, USA). Finally, the color reaction was developed using 3'3'-diaminobenzidine as chromogen.

### Results

### Immunohistochemical localization of hNTN:

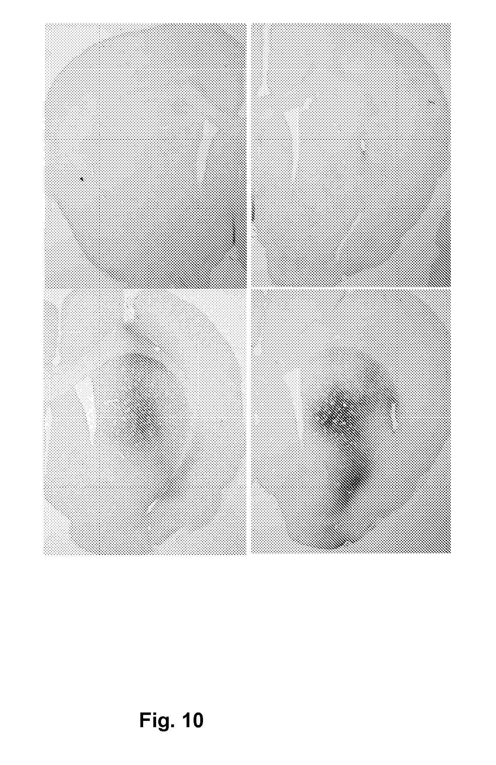

Inspection of the sections stained for hNTN show that in the striatum of animals receiving wild type hNTN encoding lentiviral vector injections (rLV-hNTN) no extracellular immunoreactivity was found in the vicinity of transduced striatal cells (Fig. 10). In contrast, animals receiving injections of rLV-IgSPhNTN had a marked staining pattern with immunoreactive material diffusely spread (extracellularly) in the striatum around the transduction site (Fig. 10).

### Functionality of hNTN:

The neuroprotective effect of hNTN was assessed by counting the nigral TH+ (dopaminergic) neurons. Compared to the intact side, animals receiving a 6-OHDA lesion and rLV-GFP virus had clearly fewer TH+ neuron remaining in the substantia nigra (23+/-3.4%). Animals receiving rLV-hNTN also showed a marked lesion induced reduction in TH+ neurons (30+/-7.7% remaining). By contrast, in the rLV-IgSPNTN treated group the number of TH+ neurons on the lesioned side was 91+/-1.2% of that on the intact, which was similar to what was observed in the group receiving GDNF treatment (86+/-3.2%).

### Example 3: Preparation of NTN expression constructs

*Vector constructs.* pHR'-CMV.SIN.hNTN.WPRE: Wild type human preproNTN was cloned into pHR'-CMV.SIN-PLT7.WPRE as follows: pHR'-CMV.SIN-PLT7.WPRE , which is a derivative of pHR'-CMV-SIN-18 containing the Woodchuck postregulatory element (WPRE) as (Zufferey et al. 1998; Zufferey et al., 1999), by addition of a polylinker site between the BamHI and Xhol sites (unpublished results) was digested with BamHI and Xhol. Human preproNTN was cut from vector pJDM2174 (=human preproNTN in pBluescript) (a kind gift from Jeff Milbrandt) as a BamHI, Xhol fragment, and ligated into the BamHI/XhoI digested lentiviral transfer vector. Human preproNTN was used as a control.

### pNS1n.hNTN: was prepared as described in Example 1.

pNS1n.ppGDNF.hNTN: The prepro region of GDNF was PCR amplified from a full length human GDNF clone using the following primers: 5' primer: 5'-TATAGAATTCGCCACCATGAAGTTATGGGATGTCG-3' (SEQ ID No. 58) and 3' primer: 5'-CCAACCGCGCCCTTTTCAGTCTTTTAATGG-3' (SEQ ID No. 59). The 3' primer contains 10 bases of the 5' end of mature NTN in the 3' end. Mature human NTN was PCR amplified from human full length NTN (pJDM2174) using the following primers: 5' primer: 5'-ACTGAAAAGGGCGCGGTTGGGGGCGCGGCCT-3' (SEQ ID No. 60) and 3' primer: 5'-TAGACTCGAGGTCGACGGTATC-3' (SEQ ID No. 61). The 5' primer contains 10 bases of the 3' end of the pro region of human GDNF. PreproGDNF was fused to mature NTN by overlapping PCR using the following primers: 5' primer: 5'-TATAGAATTCGCCACCATGAAGTTATGGGATGTCG-3' (SEQ ID No. 58) and 3' primer: 5'-TAGACTCGAGGTCGACGGTATC-3' (SEQ ID No. 61). The resulting preproGDNF-matureNTN fragment was digested with EcoRl and XhoI and inserted between EcoRl and XhoI sites of expression vector pNS1n (described above). The nucleotide sequence and encoded polypeptide are shown in Figure 14.

pHR'-CMV.SIN.IqSP.NTN.WPRE and pNS1n.IgSP.NTN: the signal peptide from mouse immunoglobulin heavy chain gene V-region (GenBank acc. #: M18950) (IgSP) was PCR amplified from pNUT-IgSP-hCNTF (ref. US 6,361,741) using the following primers: 5' primer: 5'-TATAGGATCCGCCACCATGAAATGCAGCTGGGTTATC-3' (SEQ ID No 56), 3' primer: 5'-CCAACCGCGCCGAATTCACCCCTGTAGAAAG-3' (SEQ ID No. 57). The 3' primer contains 10 bases from the 5' end of human, mature NTN sequence. Human, mature NTN was PCR amplified from a genomic clone of human NTN containing full length mature NTN (pNS1n.NTNgenome, see Example 1) using the following primers: 5' primer: 5'-GGTGAATTCGGCGCGGTTGGGGGCGCGGCCT-3' (SEQ ID No. 54) and 3' primer: 5'-TATACTCGAGTCACACGCAGGCGCACTCGC-3' (SEQ ID No. 55). The 5' primer contains 10 bases from the 3' end of the IgSP sequence. The IgSP-human mature NTN sequence was generated by overlapping PCR using the IgSP and human mature NTN PCR fragments (described above) as templates and the following primers: 5' primer: 5'-TATAGGATCCGCCACCATGAAATGCAGCTGGGTTATC-3' (SEQ ID No. 56) and 3' primer: 5'- TATACTCGAGTCACACGCAGGCGCACTCGC-3' (SEQ ID No. 55). The final PCR fragment containing IgSP fused to human mature NTN was digested with BamHl and XhoI and cloned between BamHl and XhoI sites of pHR'-CMV.SIN-PLT7.WPRE (described above) and pNS1n (described above). The nucleotide sequence and encoded polypeptide are shown in Figure 13.

pNS1n-dpro-NTN: Human delta-pro-NTN DNA sequence was generated in one PCR reaction using a genomic clone of human NTN containing full length mature NTN (pNS1 n.NTNgenome, see Example 1) as template and the following primers: 5' primer: 5'-TATAGGATCCGCCACCATGCAGCGCTGGAAGGCGGCGGCCTTGGCCTCAGTGCTCTGCA GCTCCGTGCTGTCCGCGCGGTTGGGGGCGCGG-3' (SEQ ID No. 62) and 3' primer: 5'-TATACTCGAGTCACACGCAGGCGCACTCGC-3' (SEQ ID No. 55). The delta-pro-NTN PCR fragment was digested with BamHl and XhoI and cloned between BamHl and XhoI sites of pNS1n (described above). The nucleotide sequence and encoded polypeptide of deltaproNTN is shown in Figure 14.

*Production of lentiviral vectors.* Replication-defective virus particles were generated by co-transfection of each of the different transfer vector constructs with pMD.G (VSV-G pseudo-typing vector) and pBR8.91 (packaging vector) (Zufferey et al., 1997) into 293T cells providing the required viral proteins in *trans.* Briefly, 293T cells cultured in DMEM with 4.5 g/l glucose and Glutamax™ (Invitrogen) supplemented with 10% FCS (Life Technologies) were seeded in 20 T75 flasks (2x10⁶ cells /flask) the day before transfection. For each T75 flask cells were transfected with 5 µg pMD.G, 15 µg pBR8.91 and 20 µg of transfer vector using Lipofectamine+™ (Invitrogen) following the manufacturer's instructions. Virus containing cell supernatant was collected 2-3 days after the transfection, filter-sterilized through a 0.45 µm cellulose acetate or polysulphonic filter and concentrated by ultracentrifugation at 50,000xg for 90 min. at 4°C. After a second round of ultracentrifugation, the concentrated virus pellet was resuspended in DMEM, aliquoted and stored at -80°C. To determine virus titer, reverse transcriptase (RT) activity was assayed (Cepko and Pear, Current Protocols in Molecular Biology, 9.13.5-6, supplement 36) and transducing untis (TU)/ml calculated from the determined RT activity using an EGFP lentivirus with known transducing activity as reference.

### Example 4: Analysis of expressed NTN proteins.

*Cell culture.* ARPE-19, a spontaneously arising human retinal pigment epithelial cell line (Dunn et al. 1996), was grown in medium consisting of DMEM/Nutrient Mix F-12 with Glutamax (Invitrogen, Denmark) supplemented with 10% fetal bovine serum (Sigma-Aldrich, Denmark). HiB5 (Renfranz et al. 1991), HEK293 and CHO cells were grown in DMEM (Invitrogen, Denmark) with 10% fetal bovine serum (Invitrogen, Denmark), and medium for CHO cells were further supplemented with 20 mg/L L-proline. ARPE-19 cells are available from ATCC (accession number CRL-2302). ARPE-19, HEK293 and CHO cells were grown at 37°C and HiB5 cells at 33°C in 5% CO₂.

*Transient transfection studies.* Cells were seeded in 6-well plates (Corning Costar, Biotech Line, Denmark) at a density of approximately 10⁵ cells/well. The next day, cells were transfected in triplicate wells with the different expression plasmids. ARPE-19 cells were transfected using Fugene6 and 3 µg plasmid/well, whereas the other three cell lines were transfected in triplicate wells using 2 µg plasmid/well and Lipofectamine Plus (Invitrogen, Denmark) according to the manufacturer's instructions. The next day, fresh growth medium was added to the wells, and cells were incubated for further 24 hours before collecting conditioned medium and harvesting cells. Sufficient transfection efficiency was ensured by evaluation of EGFP expression in wells transfected in parallel with the same vector containing the cDNA for EGFP.

*NTN Western blotting.* Cells were washed in PBS and lysed in 96°C hot sample buffer (2% SDS, 100 mM DTT, 60 mM Tris, pH 7.5, bromphenolblue). 5X concentrated samplebuffer was added to conditioned media. In some experiments, NTN was captured by GFRα2 from conditioned medium. This was done by incubating samples for 3 hours in ELISA plates that had been coated with goat anti-human Fc (Jackson ImmunoResearch, USA) in 50 mM Na2CO3 /NaHCO3, pH 9) overnight followed by blocking for 1 hour in 1% BSA in PBS and subsequently incubated with GFRα2-Ig fusion protein (R&D Systems, UK) in PBS with 0.1% HSA for 1 hour. After incubation with samples, wells were washed in PBST and 96°C sample buffer was added. Samples were boiled for 5 minutes and then electrophoresed on 8-18% gradient SDS gels, which were electroblotted to PVDF membranes. NTN was detected using polyclonal NTN antibody (#AF477, R&D Systems, UK) diluted 1:500 followed by HRP-linked anti-goat antibody. Bands were detected by chemoluminescence using the ECL+ system (Amersham Life Science).

*GFRa2*/*GFRa1 ELISA.* The GFRα2 ELISA detects binding of a Ret-Alkaline Phosphatase (Ret-AP) conjugate (Sanicola et al. 1997) to a complex of NTN bound to the GFRα2 receptor. Briefly, an Opti-plate plate (Packard Instruments, Perkin Elmer, Denmark) was coated with 100µl 1µg/ml Goat anti human Fc (Jackson Immunoresearch Laboratories, TriChem, Denmark) in 50mM NaHCO₃ (pH=9.6) for 16 h at 4°C. After wash in PBST, wells were blocked in 0.2% I-Block (Tropix, Roche, Denmark) in PBST for 1 hr at room temperature, followed by a brief wash in PBST. Samples from NTN-producing cells and standard dilutions of recombinant human NTN (R&D Systems, UK) in ARPE-19 growth medium were subsequently incubated in the wells with 1µg/ml GFRα2/Fc fusion protein (R&D Systems, UK) in conditioned medium from 293 EBNA cells expressing Ret-AP fusion protein (a kind gift from Biogen Idec, USA) for 1.5 h at room temperature. Wells were then washed first in PBST and then in AP-buffer (200 mM Tris (pH=9.8), 10 mM MgCl₂) followed by 30 min incubation with 10% Sapphire Enhancer (Tropix, Roche, Denmark) and 2% CSPD (Tropix, Roche, Denmark) in AP-buffer. Luminescence was quantified using Microbeta Trilux Counter (Perkin Elmer, Denmark). Binding activity to GFRα1 in conditioned medium was measured similarly, but with 1µg/ml GFRα1/Fc fusion protein (R&D Systems, UK) added instead of GFRα2/Fc. The relative GFRα2 binding activity in samples were calculated using a standard curve of recombinant NTN and with values from cells transfected with the wt construct set to 1.

*NTN ELISA.* Maxisorp plates (Nunc, Denmark) were coated by incubation with 1 µg/ml monoclonal anti-human NTN antibody (#MAB387, R&D Systems, UK) in coating solution (2.5 mM Na₂CO₃ / 2.5 mM NaHCO₃, pH 8.2) for 16 h at 4 °C. After wash in PBST (0.05% Tween-20 (Sigma-Aldrich, Denmark) in PBS), wells were blocked in blocking buffer (1% bovine serum albumin (Sigma-Aldrich, Denmark) and 5% sucrose in PBS) for 1 h at room temperature. After wash in PBST, wells were incubated with diluted media samples from NTN-producing cells for 3 h at room temperature. Recombinant NTN (#387-NE, R&D Systems,UK) was used as standard. 1 µg/ml polyclonal anti-human NTN antibody (#AF387, R&D Systems,UK) in blocking buffer was added to the wells and incubated for 16 h at 4°C. After wash in PBST, wells were incubated for 2 h at room temperature in 0.02% anti-goat-HRP (DAKO, Denmark) in blocking buffer supplemented with 1% normal mouse serum (DAKO, Denmark). Following wash in PBST, TMB substrate solution (Promega, Ramcon, Denmark) was added, and colour formation was stopped by addition of 1 N HCl after 15 min. A₄₅₀ was measured using an ELX-800 plate reader (Cambrex, Denmark).

*Expression of NTN in vitro.* Human NTN encodes a 197 amino acid (aa) prepro-protein with a 19 aa putative signal peptide, followed by a pro region of 76 aa. Pro-NTN is proteolytically cleaved at the sequence RXXR to generate the mature NTN of 102 aa. In order to characterise the influence of the pre-pro part on secretion of active NTN, we made different constructs (Fig 15A). As biologically active GDNF is readily secreted from various cell types after transfection, a NTN construct with the pre-pro part of GDNF was made (ppG-NTN). In addition, two constructs without the pro-part were established, one with wt NTN signal peptide (dpro-NTN) and one with the IgSP (IgSP-NTN). The DNA constructs were subcloned into the mammalian expression vector pNS1n and transiently transfected into HEK293 cells, CHO cells, the rat hippocampal cell line HiB5 or the human retinal epithelial cell line ARPE-19. Western blotting using an antibody against NTN was performed on cell lysates and conditioned medium. Figure 15B shows results from HEK293 cells. Similar results were obtained in the other three cell lines (data not shown). In cells transfected with wt NTN, a band corresponding in size to monomeric pro-NTN (∼22 kDa) was detected in cell lysate as well as conditioned medium. A smaller size band corresponding to NTN with the GDNF pro-region (∼19.6 kDa) was seen in cell lysate and conditioned medium from cells transfected with ppG-NTN. Thus, the pro-forms of NTN are expressed and secreted but not processed at a detectable level in the tested cell lines. In cells transfected with dpro-NTN or IgSP-NTN a band corresponding in size to mature monomeric NTN (∼12.5 kDa) was seen in lysates and conditioned medium. The level of NTN was apparently higher when using construct with the IgSP than the wt NTN SP.

We then tested the level of NTN in conditioned medium using a functional assay where binding of NTN to its receptor, GFRα2, is detected by formation of a ternary complex with the GFRα co-receptor Ret (Fig. 15C). Conditioned medium from cells transfected with wt NTN or ppG-NTN showed low levels of active NTN (7.5±0.6 ng/ml, 1.5±0.9 ng/ml, 38.6±3.3 ng/ml and 18.3 ± 1.7 ng/ml in HEK293, ARPE-19, HiB5 and CHO cells, respectively). However, GFRα2 binding activity in samples increased when using the dpro-NTN construct (90±19, 117±13, 7.5±1.7 and 4.1±0.9 fold higher NTN binding for HEK293, ARPE-19, HiB5 and CHO cells, respectively). In accordance with the Western blot results, NTN activity was further enhanced when using the IgSP-NTN construct (278±13, 771±50, 162±29 and 66±18 fold higher NTN binding for HEK293, ARPE-19, HiB5 and CHO cells, respectively). Similar results were obtained when performing the assay with the GFRα1 co-receptor (data not shown). Cell supernatants from cells transiently transfected with pNS1n-EGFP showed undetectable NTN activity confirming the specificity of the assays (data not shown). We also performed NTN Western blotting on samples where NTN from conditioned media had been bound to GFRα2-lg coated ELISA plates. When adding this binding step, no pro-forms of NTN were detected, whereas bands of the size of mature NTN were observed in samples from cells transfected with the IgSP-NTN construct and to a lesser extent when using the dpro-NTN vector (Fig 15D). In addition, a NTN sandwich ELISA on conditioned medium from cells transfected with the different NTN constructs was carried out. A monoclonal NTN antibody was used to capture NTN on ELISA plates and subsequently a polyclonal NTN antibody was used to detect the captured NTN. Both of the antibodies recognized pro-forms of NTN when used for Western blotting, but as shown in fig 15E, the NTN pro-forms were not detected in the NTN ELISA. This finding suggests that the pro-part of NTN prevent binding of the antibody to the native folded NTN, but not to denatured NTN. The NTN sandwich ELISA confirmed that exchanging the wt NTN SP with the IgSP enhances the level of NTN in conditioned medium (2-8 fold, depending on cell line).

### Example 5: In vivo gene therapy in a Parkinson's Disease model.

*Surgical procedures.* A total of 24 young adult female Sprague-Dawley rats (Møllegaarden, Denmark) were used and housed under 12 h light:dark cycle with free access to rat chow and water. Virus injections and 6-OHDA lesions were performed according to Rosenblad et al (2000) with slight modifications. Briefly, under isoflourane anesthesia (1.5-2%) animals were injected with rLV vector (3x10⁵ TU/animal) carrying the cDNA for GFP, hNTN, IgSP-hNTN or GDNF *(n* = 5-7/group). Four deposits (0.75 µl/deposit) were made into the striatum along two needle tracts at the following coordinates: AP= 1.0 mm, ML= -2.6 mm, DV₁ = -5.0 mm DV₂ = - 4.5 mm, and AP =0.0 mm, ML = -3.7 mm, DV₁ = -5.0 mm, DV₂ = -4.5 mm. The tooth bar was set at -2.3 mm. 14 days after rLV injections the animals were reanestetized and with a 10-µl Hamilton syringe a single deposit of 20 µg 6-OHDA (Sigma; calculated as free base and dissolved in 3 µl ice-cold saline supplemented with 0.02% ascorbic acid) was injected into the right striatum at the following coordinates: AP= 0.5 mm; ML= -3.4 mm DV= -5.0 mm relative to the dura and tooth bar set to 0.0 mm. The injection rate was 1 µl/min and the glass pipette was left in place for additional 3 min before withdrawal.

*Amphetamine-induced rotation.* At 10 days after rLV injections and again 4 weeks after the 6-OHDA injections, rats were injected with amphetamine (2.5 mg/kg, Mecobenzon, DK) and monitored for turning response in automated rotometer bowls over 90 min. Rotational asymmetry scores are expressed as net 90º turns per min and ipsilateral rotations (i.e. towards the injected site) were assigned as positive value.

*Histology.* At 28 days after the 6-OHDA injection the animals were deeply anesthetized with sodiumpentobarbital and transcardially perfused with saline for one min followed by 200 ml ice-cold 4% PFA in 0.1 M phosphate buffer (pH 7.4). The brains were dissected and postfixed in the same fixative for 3-4 h and then transferred into 25% sucrose/0.1 M phosphate buffer for 48 h. Six series of 40-µm sections were cut on a freezing microtome. Immunohistochemistry was performed as described previously (Rosenblad et al., 2003). In brief, sections were incubated with goat-anti-hNTN or goat-anti-hGDNF primary antibodies diluted 1:2000 (R&D Systems, UK), chicken anti-GFP, mouse anti-TH or rabbit anti-VMAT antibodies diluted 1:2000 (Chemicon) in phosphate buffered saline containing 2% normal horse or swine serum and 0.25% Triton X-100 followed by incubation with the appropriate biotinylated secondary antibody (Jackson Immunoresearch, USA) for two hours, and avidin-biotin complex (ABC) kit according to manufacturers instructions (Vector Laboratories, USA). Finally, the colour reaction was developed using 3'3'-diaminobenzidine as chromogen.

### Morphometric analysis. Quantification of the number of TH or VMAT immunoreactive cells in

*SN.* A blinded observer assessed the number of immunoreactive neurons in SN pars compacta, as described previously (Sauer and Oertel, 1994). In brief, three consecutive sections centered on the level of the medial terminal nucleus of the accessory optic tract (MTN, -5.3 in the atlas of Paxinos and Watson, 1997) were used and all stained neurons lateral to the MTN were counted at 40x magnification. Cell numbers are expressed as the mean ± S.E.M. of the percentage of the number in the intact side.

*Striatal fiber density measurements.* Striatal DA innervation was assessed by measuring optical density (OD) of the striatum at three rostrocaudal levels in sections stained for TH. Using an Olympus DP50 digital camera and a constant illumination table, digitalized images were collected . OD on the intact and lesioned side was measured using Scanlmage v. 4.02 software. Corpus callosum in each section was used as reference for background staining.

*In vivo neuroprotection in the rat 6-OHDA lesion model.* Next, we wanted to investigate to what extent IgSP-NTN construct could also give sustained secretion of active NTN *in vivo.* We therefore generated recombinant lentiviral vectors encoding the wt pre-pro NTN (rLV-wtNTN) and the IgSP-NTN (rLV-IgSPNTN), and tested if they were able to provide neuroprotection in an animal model of PD where NTN protein injections previously have been shown to prevent loss of DA neurons (Horger et al, 1998; Rosenblad et al, 1999). Vectors encoding green flourescent protein (GFP; rLV-GFP) or GDNF (rLV-GDNF) were used as negative and positive control vectors, respectively.

### Immunohistochemical localization of transgene expression

Inspection of sections from rLV-GFP treated animals showed a column of transduced cells, approximately 2 x 0.5 mm, in the central head of the caudate putamen (Fig. 16A). The majority of transduced cells had morphologies of striatal medium sized spiny projection neurons. To a lesser extent cells with astroglial morphology were seen in the striatum, and oligodendroglia in the overlying corpus callosum. The GFP expressing cells showed a distinct intracellular expression pattern. In contrast sections through the striatum and substantia nigra from animals treated with rLV-GDNF and processed for GDNF-immunohistochemistry showed a diffuse staining in the striatum, consistent with secretion of GDNF from transduced cells (Fig 16C). In animals receiving rLV-wtNTN injections, NTN-immunohistochemistry showed no extracellular immunoreactivity in the vicinity of rLV-wtNTN transduced striatal cells (Fig. 16B). At higher magnification a few NTN-immunoreactive cells with punctate cytoplasmic staining were observed along the injection tract (Fig. 16E). In contrast, animals receiving injections of rLV-IgSP-NTN had a prominent diffuse staining (extracellular) in the striatum (Fig. 16D), similar to that seen in GDNF treated animals and consistent with secretion. NTN immunoreactive cellular profiles were also observed in rLV-IgSP-NTN injected animals, but most of the immunoreactive material was located extracellularly (Fig. 16F). In animals receiving rLV-GDNF as well as rLV-IgSPNTN there was prominent labeling with the respective antibodies in the substantia nigra pars reticulata (data not shown and Fig. 16G). The dense network of immunoreactive fibers apparently originated from the striatonigral projections as they could be followed rostrally to the striatum. This result suggest that NTN can be anterogradely transported within the nigrostriatal pathway from the striatum, as has already been described for GDNF (Rosenblad et al. 1999).

At 4 weeks after the intrastriatal 6-OHDA lesion, the number of remaining dopaminergic nigral neurons were assessed by counting neurons expressing TH (Fig. 17A and Fig. 18). In control rLV-GFP treated animals clearly fewer TH-immunoreactive (IR) neurons were seen in the substantia nigra on the lesion side (23±3.4%), as compared to the intact contralateral side (Fig 17A and 18D). Similarly, animals receiving rLV-NTN (Fig. 17A and 18B) showed a marked lesion induced reduction in TH-IR neurons (30±7.7% remaining). In contrast, the rLV-IgSPNTN treated group had a significantly higher percentage of TH-IR neurons on the lesion side (91±1.2%; p<0.01) (Fig. 17A and 18C), which was indistinguishable from that observed in the group receiving GDNF treatment (86±3.2%; p<0.01) (Fig. 17A and 18H).

To confirm that the differences in the number of TH-IR nigral neurons were not due to regulation of TH enzyme we quantified in adjacent sections the number of VMAT-IR neurons, which is shown to be less prone to regulation by these neurotrophic factors (Rosenblad et al, 2003; Georgievska et al, 2002; Kirik et al, 2001). As shown in figure 17B, transduction with rLV-IgSPNTN or rLVGDNF significantly preserved the number of VMAT-IR neurons in substantia nigra on the lesion side (75.2±6.8% and 59.9±4.2% of that in the intact side, respectively) compared to rLV-GFP or rLV-NTN treated animals (15.5±2.4% and 24.9±6.4%, respectively), corroborating the results seen with TH-staining.

In addition to the protection of TH-IR and VMAT-IR neurons in the substantia nigra, it was noticeable that in specimens from rLV-IgSPNTN treated animals, the TH staining intensity was reduced in many remaining substantia nigra neurons (Fig. 18G) as compared to TH-IR neurons on the intact side (Fig. 18E), or lesioned side of rLV-GFP or rLV-wtNTN treated animals (Fig. 18F). Reduced TH staining intensity was also seen following treatment with GDNF, consistent with previous reports (Georgievska et al, 2002), and an observer familiar with the signs of this phenomenon but blinded to the specimens was unable to distinguish the "GDNF-like" reduction in IgSPNTN treated animals from that seen following GDNF administration.

Inspection of sections through the striatum processed for TH-immunohitochemistry showed that in all groups the central and lateral caudate-putamen was devoid of TH-IR fibers on the 6-OHDA injected side. Densitometric quantification of TH-IR innervation on the lesioned side showed that 15-25% remained at 4 weeks. This is in agreement with earlier studies in the intrastriatal 6-OHDA lesion model (Rosenblad et al, 1999; Georgievska et al, 2002) showing that recovery of TH-IR striatal innervation takes longer than four weeks post lesion to develop. Consistently, amphetamine-induced rotation, which can be used as a sensitive measure of dopamine denervation in the striatum (Kirik et al, 1998), showed no significant difference in the number of ipsilateral turns between any of the treatment groups at 4 weeks post-lesion (ranging from 4.5±1.5 to 13.4±3.3 net ipsilateral turns/minute; p>0.05 two-way repeated measures ANOVA). Amphetamine-induced turning assessed at 10 days after viral transduction but before the 6-OHDA lesion showed a slight but non-significant contralateral turning bias in the IgSP-NTN (3.3±1.5) and GDNF group (1.9±1.3), compared to the rLV-GFP or rLV-NTN groups (0.1±0.9 and -0.1±1.6, respectively), consistent with an upregulation of the DA function on the IgSP-NTN transduced side similar to what has been reported previously to occur after GDNF treatment (Georgievska et al, 2002; Georgievska et al, 2003).

Taken together our results indicate that secretion of NTN from a lentiviral vector can be significantly enhanced by removal of the pro-region and substitution of the wild type signal peptide with a heterologous one. The enhanced secretion of active NTN was seen also *in vivo* in transduced striatal cells and enabled for the first time efficient neuroprotection of lesioned nigral dopamine neurons *in vivo* using a lentiviral delivery approach, similar to what has previously been reported for GDNF.

### Reference List (Examples 3-5)

DUNN, K. C., A. E. AOTAKI-KEEN, F. R. PUTKEY, and L. M. HJELMELAND. 1996. ARPE-19, a human retinal pigment epithelial cell line with differentiated properties. Exp.Eye Res. 62: 155-169.
GEORGIEVSKA, B., D. KIRIK, and A. BJORKLUND. 2002. Aberrant sprouting and downregulation of tyrosine hydroxylase in lesioned nigrostriatal dopamine neurons induced by long-lasting overexpression of glial cell line derived neurotrophic factor in the striatum by lentiviral gene transfer. Exp.Neurol. 177: 461-474.
GEORGIEVSKA, B., D. KIRIK, and A. BJORKLUND. 2004. Overexpression of glial cell line-derived neurotrophic factor using a lentiviral vector induces time- and dose-dependent downregulation of tyrosine hydroxylase in the intact nigrostriatal dopamine system. J.Neurosci. 24: 6437-6445.
HORGER, B. A., M. C. NISHIMURA, M. P. ARMANINI, L. C. WANG, K. T. POULSEN, C. ROSENBLAD, D. KIRIK, B. MOFFAT, L. SIMMONS, E. JOHNSON, JR., J. MILBRANDT, A. ROSENTHAL, A. BJORKLUND, R. A. VANDLEN, M. A. HYNES, and H. S. PHILLIPS. 1998. Neurturin exerts potent actions on survival and function of midbrain dopaminergic neurons. J.Neurosci. 18: 4929-4937.
KIRIK, D., B. GEORGIEVSKA, C. ROSENBLAD, and A. BJORKLUND. 2001. Delayed infusion of GDNF promotes recovery of motor function in the partial lesion model of Parkinson's disease. Eur.J.Neurosci. 13: 1589-1599.
KIRIK, D., C. ROSENBLAD, and A. BJORKLUND. 1998. Characterization of behavioral and neurodegenerative changes following partial lesions of the nigrostriatal dopamine system induced by intrastriatal 6-hydroxydopamine in the rat. Exp.Neurol. 152: 259-277.
RENFRANZ, P. J., M. G. CUNNINGHAM, and R. D. MCKAY. 1991. Region-specific differentiation of the hippocampal stem cell line HiB5 upon implantation into the developing mammalian brain. Cell 66: 713-729.
ROSENBLAD, C., B. GEORGIEVSKA, and D. KIRIK. 2003. Long-term striatal overexpression of GDNF selectively downregulates tyrosine hydroxylase in the intact nigrostriatal dopamine system. Eur.J.Neurosci. 17: 260-270.
ROSENBLAD, C., M. GRONBORG, C. HANSEN, N. BLOM, M. MEYER, J. JOHANSEN, L. DAGO, D. KIRIK, U. A. PATEL, C. LUNDBERG, D. TRONO, A. BJORKLUND, and T. E. JOHANSEN. 2000. In vivo protection of nigral dopamine neurons by lentiviral gene transfer of the novel GDNF-family member neublastin/artemin. Mol.Cell Neurosci. 15: 199-214.
ROSENBLAD, C., D. KIRIK, B. DEVAUX, B. MOFFAT, H. S. PHILLIPS, and A. BJORK-LUND. 1999. Protection and regeneration of nigral dopaminergic neurons by neurturin or GDNF in a partial lesion model of Parkinson's disease after administration into the striatum or the lateral ventricle. Eur.J.Neurosci. 11: 1554-1566.
SANICOLA, M., C. HESSION, D. WORLEY, P. CARMILLO, C. EHRENFELS, L. WALUS, S. ROBINSON, G. JAWORSKI, H. WEI, R. TIZARD, A. WHITTY, R. B. PEPINSKY, and R. L. CATE. 1997. Glial cell line-derived neurotrophic factor-dependent RET activation can be mediated by two different cell-surface accessory proteins. Proc.Natl.Acad.Sci.U.S.A 94: 6238-6243.
SAUER, H. and W. H. OERTEL. 1994. Progressive degeneration of nigrostriatal dopamine neu-rons following intrastriatal terminal lesions with 6-hydroxydopamine: a combined retrograde tracing and immunocytochemical study in the rat. Neuroscience 59: 401-415.
ZUFFEREY, R., J. E. DONELLO, D. TRONO, and T. J. HOPE. 1999. Woodchuck hepatitis virus posttranscriptional regulatory element enhances expression of transgenes delivered by ret-roviral vectors. J.Virol. 73: 2886-2892.
ZUFFEREY, R., T. DULL, R. J. MANDEL, A. BUKOVSKY, D. QUIROZ, L. NALDINI, and D. TRONO. 1998. Self-inactivating lentivirus vector for safe and efficient in vivo gene delivery. J.Virol. 72: 9873-9880.
ZUFFEREY, R., D. NAGY, R. J. MANDEL, L. NALDINI, and D. TRONO. 1997. Multiply attenuated lentiviral vector achieves efficient gene delivery in vivo. Nat.Biotechnol. 15: 871-875.

### Example 6. Signal peptide processing predictions using SignalP 3.0

Predictions of positions for signal peptide cleavage using SignalP version 3.0. The predictions were carried out using deltaproNTN, IgSP-NTN and NTN with signal peptides from various growth factors.

Chimeric molecules with NTN signal peptide (deltaproNTNs).
>hNTN signal (SEQ ID No 37)
MQRWKAAALA SVLCSSVLS 
>DELTAPRO102NTN (SEQ ID No 16)
MQRWKAAALA SVLCSSVLSA RLGARPCGLR ELEVRVSELG LGYASDETVL FRYCAGACEA
AARVYDLGLR RLRQRRRLRR ERVRAQPCCR PTAYEDEVSF LDAHSRYHTV HELSARECAC
V
 
>DELTAPRO101NTN (SEQ ID No 25)
MQRWKAAALA SVLCSSVLSR LGARPCGLRE LEVRVSELGL GYASDETVLF RYCAGACEAA
ARVYDLGLRR LRQRRRLRRE RVRAQPCCRP TAYEDEVSFL DAHSRYHTVH ELSARECACV
 
>DELTAPRO100NTN (SEQ ID No 26)
MQRWKAAALA SVLCSSVLSL GARPCGLREL EVRVSELGLG YASDETVLFR YCAGACEAAA
RVYDLGLRRL RQRRRLRRER VRAQPCCRPT AYEDEVSFLD AHSRYHTVHE LSARECACV
 
>DELTAPRO99NTN (SEQ ID No 27)
MQRWKAAALA SVLCSSVLSG ARPCGLRELE VRVSELGLGY ASDETVLFRY CAGACEAAAR VYDLGLRRLR
QRRRLRRERV RAQPCCRPTA YEDEVSFLDA HSRYHTVHEL SARECACV
 
>DELTAPRO98NTN (SEQ ID No 28)
MQRWKAAALA SVLCSSVLSA RPCGLRELEV RVSELGLGYA SDETVLFRYC AGACEAAARV YDLGLRRLRQ
RRRLRRERVR AQPCCRPTAY EDEVSFLDAH SRYHTVHELS ARECACV
 
>DELTAPRO97NTN (SEQ ID No 29)
MQRWKAAALA SVLCSSVLSR PCGLRELEVR VSELGLGYAS DETVLFRYCA GACEAAARVY DLGLRRLRQR
RRLRRERVRA QPCCRPTAYE DEVSFLDAHS RYHTVHELSA RECACV
 
>DELTAPRO96NTN (SEQ ID No 30)
MQRWKAAALA SVLCSSVLSP CGLRELEVRV SELGLGYASD ETVLFRYCAG ACEAAARVYD
LGLRRLRQRR RLRRERVRAQ PCCRPTAYED EVSFLDAHSR YHTVHELSAR ECACV
 Chimeric molecules with Immunoglobulin Signal Peptide (IgSP-deltaproNTN):
>IgSP, mouse (SEQ ID No 4)
MKCSWVIFFL MAVVTGVNS 
>IgSP-102NTN (SEQ ID No 18)
MKCSWVIFFL MAVVTGVNSA RLGARPCGLR ELEVRVSELG LGYASDETVL FRYCAGACEA
AARVYDLGLR RLRQRRRLRR ERVRAQPCCR PTAYEDEVSF LDAHSRYHTV HELSARECAC
V
 
>IgSP-101NTN (SEQ ID No 19)
MKCSWVIFFL MAVVTGVNSR LGARPCGLRE LEVRVSELGL GYASDETVLF RYCAGACEAA ARVYDLGLRR
LRQRRRLRRE RVRAQPCCRP TAYEDEVSFL DAHSRYHTVH ELSARECACV
 
>IgSP-100NTN (SEQ ID No 20)
MKCSWVIFFL MAVVTGVNSL GARPCGLREL EVRVSELGLG YASDETVLFR YCAGACEAAA RVYDLGLRRL
RQRRRLRRER VRAQPCCRPT AYEDEVSFLD AHSRYHTVHE LSARECACV
 
>IgSP-99NTN (SEQ ID No 21)
MKCSWVIFFL MAVVTGVNSG ARPCGLRELE VRVSELGLGY ASDETVLFRY CAGACEAAAR VYDLGLRRLR
QRRRLRRERV RAQPCCRPTA YEDEVSFLDA HSRYHTVHEL SARECACV
 
>IgSP-98NTN (SEQ ID No 22)
MKCSWVIFFL MAVVTGVNSA RPCGLRELEV RVSELGLGYA SDETVLFRYC AGACEAAARV YDLGLRRLRQ
5 RRRLRRERVR AQPCCRPTAY EDEVSFLDAH SRYHTVHELS ARECACV
 
>IgSP-97NTN (SEQ ID No 23)
MKCSWVIFFL MAVVTGVNSR PCGLRELEVR VSELGLGYAS DETVLFRYCA GACEAAARVY DLGLRRLRQR
RRLRRERVRA QPCCRPTAYE DEVSFLDAHS RYHTVHELSA RECACV
 
>IgSP-96NTN (SEQ ID No 24)
MKCSWVIFFL MAVVTGVNSP CGLRELEVRV SELGLGYASD ETVLFRYCAG ACEAAARVYD LGLRRLRQRR
RLRRERVRAQ PCCRPTAYED EVSFLDAHSR YHTVHELSAR ECACV
 Chimeric growth factor-NTN molecules (growth factor SP-deltaproNTN)

>hNGF-102NTN (SEQ ID No. 31)
MSMLFYTLIT AFLIGIQAAR LGARPCGLRE LEVRVSELGL GYASDETVLF RYCAGACEAA ARVYDLGLRR
LRQRRRLRRE RVRAQPCCRP TAYEDEVSFL DAHSRYHTVH ELSARECACV

>mNGF-102NTN (SEQ ID No. 32)
MSMLFYTLIT AFLIGVQAAR LGARPCGLRE LEVRVSELGL GYASDETVLF RYCAGACEAA ARVYDLGLRR
LRQRRRLRRE RVRAQPCCRP TAYEDEVSFL DAHSRYHTVH ELSARECACV

>hGDNF-102NTN (SEQ ID No. 33)
MKLWDVVAVC LVLLHTASAA RLGARPCGLR ELEVRVSELG LGYASDETVL FRYCAGACEA
AARVYDLGLR RLRQRRRLRR ERVRAQPCCR PTAYEDEVSF LDAHSRYHTV HELSARECAC
V

>mGDNF-102NTN (SEQ ID No. 34)
MKLWDVVAVC LVLLHTASAA RLGARPCGLR ELEVRVSELG LGYASDETVL FRYCAGACEA
AARVYDLGLR RLRQRRRLRR ERVRAQPCCR PTAYEDEVSF LDAHSRYHTV HELSARECAC
V

>hNBN-102NTN (SEQ ID No. 35)
MELGLGGLST LSHCPWPRRQ PALWPTLAAL ALLSSVAEAA RLGARPCGLR ELEVRVSELG LGYASDETVL
FRYCAGACEA AARVYDLGLR RLRQRRRLRR ERVRAQPCCR PTAYEDEVSF LDAHSRYHTV HELSARECAC
V

>hPSP-102NTN (SEQ ID No. 36)
MAVGKFLLGS LLLLSLQLGQ GARLGARPCG LRELEVRVSE LGLGYASDET VLFRYCAGAC
EAAARVYDLG LRRLRQRRRL RRERVRAQPC CRPTAYEDEV SFLDAHSRYH TVHELSAREC
ACV

**Signal peptide predictions for proteins with NTN signal peptide¹ (deltapro)**

| **Protein** | **SignalP 3.0 - NN** | | | | **SignalP 3.0 HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| preproNTN | 0.835 | 0.643 | 0.442 | 19/20 | 0.997 | 0.861 | 19/20 | |
| deltapro102NTN | 0.867 | 0.738 | 0.570 | 19/20 | 1.000 | 0.397* | 24/25 | Cleavage at 19/20 predicted with almost identical probability in HMM |
| deltapro101NTN | 0.772 | 0.730 | 0.726 | 23/24 | 1.000 | 0.812 | 23/24 | |
| deltapro100NTN | 0.952 | 0.725 | 0.486 | 22/23 | 0.999 | 0.449* | 21/22 | Cleavage at 22/23 predicted with almost identical probability with HMM |
| deltapro99NTN | 0.798 | 0.659 | 0.411 | 21/22 | 1.000 | 0.518 | 21/22 | |
| deltapro98NTN | 0.835 | 0.737 | 0.584 | 19/20 | 1.000 | 0.504 | 20/21 | Cleavage at 19/20 predicted with almost identical probability with HMM |
| deltapro97NTN | 0.768 | 0.713 | 0.694 | 19/20 | 1.000 | 0.978 | 19/20 | |
| deltapro96NTN | 0.715 | 0.555 | 0.272* | 23/24 | 0.997 | 0.581 | 23/24 | Cleavage predicted after first canonical cysteine. |
| Cutoff values | 0.48 | 0.43 | 0.32 | | | 0.5 | | |

| | | | | | | | | |
|---|---|---|---|---|---|---|---|---|
| ¹MQRWKAAALASVLCSSVLS 19 amino acids (SEQ ID No 37) * Below cutoff values | | | | | | | | |

**Signal peptide predictions for proteins with Immunoglobulin signal peptide¹ (IgSP-NTN)**

| **Protein** | **SignalP 3.0 - NN** | | | | **SignalP 3.0 HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| IgSP-102NTN | 0.946 | 0.911 | 0.964 | 19/20 | 1.000 | 0.911 | 19/20 | |
| IgSP-101NTN | 0.929 | 0.846 | 0.752 | 19/20 | 0.998 | 0.488* | 19/20 | |
| IgSP-100NTN | 0.945 | 0.874 | 0.826 | 19/20 | 0.999 | 0.481* | 22/23 | Cleavage predicted with lower probability at 19/20 with HMM |
| IgSP-99NTN | 0.932 | 0.858 | 0.755 | 19/20 | 0.999 | 0.537 | 19/20 | |
| IgSP-98NTN | 0.933 | 0.916 | 0.977 | 19/20 | 1.000 | 0.843 | 19/20 | |
| IgSP-97NTN | 0.898 | 0.890 | 0.988 | 19/20 | 1.000 | 0.988 | 19/20 | |
| IgSP-96NTN | 0.906 | 0.761 | 0.516 | 19/20 | 0.996 | 0.582 | 16/17 | Cleavage predicted with lower probability at 19/20 with HMM |
| Cutoff values | 0.48 | 0.43 | 0.32 | | | 0.5 | | |

| | | | | | | | | |
|---|---|---|---|---|---|---|---|---|
| ¹MKCSWVIFFLMAVVTGVNS 19 amino acids (SEQ ID No 4) * Below cutoff values | | | | | | | | |

**Signal peptide predictions for proteins with growth factor signal peptides**

| **Protein** | **SignalP 3.0 - NN** | | | | **SignalP 3.0 HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| hNGF-102NTN' | 0.937 | 0.857 | 0.849 | 18/19 | 0.996 | 0.563 | 19/20 | |
| mNGF-102NTN² | 0.944 | 0.895 | 0.964 | 18/19 | 0.999 | 0.818 | 18/19 | |
| hGDNF-102NTN³ | 0.947 | 0.907 | 0.988 | 19/20 | 1.000 | 0.918 | 19/20 | |
| mGDNF-102NTN⁴ | 0.947 | 0.907 | 0.988 | 19/20 | 1.000 | 0.918 | 19/20 | |
| mGDNF-102NTN⁵ | 0.156* | 0.121* | 0.123* | NO | 0.014 | 0.010* | 16/17 | "signal peptide" corresponding to wrongly predicted start codon in mouse GDNF (Genbank # NM_010275) |
| hNBN-102NTN⁶ | 0.514 | 0.681 | 0.852 | 39/40 | 0.998 | 0.879 | 39/40 | |
| hPSP-102NTN⁷ | 0.955 | 0.874 | 0.801 | 21/22 | 1.000 | 0.869 | 21/22 | |
| Cutoff values | 0.48 | 0.43 | 0.32 | | | 0.5 | | |

| | | | | | | | | |
|---|---|---|---|---|---|---|---|---|
| hNGF¹ SP msmlfytlitafligiqa 18 amino acids (SEQ ID No 40) mNGF² SP msmlfytlitafligvqa 18 amino acids (SEQ ID No 41) hGDNF³ SP mklwdvvavclvllhtasa 19 amino acids (SEQ ID No 42) mGDNF⁴ SP mklwdvvavclvllhtasa 19 amino acids (SEQ ID No 43) mGONF⁵ "N-terminal" mgfgplgvnvqlgvygdri 19 amino acids (SEQ ID No 44) hNBN⁶ SP melglgglstlshcpwprrqpalwptlaalallssvaea 39 amino acids (SEQ ID No 45) hPSP⁷ SP mavgkfllgsllllslqlgqg 21 amino acids (SEQ ID No 46) * Below cutoff values | | | | | | | | |

### SEQUENCE LISTING

| SEQ ID NO | TYPE | DESCRIPTION |
|---|---|---|
| 1 | P | IgSP human |
| 2 | P | IgSP Monkey |
| 3 | P | IgSP Marmoset |
| 4 | P | IgSP Mouse |
| 5 | P | IgSP Pig |
| 6 | P | IgSP Rat |
| 7 | N | mature NTN, human |
| 8 | P | mature NTN, human |
| 9 | P | 96NTN, human |
| 10 | P | mature NTN, mouse |
| 11 | P | mature NTN, rat |
| 12 | P | preproNTN, human |
| 13 | P | preproNTN, mouse |
| 14 | P | preproNTN, rat |
| 15 | N | deltapro102NTN, human |
| 16 | P | deltapro102NTN, human |
| 17 | N | IgSP-102NTN, chimeric |
| 18 | P | IgSP-102NTN, chimeric |
| 19 | P | IgSP-101NTN, chimeric |
| 20 | P | IgSP-100NTN, chimeric |
| 21 | P | IgSP-99NTN, chimeric |
| 22 | P | IgSP-98NTN, chimeric |
| 23 | P | IgSP-97NTN, chimeric |
| 24 | P | IgSP-96NTN, chimeric |
| 25 | P | deltapro101NTN, human |
| 26 | P | deltapro100NTN, human |
| 27 | P | deltapro99NTN, human |
| 28 | P | deltapro98NTN, human |
| 29 | P | deltapro97NTN, human |
| 30 | P | deltapro96NTN, human |
| 31 | P | hNGF-102NTN |
| 32 | P | mNGF-102NTN |
| 33 | P | hGDNF-102NTN |
| 34 | P | mGDNF-102NTN |
| 35 | P | hNBN-102NTN |
| 36 | P | hPSP-102NTN |
| 37 | P | hNTN signal peptide |
| 38 | P | mNTN, signal peptide |
| 39 | P | rNTN, signal peptide |
| 40 | P | hNGF, signal peptide |
| 41 | P | mNGF, signal peptide |
| 42 | P | hGDNF, signal peptide |
| 43 | P | mGDNF, signal peptide |
| 44 | P | mGDNF, putative signal peptide |
| 45 | P | hNBN, signal peptide |
| 46 | P | hPSP, signal peptide |
| 47 | P | preproNBN, human |
| 48 | P | preproPSP, human |
| 49 | P | preproGDNF, human |
| 50 | N | preproGDNF-102NTN, human |
| 51 | P | preproGDNF-102NTN, human |
| 52-62 | N | PCR-primers |

### EMBODIMENTS

In the following the invention is described as numbered embodiments starting with the arbitrary number 1.
1. A method for treatment of Parkinson's Disease, said method comprising administering to the central nervous system of an individual in need thereof a therapeutically effective amount of a viral expression vector, said vector comprising a promoter sequence capable of directing the expression of an operably linked polypeptide, said polypeptide comprising a signal peptide capable of functioning in a mammalian cell, and a human, murine or rat Neurturin (NTN) selected from the group consisting of pro-NTN, mature NTN, N-terminally truncated mature NTN, and a sequence variant of any such NTN.
2. The method of embodiment 1, wherein NTN is selected from the group consisting of mature NTN (SEQ ID No 8, 10, and 11), N-terminally truncated mature NTN, and a sequence variant of mature or N-terminally truncated NTN.
3. The method of embodiment 1, wherein the sequence variant comprises a sequence having at least 89% sequence identity to the amino acid sequence of SEQ ID No. 9, preferably at least 90%, more preferably at least 95%.
4. The method of embodiment 1, wherein NTN is human or murine NTN.
5. The method of embodiment 1, wherein NTN is human NTN.
6. The method of embodiment 1, wherein NTN is human mature NTN (SEQ ID No 8).
7. The method of embodiment 1, wherein the individual is a human being.
8. The method of embodiment 7, wherein the administered virus composition has at least 10⁸ t.u/mL, preferably from 10⁸ to 10¹⁰ t.u./mL, more preferably at least 10⁹ t.u./mL.
9. The method of embodiment 7, wherein the amount of composition administered is from 1-10 µL per injection site.
10. The method of embodiment 7, wherein the administered virus composition has at least 10¹⁰ t.u/mL to 10¹⁵ t.u./mL.
11. The method of embodiment 1, wherein the virus vector is a lentivirus.
12. The method of embodiment 1, wherein the virus vector is an adeno-associated virus.
13. The method of embodiment 1, wherein the virus is administered to the striatum.
14. The method of embodiment 1, wherein the virus is administered to the substantia nigra.
15. Use of a viral expression vector, said vector comprising a promoter sequence capable of directing the expression of an operably linked polypeptide, said polypeptide comprising a signal peptide capable of functioning in a mammalian cell, and a human, murine or rat Neurturin (NTN) selected from the group consisting of pro-NTN, mature NTN, N-terminally truncated mature NTN, and a sequence variant of any such NTN; for the preparation of a medicament for the treatment of Parkinson's Disease.
16. The use of embodiment 15, wherein NTN is selected from the group consisting of mature NTN (SEQ ID No. 8, 10, and 11), N-terminally truncated mature NTN, and a sequence variant of mature or N-terminally truncated NTN.
17. The use of embodiment 15, wherein NTN is human or murine NTN.
18. The use of embodiment 15, wherein NTN is human NTN.
19. The use of embodiment 15, wherein NTN is human mature NTN (SEQ ID No 8).
20. The use of embodiment 15, wherein the administered virus composition has at least 10⁸ t.u/mL, preferably from 10⁸ to 10¹⁰ t.u./mL, more preferably at least 10⁹ t.u./mL.
21. The use of embodiment 20, wherein the virus vector is a lentivirus.
22. The use of embodiment 15, wherein the administered virus composition has at least 10¹⁰ t.u/mL to 10¹⁵ t.u./mL.
23. The use of embodiment 22, wherein the virus vector is an adeno-associated virus.
24. A viral expression vector comprising a polynucleotide sequence comprising a promoter sequence capable of directing the expression of an operably linked polypeptide, said polypeptide comprising a signal peptide capable of functioning in a mammalian cell, and a Neurturin selected from the group consisting of mature NTN, N-terminally truncated mature NTN, and a sequence variant of mature or N-terminally truncated NTN.
25. The vector of embodiment 24, wherein the polynucleotide sequence does not encode a functional Neurturin pro-region.
26. The vector of embodiment 24, wherein the signal peptide is NGF signal peptide.
27. The vector of embodiment 24, wherein the signal peptide is GDNF signal peptide.
28. The vector of embodiment 24, wherein the signal peptide is Persephin signal peptide.
29. The vector of embodiment 24, wherein the signal peptide is Neublastin signal peptide.
30. The vector of embodiment 24, wherein the signal peptide is selected from the group consisting of: human NGF signal peptide (SEQ ID No 40), murine NGF signal peptide (SEQ ID No. 41), human GDNF signal peptide (SEQ ID No. 42), murine GDNF signal peptide (SEQ ID No. 43).
31. The vector of embodiment 24, wherein the signal peptide is a immunoglobulin G signal peptide (IgSP).
32. The vector according to embodiment 30, wherein the IgSP is selected from the group consisting of murine IgSP (SEQ ID NO 4), rat IgSP (SEQ ID NO 6), porcine IgSP (SEQ ID NO 5), monkey IgSP (SEQ ID NO 2 or 3), human IgSP (SEQ ID NO 1).
33. The vector of embodiment 32, wherein the IgSP is murine IgSP (SEQ ID No 4).
34. The vector of embodiment 32, wherein the IgSP is human IgSP (SEQ ID No 1).
35. The vector according to embodiment 24, the vector being selected from the group consisting of HIV, SIV, FIV, EIAV, AAV, adenovirus, retrovirus, herpes virus.
36. The vector according to embodiment 24, wherein the vector is a replication-defective lentivirus particle.
37. The vector according to embodiment 24, wherein said vector particle being produced from a lentiviral vector comprising a 5' lentiviral LTR, a tRNA binding site, a packaging signal, a promoter operably linked to a polynucleotide signal encoding said fusion protein, an origin of second strand DNA synthesis and a 3' lentiviral LTR.
38. The vector according to any of the preceding embodiments 24 to 37, wherein the promoter capable of directing the expression of the fusion protein is selected from the group consisting of: ubiquitin promoter, CMV promoter, JeT promoter, SV40 promoter, Elongation Factor 1 alpha promoter (EF1-alpha).
39. The vector according to any of the preceding embodiments 24 to 38, wherein the promoter is an inducible/repressible promoter, such as: Tet-On, Tet-Off, Rapamycin-inducible promoter, Mx1.
40. The vector according to any of the preceding embodiments 24 to 39, further comprising at least one expression enhancing sequence, such as Woodchuck hepatitis virus post-transcriptional regulation element, WPRE, SP163, rat Insulinll-intron or other introns, CMV enhancer, and Chicken [beta]-globin insulator or other insulators.
41. The vector according to any of preceding embodiments 24 to 40, further comprising a sequence coding for the Cre-recombinase protein, and LoxP sequences.
42. A pharmaceutical composition comprising the vector according to any of the preceding embodiments 24 to 41 and one or more of pharmaceutically acceptable adjuvants, excipients, carriers and/or diluents.
43. An isolated host cell transduced with the vector of any of the preceding embodiments 24 to 41.
44. The host cell of embodiment 43, being a mammalian host cell.
45. The host cell of embodiment 44, wherein said mammal is selected from the group consisting of rodent (mouse, rat), rabbit, dog, cat, pig, monkey, human being.
46. The host cell of embodiment 44, being selected from the group consisting of CHO, HEK293, COS, PC12, HiB5, RN33b, neuronal cells, foetal cells, ARPE-19, C2C12, HeLa, HepG2, striatal cells, neurons, astrocytes, interneurons.
47. A packaging cell line capable of producing an infective vector particle, said vector particle comprising a retrovirally derived genome comprising a 5' retroviral LTR, a tRNA binding site, a packaging signal, a promoter operably linked to a polynucleotide sequence encoding a protein with a signal peptide capable of functioning in a mammalian cell, and a Neurturin, said NTN being human, murine or rat Neurturin selected from the group consisting of pro-NTN, mature NTN, N-terminally truncated mature NTN, and a sequence variant of NTN; an origin of second strand DNA synthesis, and a 3' retroviral LTR.
48. The packaging cell line according to embodiment 47, wherein the vector particle is replication defective.
49. The packaging cell line according to embodiment 48, wherein the genome is lentivirally derived and the LTRs are lentiviral.
50. A chimeric non-human mammal comprising at least one cell being transduced with the vector of any of the preceding embodiments 24 to 41.
51. The mammal of embodiment 50, wherein the at least one transduced cell comprises the genome of the mammal.
52. An implantable cell culture device, the device comprising:
   i. a semipermeable membrane permitting the diffusion of a growth factor therethrough; and
   ii. at least one isolated host cell as defined by any of the preceding embodiments 43 to 46 or isolated host cells as defined in any of embodiments 74 to 79.
53. The device of embodiment 52, wherein the semipermeable membrane is immunoisolatory.
54. The device of embodiment 52, wherein the semipermeable membrane is microporous.
55. The device of embodiment 52, wherein the device further comprises a matrix disposed within the semipermeable membrane.
56. The device of embodiment 52, wherein the device is capable of secreting in excess of 10 ng of biologically active Neurturin per 24 hours, preferably in excess of 20 ng of biologically active Neurturin, more preferably in excess of 40 ng/24 hours, more preferably in excess of 60 ng/24 hours.
57. The device of embodiment 52, wherein the device further comprises a tether anchor.
58. A biocompatible capsule comprising: a core comprising living packaging cells that secrete a viral vector for infection of a target cell, wherein the viral vector is a vector according to any of the embodiments 24 to 41; and an external jacket surrounding said core, said jacket comprising a permeable biocompatible material, said material having a porosity selected to permit passage of retroviral vectors of approximately 100 nm diameter thereacross, permitting release of said viral vector from said capsule.
59. The capsule of embodiment 58, wherein the core additionally comprises a matrix, the packaging cells being immobilized by the matrix.
60. The capsule of embodiment 58, wherein the jacket comprises a hydrogel or thermoplastic material.
61. Use of the vector according to any of the embodiments 24 to 41 as a medicament.
62. Use of the vector according to any of the embodiments 24 to 41 for the preparation of a medicament for the treatment of a nervous system disorder.
63. Use of the vector according to any of the embodiments 24 to 41 for the preparation of a medicament for the treatment of a CNS disorder.
64. The use of embodiment 62, wherein the CNS disorder is a neurodegenerative disease.
65. The use of embodiment 64, wherein the neurodegenerative disease is a neurodegenerative disease involving lesioned and traumatic neurons, such as traumatic lesions of peripheral nerves, the medulla, the spinal cord, cerebral ischaemic neuronal damage, neuropathy, peripheral neuropathy, neuropathic pain, Alzheimer's disease, Huntington's disease, Parkinson's disease, amyotrophic lateral sclerosis, memory impairment connected to dementia.
66. The use of embodiment 64, wherein the neurodegenerative disease is Parkinson's Disease.
67. The use of embodiment 64, wherein the neurodegenerative disease is spinal cord injury.
68. The use of embodiment 64, wherein the neurodegenerative disease is amyotrophic lateral sclerosis.
69. The use of embodiment 64, wherein the disease is an eye disease, such as retinitis pigmentosa, macular degeneration, glaucoma, diabetic retinopathy.
70. A method of treating a nervous system disease, said method comprising administering to an individual in need thereof:
   i. a therapeutically effective amount of the vector of any of the embodiments 24 to 41; or
   ii. a therapeutically effective amount of the pharmaceutical composition of any of embodiments 42.
71. The method of embodiment 70, wherein the vector or composition is injected into the striatum of said individual.
72. A method of treating a nervous system disease, said method comprising transplanting to an individual in need thereof:
   i. a therapeutically effective amount of the transduced cells of embodiments 43 to 46;
   ii. a therapeutically effective amount of the cells of embodiments 74 to 81;
   iii. an implantable device according to any of the embodiments 52 to 57; or
   iv. a biocompatible device according to any of the embodiments 58 to 60.
73. The method of embodiment 71, wherein transplantation comprises an autologous transplant, an allogeneic transplant or a xenogeneic transplant.
74. A mammalian cell capable of secreting neurturin or a functional equivalent thereof in amounts in excess of 500 ng/10⁶ cells/24 hours.
75. The cell of embodiment 74, being capable of secreting at least of 1000 ng/10⁶ cells/24 hours, more preferably at least 5000, more preferably at least 10,000, more preferably at least 15,000, more preferably at least 20,000, more preferably at least 25,000, more preferably at least 30,000, more preferably at least 35,000.
76. The cell of embodiment 74, being selected from the group consisting of ARPE-19 cells, CHO cells, BHK cells, R1.1 cells, COS cells, killer cells, helper T-cells, cytotoxic T-lymphocytes and macrophages.
77. The cell of embodiment 74, wherein said mammal is selected from the group consisting of rodent (mouse, rat), rabbit, dog, cat, pig, monkey, human being.
78. The cell of embodiment 77, being a human cell.
79. The cell of embodiment 74, being selected from the group consisting of CHO, HEK293, COS, PC12, HiB5, RN33b, neuronal cells, foetal cells, ARPE-19, MDX12, C2C12, HeLa, HepG2, striatal cells, neurons, astrocytes, interneurons.
80. The mammalian cell according to embodiment 74, being transduced with the vector of any of the embodiments 24 to 41.
81. The mammalian cell according to any of the embodiments 74 to 79 being attached to a support matrix.
82. A method of producing neurturin or a functional equivalent thereof, said method comprising culturing the cell of any of the embodiments 74 to 79 and recovering the neurturin from the culture medium.

## Claims

1. A mammalian cell capable of secreting neurturin or a functional equivalent thereof in amounts in excess of 500 ng/10⁶ cells/24 hours.

2. The cell of claim 1, being capable of secreting at least of 1000 ng/10⁶ cells/24 hours, more preferably at least 5000, more preferably at least 10,000, more preferably at least 15,000, more preferably at least 20,000, more preferably at least 25,000, more preferably at least 30,000, more preferably at least 35,000.

3. The cell of claim 1, being selected from the group consisting of ARPE-19 cells, CHO cells, BHK cells, R1.1 cells, COS cells, killer cells, helper T-cells, cytotoxic T-lymphocytes and macrophages.

4. The cell of claim 1, wherein said mammal is selected from the group consisting of rodent (mouse, rat), rabbit, dog, cat, pig, monkey, human being.

5. The cell of claim 4, being a human cell.

6. The cell of claim 1, being selected from the group consisting of CHO, HEK293, COS, PC12, HiB5, RN33b, neuronal cells, foetal cells, ARPE-19, MDX12, C2C12, HeLa, HepG2, striatal cells, neurons, astrocytes, interneurons.

7. The mammalian cell according to any of the claims 1 to 6 being attached to a support matrix.

8. A method of producing neurturin or a functional equivalent thereof, said method comprising culturing the cell of any of the claims 1 to 6 and recovering the neurturin from the culture medium.

9. An implantable cell culture device, the device comprising:
i. a semi-permeable membrane permitting the diffusion of a growth factor there through; and
ii. isolated host cells as defined in any of claims 1 to 6.

10. The device of claim 9, wherein the semi-permeable membrane is immunoisolatory.

11. The device of claim 9, wherein the device further comprises a matrix disposed within the semi-permeable membrane.

12. The device of claim 9, wherein the device is capable of secreting in excess of 10 ng of biologically active Neurturin per 24 hours, preferably in excess of 20 ng of biologically active Neurturin, more preferably in excess of 40 ng/24 hours, more preferably in excess of 60 ng/24 hours.

13. The device of claim 9, wherein the device further comprises a tether anchor.

14. The mammalian cell of any of claims 1 to 7 for use in therapy.

15. The cells of any of claims 1 to 7 for use in a method of treating a nervous system disease, said method comprising transplanting to an individual in need thereof a therapeutically effective amount of the cells of any of claims 1 to 7.
